# EUROPEAN PATENT APPLICATION

(11) **EP 2 397 468 A1**
(43) Date of publication of application: **21.12.2011**
(21) Application number: 09828829.3
(22) Date of filing: 24.11.2009
(51) Int. Cl.: C07D 233/58, C07F 7/10, C09K 9/02, G03C 1/73

(54) **NOVEL CROSSLINKED HEXAARYL BISIMIDAZOLE COMPOUND AND DERIVATIVE THEREOF, METHOD FOR PRODUCING THE COMPOUND AND PRECURSOR COMPOUND TO BE USED IN THE PRODUCTION METHOD**

(30) Priority: 28.11.2008 JP 2008304881; 26.03.2009 JP 2009077826; 26.03.2009 JP 2009077827; 26.08.2009 JP 2009195905
(71) Applicant: Abe, Jiro, Kanagawa 214-0005 (JP); Kanto Kagaku Kabushiki Kaisha, Chuo-ku, Tokyo 103-0023 (JP)
(72) Inventor: KISHIMOTO, Yuta, Okayama-shi Okayama 704-8174 (JP); KATO, Daisuke, Kurokawa-gun Miyagi 981-3412 (JP); KIMOTO, Atsushi, Akashi-shi Hyogo 673-0876 (JP)
(74) Representative: Zech, Stefan Markus
(86) International application number: PCT/JP2009/006326
(87) International publication number: WO 2010/061579

(57) **Abstract**

Provided is a crosslinked hexaaryl bisimidazole compound which can achieve photochromic characteristics, i.e., visually showing decoloring simultaneously with the stop of light irradiation and enables precise control of color tone, density and so on in coloring. Also provided are a method for producing the aforesaid compound whereby the degrees of freedom in molecular design and synthesis can be increased, and a precursor compound to be used in the production method.

## Description

### [Technical Field]

The present invention relates to a novel bridged hexaarylbisimidazole compound and a method for producing the compound, and a precursor compound used in the production method. More particularly, it relates to a novel bridged hexaarylbisimidazole compound that has rapid color switching characteristics and high color density and enables precise control of coloration tone and density, a multimer compound of the compound in which a plurality of bisimidazole skeleton-containing structural units are polymerized, a method for producing the compound that has a high degree of freedom in terms of molecular design and synthesis, and a precursor compound that is a key compound used in the production method.

### [Background Art]

As photochromic compounds exhibiting photochromism, hexaarylbisimidazole (hereinafter, also called 'HABI')(Non-Patent Document 1), diarylethene (Patent Document 1), spirooxazine (Patent Document 2), etc. are known, and since these compounds exhibit reversible coloration by irradiation with light, research for applying these compounds to light control materials (Patent Document 3) or to photorecording materials (Patent Documents 4 and 5) has been actively carried out.

Hexaarylbisimidazole (HABI) generates a triarylimidazolyl radical (hereinafter, also called a 'TAIR'), which is a highly reactive radical species, upon irradiation with UV light and is therefore conventionally widely used as a photopolymerization initiator (Patent Documents 6 to 8).

With regard to photochromic compounds, P-type photochromic compounds, which after isomerization by irradiation with light reversibly turn back to the original structure by irradiation with light having a different wavelength, and T-type photochromic compounds, which after isomerization by irradiation with light reversibly turn back to the original structure by a thermal reaction over a few hours to a few minutes, are known.
However, there is the problem that, since these conventional photochromic compounds go back and forth between isomers having different structures, a decoloration reaction takes at least a few minutes to a few seconds. Furthermore, HABI has the problem that two triarylimidazolyl radicals generated by carbon-nitrogen bond cleavage diffuse in a medium, it takes time for the radicals to recombine, the decoloration reaction rate is slow, and stability characteristics over time, such as repetition durability, are poor.

As one attempt to solve this problem, there is a report of the synthesis of a molecule in which two triarylimidazolyl radical molecules are introduced into the 1-and 8- positions of naphthalene (1,8-NDPI-TPI-naphthalene) (Non-Patent Document 2). Diffusion of radicals is suppressed by stabilizing the colored form as a result of formation of a resonant structure in which two naphthalene skeletons and an imidazolyl radical are conjugated; however, not only is this compound not fully satisfactory in terms of decoloration speed but there is also the serious problem that the colored form, which is a stabilized radical species, undergoes a hydrogen abstraction reaction from a surrounding medium and is degraded, and it is not a photochromic compound which has sufficient properties from the viewpoint of application in uses for various purposes.

### [Prior Art Documents]

[Patent Document 1] JP-A-2005-325087
[Patent Document 2] JP-A-2005-266608
[Patent Document 3] JP-A-2005-215640
[Patent Document 4] JP-A-2000-112074
[Patent Document 5] JP-A-08-245579
[Patent Document 6] JP-A-2008-089789
[Patent Document 7] JP-A-2005-309442
[Patent Document 8] JP-A-08-292573

[Non-Patent Document 1] Hayashi, T.; Maeda, K., Bull. Chem. Soc. Jpn. 1960, 33, 565-566.
[Non-Patent Document 2] Fujita, K.; Hatano, S.; Kato, D.; Abe, J., Org. Lett. 2008, 10, 3105-3108.

### [Disclosure of Invention]

### [Problems to be Solved by the Invention]

It is therefore an object of the present invention to solve the above-mentioned problems and provide a photochromic compound that has excellent thermal stability and stability over time, achieves a rapid color switching reaction and high color density, realizes precise control of tone and density of coloration, and can be applied in uses for various purposes. It is another object of the present invention to provide a method for producing a photochromic compound that enables compounds having various structures to be synthesized by increasing the degree of freedom in terms of molecular design and synthesis, and a precursor compound used in the production method.

### [Means for Solving the Problems]

While carrying out an intensive investigation in order to solve the above-mentioned problems, the present inventors have found that, by restraining two triarylimidazolyl radicals (TAIRs) generated by irradiation with light by means of a bridging group, in particular by means of a bridging group that does not conjugate with the TAIR, excellent thermal stability and stability over time can be obtained, and a rapid color switching reaction and high color density can be realized. The present inventors have also found that, by forming a bridged hexaarylbisimidazole compound having an asymmetric structure with the optimum molecular design of the structure of two TAIR moieties depending on the intended application, precise control of tone and density of coloration becomes possible. The present inventors have further found that, by carrying out synthesis of, as a key compound, a precursor compound with a specific structure having an imidazole skeleton and an aldehyde group, a method for producing a photochromic compound that enables the degree of freedom in terms of molecular structure design and synthesis to be increased can be realized. Moreover, as a result of further investigation, the present invention has been accomplished.

That is, the present invention relates to a compound represented by general formula (1) below 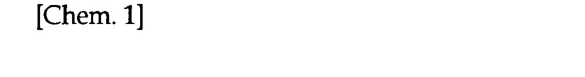 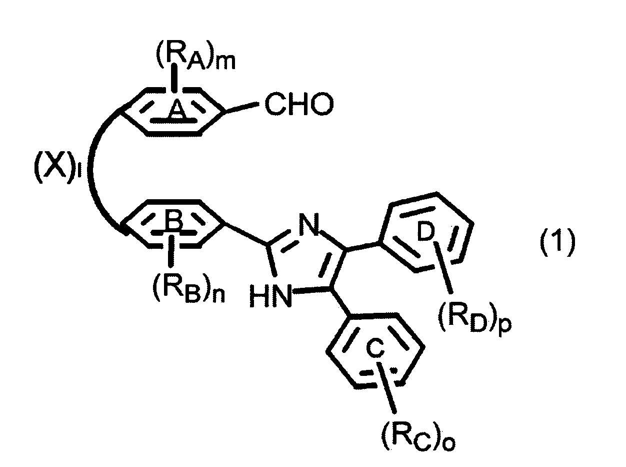 wherein, the two aryl groups A and B are bridged to each other via carbon atoms by means of a bridging group X (excluding 1,8-naphthalenylene), 1 is an integer of 1 to 5,
when the bridging group X contains hydrogen atoms, said hydrogen atoms may be mutually independently replaced by one or more substituents Rx,
the Rxs are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiZ₂Z₁Z₂ group, and a -Y₁-SiZ₂Z₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons),

the four aryl groups A to D mutually independently may or may not have substituents R_{A} to R_{D},
m and n are mutually independently an integer of 0 to 4,
o and p are mutually independently an integer of 0 to 5,
the substituents R_{A} and R_{B} are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons),

the substituents R_{C} and R_{D} are independently identical to or different from each other and are one or more substituents selected from the group consisting of a substituent having the same meaning as that of the above substituents R_{A} and R_{B}, a substituent represented by partial structural formula (i) below 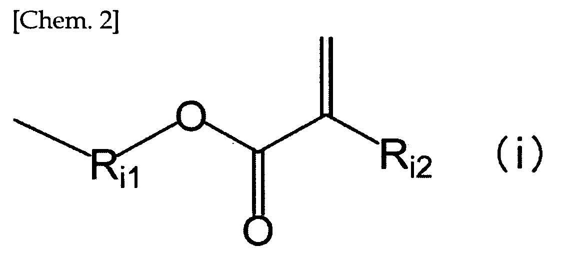 (here, Rᵢ₁ denotes an alkylene group or alkoxylene group having 1 to 20 carbons, and Rᵢ₂ denotes hydrogen or an alkyl group having 1 to 3 carbons),

a substituent represented by partial structural formula (ii) below 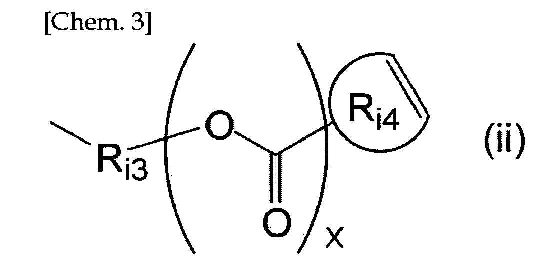 (here, Rᵢ₃ denotes an alkylene group or alkoxylene group having 1 to 20 carbons, Rᵢ₄ denotes a cyclic olefin having a total number of carbons and silicons of 5 to 10, and × denotes 0 or 1), and a substituent represented by partial structural formula (iii) below

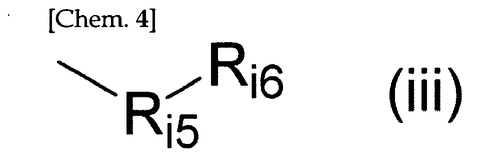

(here, Rᵢ₅ denotes an alkylene group or alkoxylene group having 1 to 20 carbons, and Rᵢ₆ denotes an ethylene group or an acetylene group),
the above substituents R_{A} to R_{D} furthermore may or may not form an aliphatic or aromatic ring together with the carbon atom to which they are bonded and another substituent, and said ring may or may not further have a substituent having the same meaning as that of the above substituents Rc and R_{D}.

Furthermore, the present invention relates to the compound wherein the bridging group is a bridging group that does not conjugate with a triarylimidazolyl radical (TAIR).
Moreover, the present invention relates to the compound wherein m and n are 0 and the bridging group X is unsubstituted, or the substituents R_{A}, R_{B}, and Rx are methyl groups.
Furthermore, the present invention relates to the compound, wherein it is represented by general formula (1a) below. 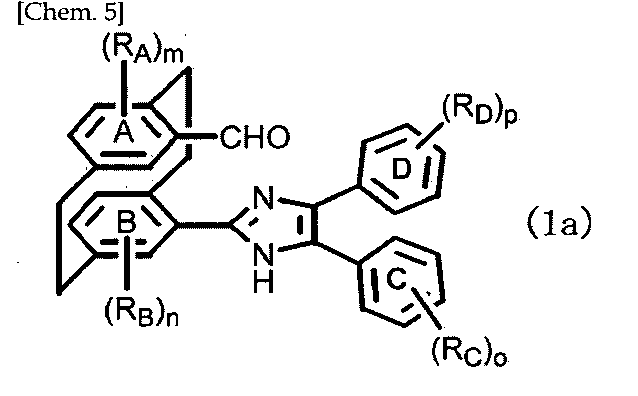 Moreover, the present invention relates to a compound represented by general formula (2) below 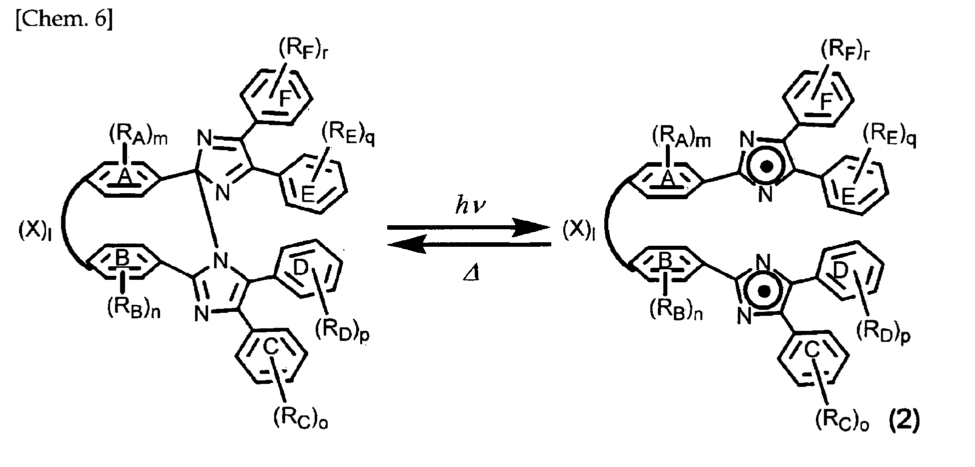 wherein, the two aryl groups A and B are bridged to each other via carbon atoms by means of a bridging group X (excluding 1,8-naphthalenylene), 1 is an integer of 1 to 5,
when the bridging group X contains hydrogen atoms, said hydrogen atoms may mutually independently be replaced by one or more substituents Rx,
the Rxs are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons), the six aryl groups A to F may or may not, mutually independently, have substituents R_{A} to R_{F},

m and n are mutually independently an integer of 0 to 4,
o to r are mutually independently an integer of 0 to 5,
the substituents R_{A} and R_{B} are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons), the substituents Rc to R_{F} are independently identical to or different from each other and are one or more substituents selected from the group consisting of a substituent having the same meaning as that of the above substituents R_{A} and R_{B}, a substituent represented by partial structural formula (i) below 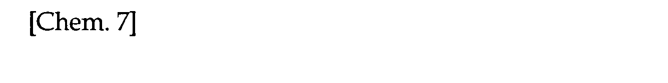 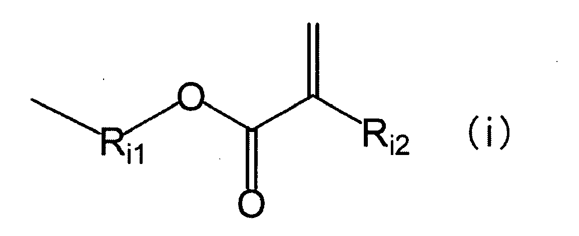 (here, Rᵢ₁ denotes an alkylene or alkoxylene group having 1 to 20 carbons, and Rᵢ₂ denotes hydrogen or an alkyl group having 1 to 3 carbons),

a substituent represented by partial structural formula (ii) below 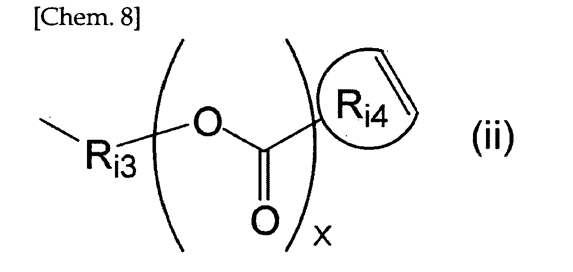 (here, Rᵢ₃ denotes an alkylene or alkoxylene group having 1 to 20 carbons, Rᵢ₄ denotes a cyclic olefin having a total number of carbons and silicons of 5 to 10, and x denotes 0 or 1), and a substituent represented by partial structural formula (iii) below

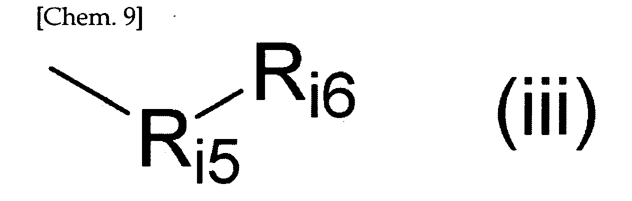

(here, Rᵢ₅ denotes an alkylene or alkoxylene group having 1 to 20 carbons, and Rᵢ₆ denotes an ethylene group or an acetylene group),
the substituents R_{A} to R_{F} may or may not form an aliphatic or aromatic ring together with the carbon atom to which they are bonded and another substituent, and said ring may or may not further have a substituent having the same meaning as that of the above substituents Rc to R_{F}.

Furthermore, the present invention relates to the compound wherein at least one of the substituents R_{A} to R_{F} is not hydrogen.
Moreover, the present invention relates to the compound wherein it is asymmetric in that the structure of a triarylimidazole moiety containing the aryl group A and the structure of a triarylimidazole moiety containing the aryl group B are different.
Furthermore, the present invention relates to the compound wherein the bridging group is a bridging group that does not conjugate with a triarylimidazolyl radical (TAIR).
Moreover, the present invention relates to the compound wherein the bridging group is one or more types of bridging group selected from the group consisting of a -CH₂CH₂- group, a -CH₂CH₂CH₂- group, a -CH₂CH₂CH₂CH₂- group, an -Si(CH₃)₂Si(CH₃)₂- group, an -Si(CH₃)₂Si(CH₃)₂Si(CH₃)₂- group, an - Si(CH₃)₂Si(CH₃)₂Si(CH₃)₂Si(CH₃)₂- group, an -SiH₂OSiH₂- group, an - Si(CH₃)₂OSi(CH₃)₂- group, an -Si(CH₃CH₂)₂OSi(CH₂CH₃)₂- group, a -CH₂SCH₂-group, a -CH₂OCH₂- group, an -OCH₂CH₂O- group, and a -CH₂OCH₂CH₂OCH₂-group.

Furthermore, the present invention relates to the compound wherein m and n are 0 and the bridging group X is unsubstituted, or the substituents R_{A}, R_{B}, and Rx are methyl groups.
Moreover, the present invention relates to the compound wherein general formula (2) is represented by general formula (2a) below 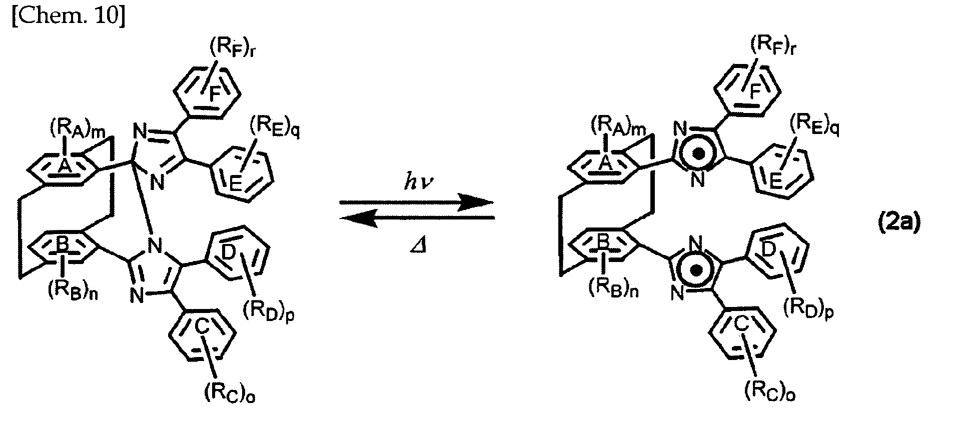 wherein, m and n are mutually independently 0 to 3,
o to r are mutually independently an integer of 0 to 5, and
the substituents R_{A}, R_{B}, and R_{C} to R_{F} are independently identical to or different from each other and have the same meanings as those of the substituents R_{A}, R_{B}, and R_{C} to R_{F} respectively in general formula (2) above.

Furthermore, the present invention relates to a compound represented by general formula (3) below 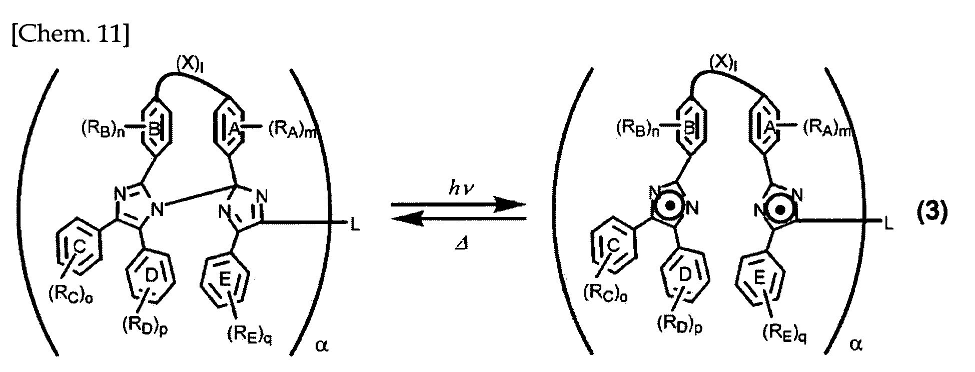 wherein, the two aryl groups A and B are bridged to each other via carbon atoms by means of a bridging group X (excluding 1,8-naphthalenylene), 1 is an integer of 1 to 5,
when the bridging group X contains hydrogen atoms, said hydrogen atoms may mutually independently be replaced by one or more substituents Rx,
α is an integer of 1 to 9,
the linking group L is a monocyclic or polycyclic aromatic compound containing 1 to 12 aromatic rings having 5 to 8 carbon atoms per ring structure,
the five aryl groups A to E in the bisimidazole skeleton-containing structural unit may or may not, mutually independently, have substituents R_{A}, R_{B}, and Rc to R_{E},
all of the substituents R_{A}, R_{B}, Rc to R_{E}, and Rx are independently identical to or different from each other and have the same meanings as those of the substituents R_{A}, R_{B}, Rc to R_{E}, and Rx respectively in general formula (2) above,
m and n are mutually independently an integer of 0 to 4,
o to r are mutually independently an integer of 0 to 5,
the substituents R_{A}, R_{B}, and Rc to R_{E} may or may not form an aliphatic or aromatic ring together with the carbon atom to which they are bonded and another substituent, and said ring and the aromatic ring of the linking group L may or may not further have a substituent R_{L}.

Furthermore, the present invention relates to the compound wherein general formula (3) above is represented by general formula (3b) below

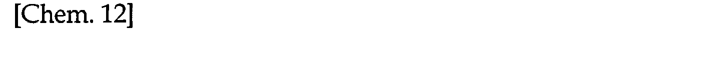

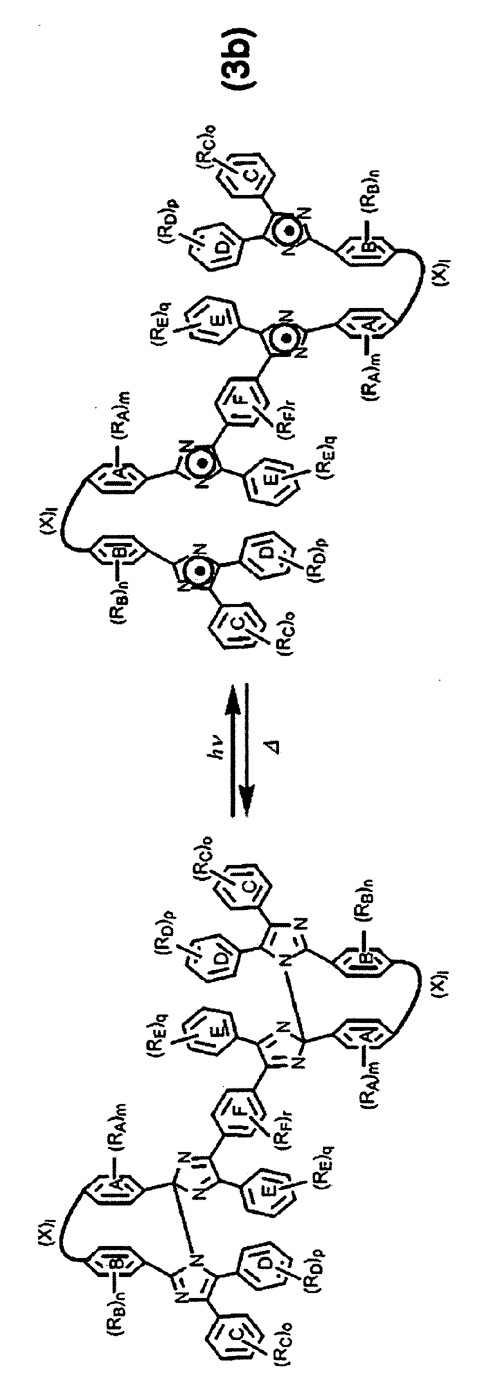

wherein, the 11 aryl groups may or may not have substituents R_{A}, R_{B}, and Rc to R_{F}, all of the substituents R_{A}, R_{B}, and Rc to R_{F} of the aryl groups are independently identical to or different from each other and have the same meanings as those of the substituents R_{A}, R_{B}, and R_{C} to R_{F} respectively in general formula (2) above,
m, n, and r are mutually independently an integer of 0 to 4, and o to q are mutually independently an integer of 0 to 5.

Furthermore, the present invention relates to the compound wherein general formula (3) above is represented by general formula (3c) below

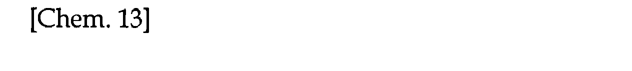

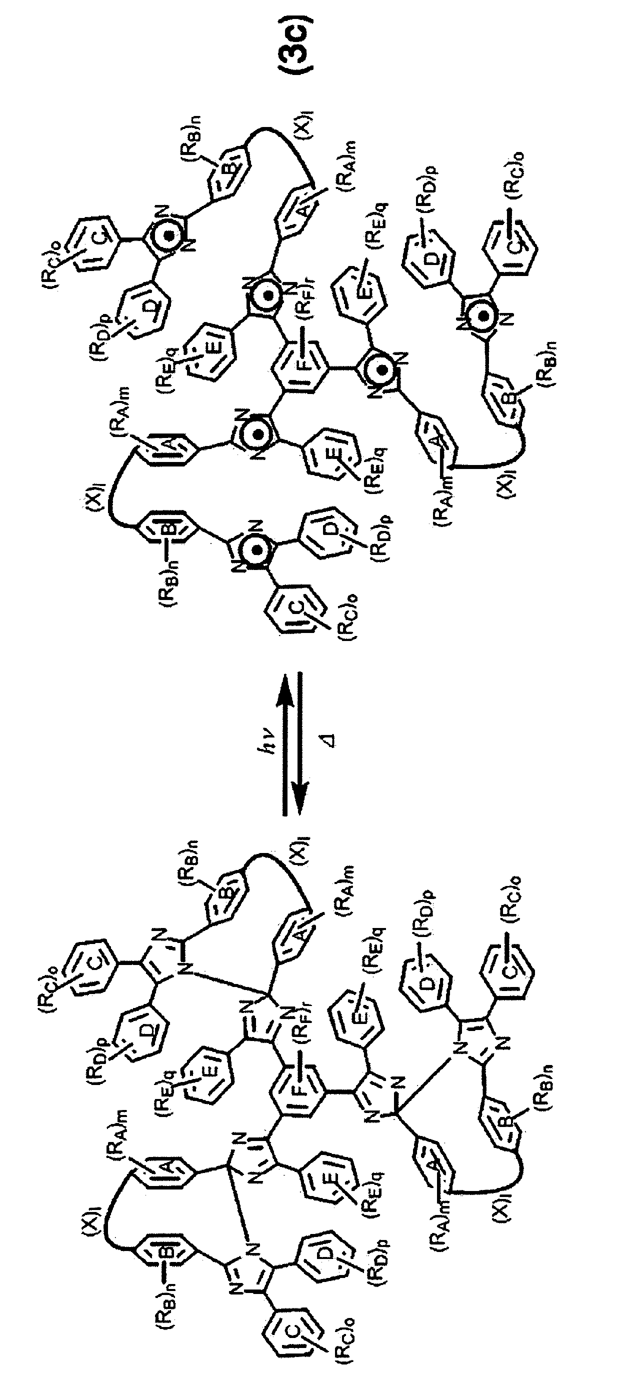

wherein, the 16 aryl groups may or may not have substituents R_{A}, R_{B}, and Rc to R_{F}, all of the substituents R_{A}, R_{B}, and R_{C} to R_{F} of the aryl groups are independently identical to or different from each other and have the same meanings as those of the substituents R_{A}, R_{B}, and R_{C} to R_{F} respectively in general formula (2) above,
m and n are mutually independently an integer of 0 to 4,
o to q are mutually independently an integer of 0 to 5, and
r is an integer of 0 to 3.

Moreover, the present invention relates to the compound wherein the bridging group is a bridging group that does not conjugate with a triarylimidazolyl radical (TAIR).
Furthermore, the present invention relates to the compound wherein the bridging group is one or more types of bridging group selected from the group consisting of a -CH₂CH₂- group, a -CH₂CH₂CH₂- group, a -CH₂CH₂CH₂CH₂- group, an -Si(CH₃)₂Si(CH₃)₂- group, an -Si(CH₃)₂Si(CH₃)₂Si(CH₃)₂- group, an - Si(CH₃)₂Si(CH₃)₂Si(CH₃)₂Si(CH₃)₂- group, an -SiH₂OSiH₂- group, an - Si(CH₃)₂OSi(CH₃)₂- group, an -Si(CH3CH₂)₂OSi(CH₂CH3)₂- group, a -CH₂SCH₂-group, a -CH₂OCH₂- group, an -OCH₂CH₂O- group, and a -CH₂OCH₂CH₂OCH₂-group.
Moreover, the present invention relates to the compound wherein m and n are 0 and the bridging group X is unsubstituted, or the substituents R_{A}, R_{B}, and Rx are methyl groups.

Furthermore, the present invention relates to the compound wherein the structure represented by partial structural formula (vi) below 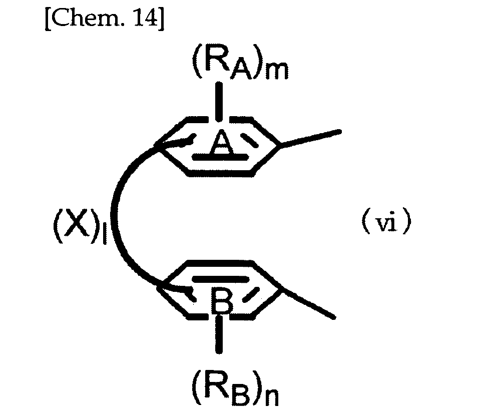 is represented by partial structural formula (vii) below 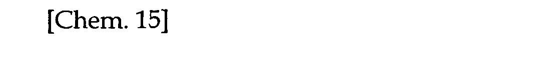 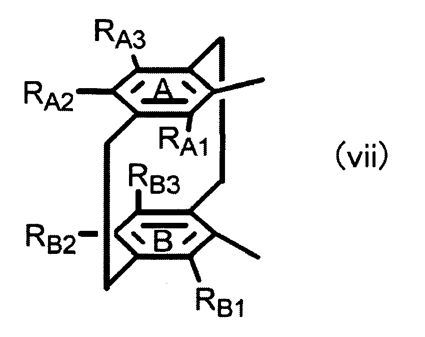 wherein, substituents R_{A1} to R_{A3} and R_{B1} to R_{B3} are independently identical to or different from each other and are substituents having the same meanings as those of the above substituents R_{A} and R_{B} respectively in general formula (3) above or hydrogen atoms.

Furthermore, the present invention relates to a polymer compound having a repeating structural unit represented by partial structural formula (iv) below

[Chem. 16] -[(A)ᵣ₋(B)_{δ}]_{ε} - (iv)

and/ or partial structural formula (v) below 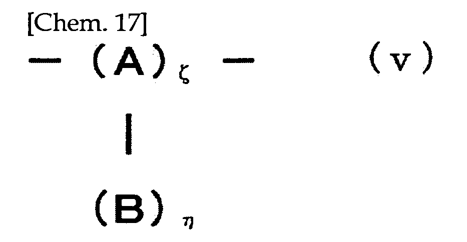 wherein, A is any linking group containing one or more types of atoms selected from the group consisting of carbon, nitrogen, and oxygen atoms,
B is a derivative of the compound,
A-B denotes a bond between the linking group and one or two substituents selected from substituents Rc to R_{F} and R_{L} of the compound,
Y is an integer of 0 or greater, and
δ, ε, ζ, and η are mutually independently an integer of 1 or greater.

Furthermore, the present invention relates to a photochromic material containing the compound and/or polymer compound according to any of the above.
Moreover, the present invention relates to a solvent containing the compound and/ or polymer compound according to any of the above.
Furthermore, the present invention relates to a resin containing the compound and/or polymer compound according to any of the above.
Moreover, the present invention relates to a material composition having photochromism and containing one or more types selected from the group consisting of the compound, polymer compound, photochromic material, solvent, and resin according to any of the above, the material composition being selected from the group consisting of a light control material, a holographic material, an ink material, an optical information display device, an optical switch element, and a photoresist material.

Furthermore, the present invention relates to a method for producing the compound, the method including reacting the precursor compound and a compound represented by general formula (4) below 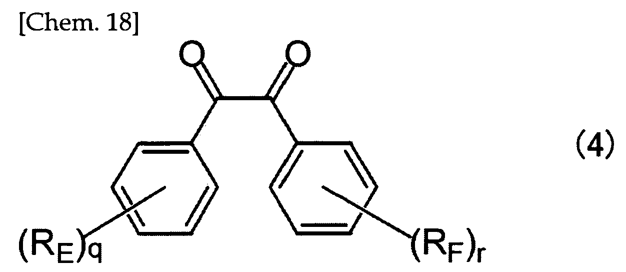 wherein, the two aryl groups may or may not, mutually independently, have substituents R_{E} and R_{F},
said substituents R_{E} and R_{F} are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons), a substituent represented by partial structural formula (i) below 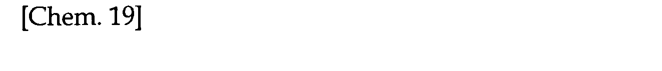 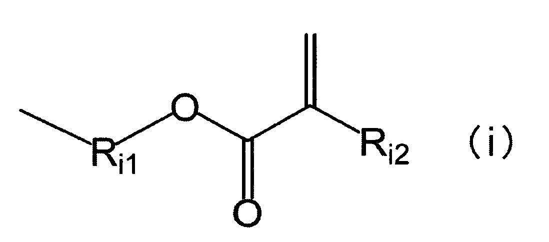 (here, Rᵢ₁ denotes an alkylene or alkoxylene group having 1 to 20 carbons and Rᵢ₂ denotes hydrogen or an alkyl group having 1 to 3 carbons),

a substituent represented by partial structural formula (ii) below 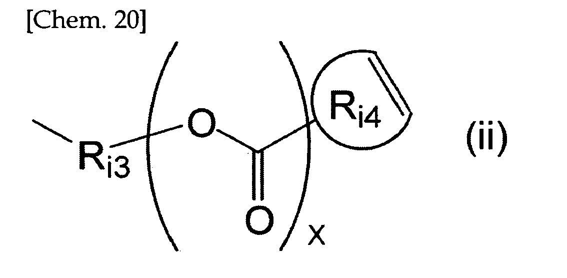 (here, Rᵢ₃ denotes an alkylene or alkoxylene group having 1 to 20 carbons, Rᵢ₄ denotes a cyclic olefin having a total number of carbons and silicons of 5 to 10, and x denotes 0 or 1), and a substituent represented by partial structural formula (iii) below

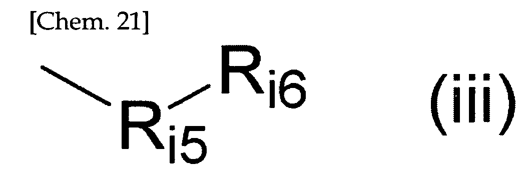

(here, Rᵢ₅ denotes an alkylene or alkoxylene group having 1 to 20 carbons and Rᵢ₆ denotes an ethylene group or an acetylene group),
q and r are mutually independently an integer of 0 to 5,
the substituent of the aryl group may or may not form, together with the carbon atom to which it is bonded and another substituent, an aliphatic or aromatic ring, and said ring may or may not have a substituent having the same meaning as that of the substituents of the aryl group.

Furthermore, the present invention relates to a method for producing the compound, the method including reacting the precursor compound and a compound represented by general formula (5) below 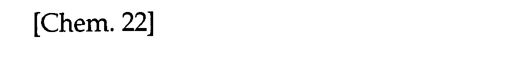 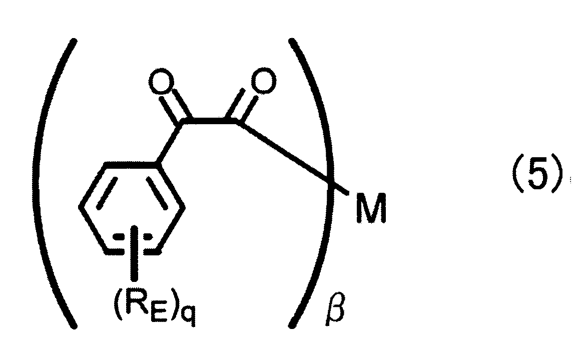 wherein, β is an integer of 1 to 9,
the linking group M is a monocyclic or polycyclic aromatic compound containing 1 to 12 aromatic rings having 5 to 8 carbon atoms per ring structure,
the aryl group in the 1,2-diketone skeleton-containing structural unit may or may not have a substituent R_{E},
all of the substituents of the aryl group are independently identical to or different from each other and have the same meaning as that of R_{E} of general formula (4) above,
q is an integer of 1 to 5,
the substituent R_{E} may or may not form, together with the carbon atom to which it is bonded and another substituent, an aliphatic or aromatic ring, and said ring and the aromatic ring of the linking group M may or may not further have a substituent R_{M}.

In general formulae (2), (2a), (3), (3b), and (3c) above, 'hv→' means a shift from the compound of the present invention to a radical species, which is a colored form having a high energy level, due to absorption of energy such as UV light, and '←Δ' means a reversible shift from said radical species to the original bisimidazole monomer or multimer, which has a low energy level, due to absorption of thermal energy. In the present specification, for example, the term 'compound represented by formula (2)' means a compound represented by the left side of formula (2), which undergoes a transition to a radical species on the right side by absorption of energy (hv) and reversibly shifts to the compound on the left side by absorption of thermal energy.

The compound containing an HABI of the present invention is characterized by the aryl groups of two triarylimidazolyl radicals (TAIRs) formed by irradiation with UV light, visible light, etc. being restrained by a bridging group, in particular a bridging group that does not conjugate with a TAIR, thereby suppressing separation and diffusion of the two TAIRs and, furthermore, suppressing unnecessary stabilization of the radicals due to the TAIR and the bridging group taking a resonant structure by conjugation.

### [Effects of the Invention]

Compared with a conventional photochromic compound, the bridged hexaarylbisimidazole compound of the present invention and a multimer compound of said compound have excellent thermal stability and stability over time and have both rapid color switching characteristics and high color density. In particular, they enable the photochromic property of color disappearing visually at the same time as irradiation with light stops to be realized. Furthermore, the molecular structure can be designed optimally according to the intended application or purpose, and by means of any one of R_{A} to R_{F} in general formula (2) above preferably being a substituent other than a hydrogen atom, more preferably having an asymmetric structure, or by means of a multimer structure represented by general formula (3) above, it becomes possible to carry out precise control of photochromic properties such as tone and density of coloration. It can therefore be anticipated that the compound of the present invention will be applied to a wide range of fields such as light control materials that react with sunlight, optical switch elements, optical information display devices, photoresist materials, holographic materials, and ink materials.

Furthermore, the method for producing the compound using the precursor compound of the present invention can increase the degree of freedom of molecular structure design and synthesis of a photochromic compound. It can therefore be anticipated that the method will be applied to the production of photochromic compounds having various structures that can be applied to a wide range of uses.

### [Brief Description of Drawings]

[FIG. 1] A diagram showing the molecular structure of *pseudogem-*bis(diphenylimidazole)[2.2]paracyclophane of Example 1 (hereinafter also called '*pseudogem*-bisDPI[2.2]paracyclophane'), elucidated by single crystal X-ray diffraction structural analysis.
[FIG. 2] A graph showing the results of measurement of visible/near-IR absorption spectra of the compound of Example 1 immediately after irradiation with a nanosecond UV laser in a nanosecond laser flash photolysis measurement.
[FIG. 3] A graph showing the results of measurement of decay of the absorption band (400 nm) of the compounds of Example 1 and Comparative Example 1 in a nanosecond laser flash photolysis measurement.

[FIG. 4] A graph showing the results of measurement of visible/near-IR absorption spectra of *pseudogem*-bisDPI[2.2]paracyclophane (solvent: dichloromethane, concentration: 2.1 × 10⁻⁴ M) immediately after irradiation with a nanosecond UV laser in a nanosecond laser flash photolysis measurement.
[FIG. 5] A graph showing the results of measurement of decay of the absorption band (400 nm) of *pseudogem*-bisDPI[2.2]paracyclophane (solvent: dichloromethane, concentration: 2.1 × 10⁻⁴ M) in a nanosecond laser flash photolysis measurement.

[FIG. 6] A graph showing the results of measurement of a visible/near-IR absorption spectrum of a PMMA thin film containing *pseudogem*-bisDPI[2.2]paracyclophane of Example 2 immediately after irradiation with a nanosecond UV laser in a nanosecond laser flash photolysis measurement.
[FIG. 7] A graph showing the results of measurement of decay of the absorption band (400 nm) of the PMMA thin film of Example 2 before irradiation with a nanosecond UV laser and after every 1,000 times of irradiation with a nanosecond UV laser in a nanosecond laser flash photolysis measurement.
[FIG. 8] A graph showing a comparison of the results of measurement of decay of the absorption band (400 nm) of the PMMA thin film of Example 2 before irradiation with a nanosecond UV laser and after irradiating 10,000 times with a nanosecond UV laser in a nanosecond laser flash photolysis measurement.

[FIG. 9] A graph showing the results of measurement of a visible absorption spectrum of a benzene solution of 1,3-bis(triphenylimidazole)-1,1,3,3-tetramethyldisiloxane (hereinafter also called 'bisTPI-TMDS') of Example 3 before irradiation with UV light and after irradiation with UV light.
[FIG. 10] A graph showing the results of measurement of visible/near-IR absorption spectra of a compound of Example 5 immediately after irradiation with a nanosecond UV laser in a nanosecond laser flash photolysis measurement.
[FIG. 11] A graph showing the results of measurement of decay of the absorption band (400 nm) of the compound of Example 5 in a nanosecond laser flash photolysis measurement.

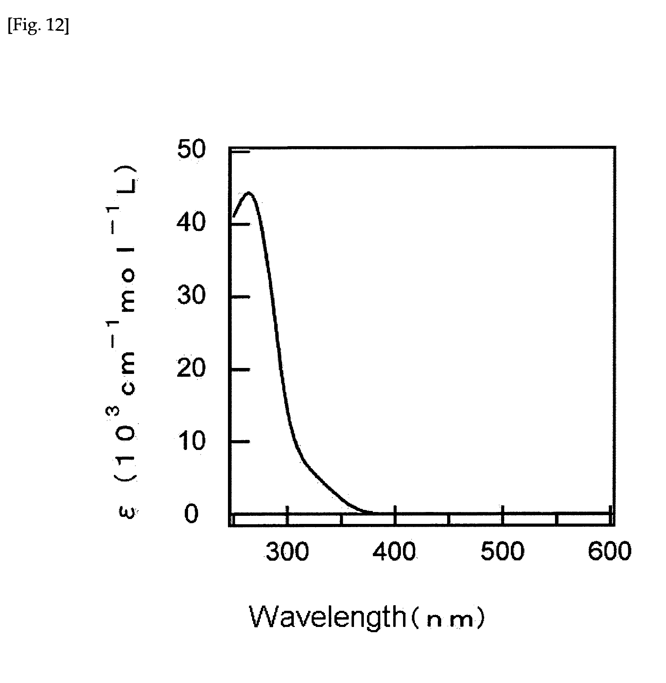

[FIG. 12] A graph showing a UV-visible absorption spectrum of the compound of Example 1.
[FIG. 13] A diagram showing the molecular structure of a compound of Example 7, elucidated by single crystal X-ray diffraction structural analysis.
[FIG. 14] A graph showing a UV-visible absorption spectrum of the compound of Example 7.
[FIG. 15] A graph showing the results of measurement of visible/near-IR absorption spectra of the compound of Example 7 immediately after irradiation with a nanosecond UV laser in a nanosecond laser flash photolysis measurement.
[FIG. 16] A graph showing the results of measurement of decay of the absorption band (400 nm) of the compound of Example 7 in a nanosecond laser flash photolysis measurement.

[FIG. 17] A graph showing a UV-visible absorption spectrum of a compound of Example 8.
[FIG. 18] A graph showing the results of measurement of visible/near-IR absorption spectra of the compound of Example 8 immediately after irradiation with a nanosecond UV laser in a nanosecond laser flash photolysis measurement.
[FIG. 19] A graph showing the results of measurement of decay of the absorption band (400 nm) of the compound of Example 8 in a nanosecond laser flash photolysis measurement.
[FIG. 20] A graph showing a UV-visible absorption spectrum of a compound of Example 9.

[FIG. 21] A graph showing the results of measurement of visible/near-IR absorption spectra of the compound of Example 9 immediately after irradiation with a nanosecond UV laser in a nanosecond laser flash photolysis measurement.
[FIG. 22] A graph showing the results of measurement of decay of the absorption band (400 nm) of the compound of Example 9 in a nanosecond laser flash photolysis measurement.
[FIG. 23] A graph showing a UV-visible absorption spectrum of a compound of Example 10.
[FIG. 24] A graph showing the results of measurement of visible/near-IR absorption spectra of the compound of Example 10 immediately after irradiation with a nanosecond UV laser in a nanosecond laser flash photolysis measurement.

[FIG. 25] A graph showing the results of measurement of decay of the absorption band (400 nm) of the compound of Example 10 in a nanosecond laser flash photolysis measurement.
[FIG. 26] A graph showing a UV-visible absorption spectrum of a compound of Example 11.
[FIG. 27] A graph showing the results of measurement of visible/near-IR absorption spectra of the compound of Example 11 immediately after irradiation with a nanosecond UV laser in a nanosecond laser flash photolysis measurement.
[FIG. 28] A graph showing the results of measurement of decay of the absorption band (400 nm) of the compound of Example 11 in a nanosecond laser flash photolysis measurement.

[FIG. 29] A diagram showing the molecular structure of a compound of Example 12, elucidated by single crystal X-ray diffraction structural analysis.
[FIG. 30] A graph showing the results of measurement of visible/near-IR absorption spectra of the compound of Example 12 immediately after irradiation with a nanosecond UV laser in a nanosecond laser flash photolysis measurement.
[FIG. 31] A graph showing the results of measurement of decay of the absorption band (400 nm) of the compound of Example 12 in a nanosecond laser flash photolysis measurement.
[FIG. 32] A graph showing a UV-visible absorption spectrum of a compound of Example 13.
[FIG. 33] A graph showing the results of measurement of visible/near-IR absorption spectra of the compound of Example 13 immediately after irradiation with a nanosecond UV laser in a nanosecond laser flash photolysis measurement.
[FIG. 34] A graph showing the results of measurement of decay of the absorption band (750 nm) of the compound of Example 13 (solid line) and the compound of Example 1 (dotted line), which is an unsubstituted derivative, in a nanosecond laser flash photolysis measurement.
[FIG. 35] A graph showing the results of measurement of visible/near-IR absorption spectra of a compound of Example 14 immediately after irradiation with a nanosecond UV laser in a nanosecond laser flash photolysis measurement.
[FIG. 36] A graph showing the results of measurement of decay of the absorption band (400 nm) of the compound of Example 14 in a nanosecond laser flash photolysis measurement.

[FIG. 37] A graph showing the results of measurement of visible/near-IR absorption spectra of a polymer of Example 15 in the solution state immediately after irradiation with a nanosecond UV laser in a nanosecond laser flash photolysis measurement.
[FIG. 38] A graph showing the results of measurement of decay of the absorption band (400 nm) of the polymer of Example 15 in the solution state in a nanosecond laser flash photolysis measurement.
[FIG. 39] A graph showing the results of measurement of a visible/near-IR absorption spectrum of the polymer of Example 15 in a thin film state immediately after irradiation with a nanosecond UV laser in a nanosecond laser flash photolysis measurement.
[FIG. 40] A graph showing the results of measurement of decay of the absorption band (400 nm) of the polymer of Example 15 in the thin film state in a nanosecond laser flash photolysis measurement.

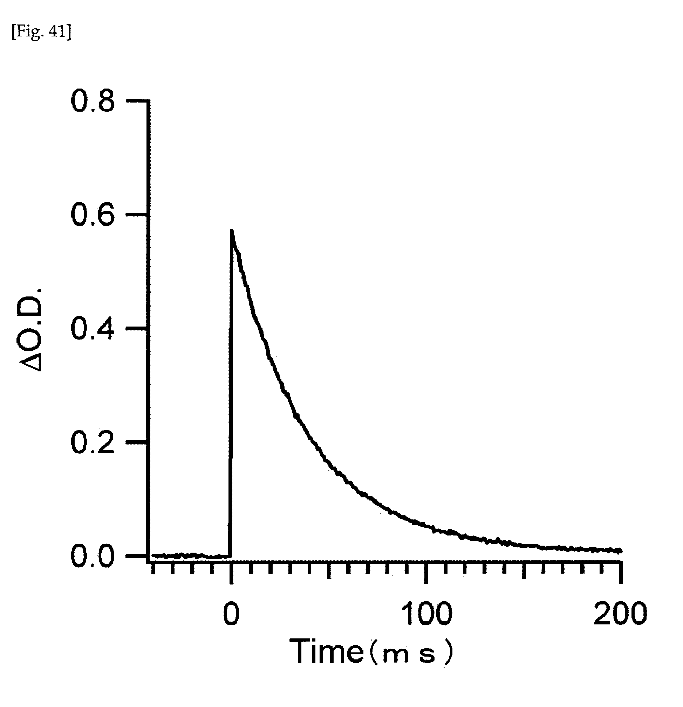

[FIG. 41] A graph showing the results of measurement of decay of the absorption band (400 nm) of a compound of Example 16 in a nanosecond laser flash photolysis measurement.

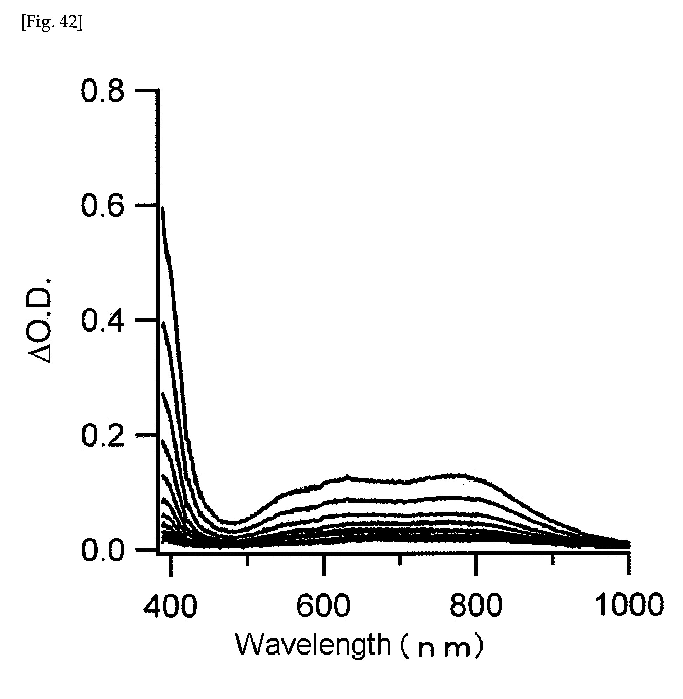

[FIG. 42] A graph showing the results of measurement of visible/near-IR absorption spectra of the compound of Example 16 immediately after irradiation with a nanosecond UV laser in a nanosecond laser flash photolysis measurement.

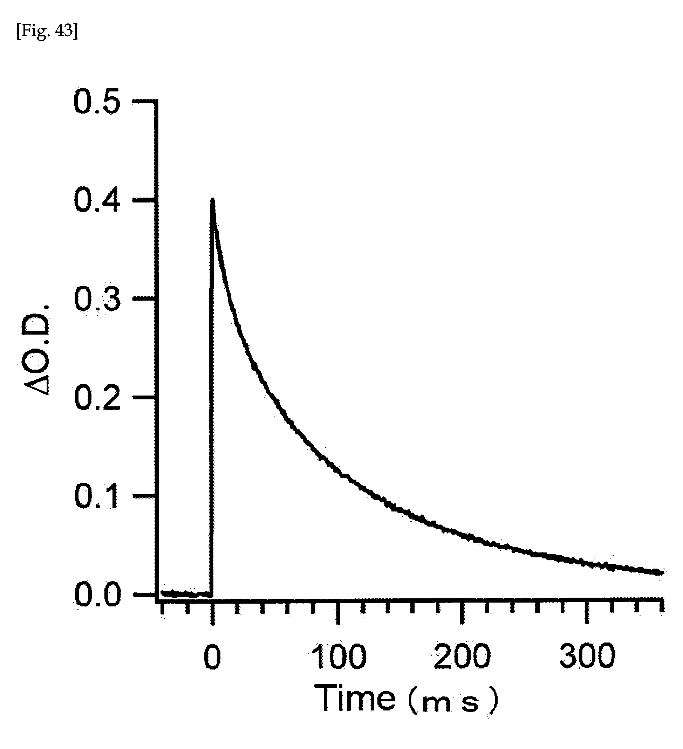

[FIG. 43] A graph showing the results of measurement of decay of the absorption band (400 nm) of a compound of Example 17 in a nanosecond laser flash photolysis measurement.

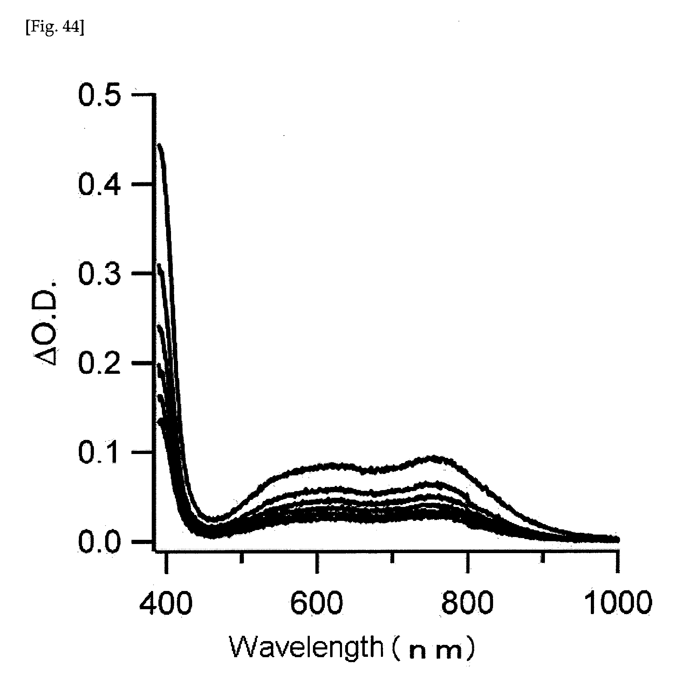

[FIG. 44] A graph showing the results of measurement of visible/near-IR absorption spectra of the compound of Example 17 immediately after irradiation with a nanosecond UV laser in a nanosecond laser flash photolysis measurement.

### [Modes for Carrying Out the Invention]

One embodiment of the bridged hexaarylbisimidazole compound of the present invention is represented by general formula (2) above, and specifically for example (2a).
In the general formulae, the two aryl groups A and B are bridged to each other via carbon atoms by means of a bridging group X (excluding 1,8-naphthalenylene); the number 1 of bridging groups X is preferably 1 to 3, and more preferably 2 or 3, while taking into consideration the number of steps in an organic synthesis, application-dependent photochromic properties, thermal stability, etc. Furthermore, when both A and B are a phenyl group, which is the simplest aryl group, the position to which the bridging group is bonded may be any of the ortho position, meta position, and para position of the two phenyl groups A and B. Moreover, one end or both ends of one bridging group may be bonded to and bridge two or more carbon atoms by means of a bridging group having a branched structure.

With regard to the compound of the present invention, preferably at least one of the substituents R_{A} to R_{F} is not hydrogen. Due to at least one of R_{A} to R_{F} being a substituent other than hydrogen, desired photochromic properties such as tone and density of coloration and color switching response rate can be controlled. It is more preferable to have an asymmetric structure, and this enables these photochromic properties to be controlled precisely. Furthermore, precise control can also be carried out similarly by a multimer structure.

The bridging group of the compound of the present invention may have any structure known to a person skilled in the art as long as it is a group that can link the aryl group A and the aryl group B. It is preferably a bridging group other than an aromatic skeleton, which has high resonance stabilization energy, and more preferably a bridging group that does not conjugate with a triarylimidazolyl radical (TAIR). A bridging group that does not conjugate with a TAIR means a bridging group that does not have a conjugate double bond in a bridging group moiety so that two TAIRs and said bridging group cannot be conjugated to give a resonant structure.

Specific preferred examples of the bridging group of the compound of the present invention include a -CH₂CH₂- group, a -CH₂CH₂CH₂- group, a - CH₂CH₂CH₂CH₂- group, an -Si(CH₃)₂Si(CH₃)₂- group, an -Si(CH₃)₂Si(CH₃)₂Si(CH₃)₂-group, an -Si(CH₃)₂Si(CH₃)₂Si(CH₃)₂Si(CH₃)₂- group, an -SiH₂OSiH₂- group, an - Si(CH₃)₂OSi(CH₃)₂- group, an -Si(CH₃CH₂)₂OSi(CH₂CH₃)₂- group, a -CH₂SCH₂-group, a -CH₂OCH₂- group, an -OCH₂CH₂O- group, a -CH₂OCH₂CH₂OCH₂- group, and a -CH₂COCH₂- group, which are σ bonding, and more preferred examples include a -CH₂CH₂- group, an -Si(CH₃)₂Si(CH₃)₂- group, an -Si(CH₃)₂OSi(CH₃)₂-group, and a -CH₂SCH₂- group. Bridging may be carried out using one or more types of these bridging groups.

When the bridging group X contains a hydrogen atom, the hydrogen atom may be replaced by a substituent Rx. The substituents Rx are independently identical to or different from each other and are substituents selected from a halogen atom, a nitro group, a cyano group, a trifluoromethyl group, a hydroxy group, a thiol group, an amino group, a diphenylamino group, a carbazole group, a straight-chain or branched alkyl group having 1 to 20 carbons, a straight-chain or branched alkylamino group having 1 to 20 carbons, a straight-chain or branched alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other, Y₁ to Y₃ denote a straight-chain or branched alkyl or alkylene group having 1 to 20 carbons, and Z₁ to Z₃ are independently identical to or different from each other and denote a hydrogen atom, a halogen atom, or an alkoxy group having 1 to 8 carbons). Substitution may be carried out using one or more types of these substituents.

By adjusting the distance and angle of the two aryl groups A and B of the compound of the present invention, the distance and the angle of two imidazole rings, the flexibility of the molecule, etc. by means of the number, the type, and the length of these bridging groups, it is possible to adjust photochromic properties such as color switching reaction rate and color density as appropriate according to the intended application of the compound of the present invention.

In general formulae (2) and (2a) above, carbon atoms to which the bridging group X for the two aryl groups A and B is not bonded may mutually independently have substituents R_{A} and R_{B}, the subscripts m and n are mutually independently an integer of 0 to 4, the other four aryl groups C to F may mutually independently have substituents R_{C} to R_{F}, and the subscripts o to r are mutually independently an integer of 0 to 5.

The substituents R_{A} and R_{B} are independently identical to or different from each other and are substituents selected from a halogen atom, a nitro group, a cyano group, a trifluoromethyl group, a hydroxy group, a thiol group, an amino group, a diphenylamino group, a carbazole group, a straight-chain or branched alkyl group having 1 to 20 carbons, a straight-chain or branched alkylamino group having 1 to 20 carbons, a straight-chain or branched alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other, Y₁ to Y₃ denote a straight-chain or branched alkyl or alkylene group having 1 to 20 carbons, and Z₁ to Z₃ are independently identical to or different from each other and denote a hydrogen atom, a halogen atom, or an alkoxy group having 1 to 8 carbons). Substitution may be carried out using one or more types of these substituents.

Furthermore, the substituents R_{C} to R_{F} above are independently identical to or different from each other and are one or more substituents selected from the group consisting of a hydrogen atom, a halogen atom, a nitro group, a cyano group, a trifluoromethyl group, a hydroxy group, a thiol group, an amino group, a diphenylamino group, a carbazole group, a straight-chain or branched alkyl group having 1 to 20 carbons, a straight-chain or branched alkylamino group having 1 to 20 carbons, a straight-chain or branched alkoxy group having 1 to 20 carbons, a -Y₁-SiZ₁Z₂Z₃ group, a -Y₁-SiY₂Z₁Z₂ group, a -Y₁-SiY₂Y₃Z₁ group (here, Y₁ to Y₃ and Z₁ to Z₃ are independently identical to or different from each other, Y₁ to Y₃ denote a straight-chain or branched alkyl or alkylene group having 1 to 20 carbons, and Z₁ to Z₃ denote a hydrogen atom, a halogen atom, or an alkoxy group having 1 to 8 carbons),

a substituent represented by partial structural formula (i) below 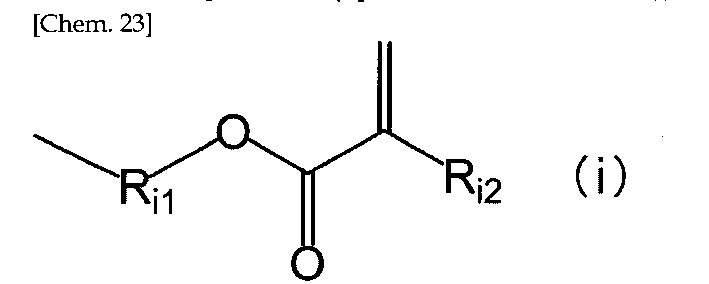 (here, Rᵢ₁ denotes an alkylene or alkoxylene group having 1 to 20 carbons, and Rᵢ₂ denotes hydrogen or an alkyl group having 1 to 3 carbons), and

a substituent represented by partial structural formula (ii) below 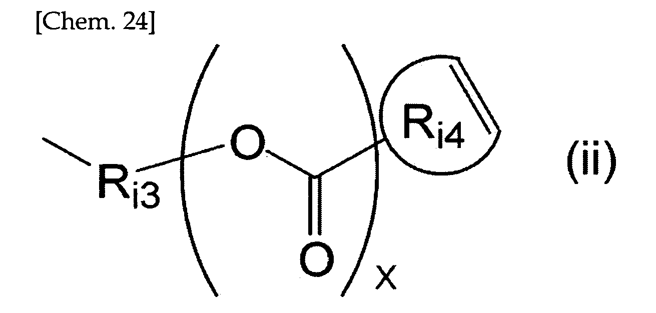 (here, Rᵢ₃ denotes an alkylene or alkoxylene group having 1 to 20 carbons, Rᵢ₄ denotes a cyclic olefin having a total number of carbons and silicons of 5 to 10, and x denotes 0 or 1), and

a substituent selected from substituents represented by partial structural formula (iii) below 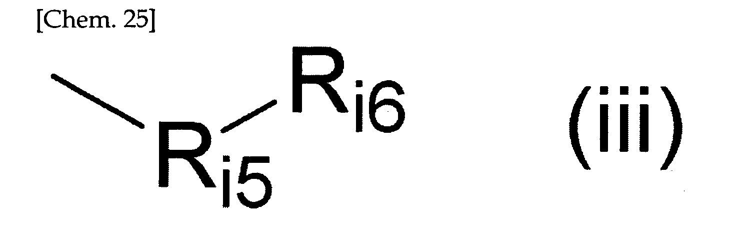 (here, Rᵢ₅ denotes an alkylene or alkoxylene group having 1 to 20 carbons, and Rᵢ₆ denotes an ethylene group or an acetylene group), etc. Substitution may be carried out using one or more types of these substituents. In the cyclic olefin of Rᵢ₄ above, the total number of carbons and silicons is 5 to 10. Therefore, for example, a case in which the number of carbons is 3 and the number of silicons is 3, a case in which the number of carbons is 6 and the number of silicons is 0, etc. are included.

The compound of the present invention is preferably a substituted compound of a bridged hexaarylbisimidazole for which in the general formula above at least one of the substituents R_{A} to R_{F} is not a hydrogen atom, that is, at least one of m to r is not 0. Due to any of the aryl groups A to F having a substituent, compared with an unsubstituted bridged hexaarylbisimidazole compound, desired properties such as tone and density of coloration or light responsiveness can be improved. It is more preferably a bridged hexaarylbisimidazole compound having an asymmetric structure in which the structure of the triarylimidazole moiety containing the aryl group A and the structure of the triarylimidazole moiety containing the aryl group B are different. By combining two triarylimidazolyl radicals (TAIRs) having different structures and different energy levels, absorption wavelengths, etc., it becomes possible to optimally design a molecular structure for the compound of the present invention according to the intended application and to more precisely control photochromic properties such as tone and density of coloration. Therefore, a compound represented by general formula (1) above, which is a compound used for synthesis of, in particular, a bridged hexaarylbisimidazole having an asymmetric structure, is also included in the invention of the present application.

For example, there can be cited an example in which an asymmetric structure is formed by varying from each other the type of substituent (various electron-donating or electron-attracting substituents), the number of substituents (1 to 5), the substituent bonding position (ortho position, meta position and para position of aryl group), etc. for substituents R_{A}, R_{E}, and R_{F} of the triarylimidazole moiety containing the aryl group A and substituents R_{B}, R_{C}, and R_{D} of the triarylimidazole moiety containing the aryl group B, which changes the resonant structure, energy level, absorption wavelength, etc. of the TAIR, and there can be cited an example in which an asymmetric structure is preferably formed by varying from each other the type of substituent, the number of substituents, the substituent bonding position, etc. for the substituents R_{E} and R_{F} and the substituents R_{C} and R_{D} from the viewpoint of the degree of freedom in terms of organic synthetic route design and molecular design.

The substituents R_{A} to R_{F} introduced for the purpose of precise control of photochromic properties are, from the viewpoint of tone/response rate control, preferably substituents selected from an electron-donating group such as a dimethylamino group or a methoxy group, an electron-attracting group such as a nitro group or a cyano group, a straight-chain or branched alkoxy group having 1 to 20 carbons, etc., and are more preferably substituents selected from a methoxy group, a nitro group, etc. Substitution may be carried out using one or more types of these substituents. By appropriately controlling the electron attracting properties and electron donating properties of these substituents, the degree of electron density, stability, etc. of a moiety bonded to an imidazole ring including aryl groups A to F, it becomes possible to appropriately control desired properties such as tone/response rate and color density.

Among the substituents R_{A} to R_{F} of the compound of the present invention, a substituent other than the substituent introduced for the purpose of precise control of photochromic properties and the substituent used in polymerization for the polymer compound and the substituent Rx on the bridging group are preferably selected from straight-chain or branched alkyl groups having 1 to 20 carbons, etc., and are more preferably methyl groups. Substitution may be carried out using one or more types of these substituents. It is also similarly preferable for it to be unsubstituted.

Furthermore, the substituents R_{A} to R_{F} above may form, together with the carbon atom to which the substituent is bonded, the above other substituent, and the carbon atom to which said other substituent is bonded, an aliphatic ring, an aromatic ring, or a hetero ring, the ring may further have another substituent, and said substituent preferably has the same meaning as that of the substituents R_{A} to R_{F}. The two triarylimidazole moieties of the present invention may have mutually asymmetric structures due to these ring structures or substituents.
Moreover, by adjusting the distance and the angle of the two aryl groups A and B, the distance and the angle of the two imidazole rings, the molecular flexibility, etc. by the number of substituents, the type, the structure of an aromatic ring, etc. formed by the substituents on the aryl groups A to F of the compound of the present invention, it is also possible to adjust photochromic properties such as color switching reaction rate or color density appropriately according to the intended application of the compound of the present invention.

Specific examples of the compounds represented by general formulae (2) and (2a) above include *pseudogem*-bisDPI[2.2]paracyclophane, 1,3-bis(triphenylimidazole)-1,1,3,3-tetramethyldisiloxane (bisTPI-TMDS), and derivatives of these compounds.

As another embodiment of the present invention, a multimer compound of a bridged hexaarylbisimidazole compound represented by general formula (3) above, for example, (3b) or (3c) can be cited.
In the general formula and partial structural formula (vi) above, the two aryl groups A and B are bridged to each other via carbon atoms by means of the bridging group X (excluding 1,8-naphthalenylene), and hydrogen atoms of the bridging group X may be mutually independently replaced by one or more of any substituent Rx. The number 1 of bridging groups X is preferably 1 to 3, and more preferably 2 or 3 while taking into consideration the number of steps in an organic synthesis, application-dependent photochromic properties, thermal stability, etc. Furthermore, when A and B are both a phenyl group, which is the simplest aryl group, the position to which the bridging group is bonded may be any of the ortho position, meta position, and para position of the two phenyl groups A and B. Moreover, bridging may be achieved by means of a bridging group having a branched structure, one end or both ends of one bridging group being bonded to two or more carbon atoms.

The bridging group of the compound in this embodiment may have any structure known to a person skilled in the art as long as it is a group that can link the aryl group A and the aryl group B. It is preferably a bridging group other than an aromatic skeleton, which has high resonance stabilization energy, and is more preferably a bridging group that does not conjugate with a triarylimidazolyl radical (TAIR). A bridging group that does not conjugate with a TAIR means a bridging group that does not have a conjugate double bond in a bridging group moiety so that two TAIRs and said bridging group cannot be conjugated to give a resonant structure.

Specific preferred examples of the bridging group of the compound of this embodiment include a -CH₂CH₂- group, a -CH₂CH₂CH₂- group, a -CH₂CH₂CH₂CH₂-group, an -Si(CH₃)₂Si(CH₃)₂- group, an -Si(CH₃)₂Si(CH₃)₂Si(CH₃)₂- group, an - Si(CH₃)₂Si(CH₃)₂Si(CH₃)₂Si(CH₃)₂- group, an -SiH₂OSiH₂- group, an - Si(CH₃)₂OSi(CH₃)₂- group, an -Si(CH₃CH₂)₂OSi(CH₂CH₃)₂- group, a -CH₂SCH₂-group, a -CH₂OCH₂- group, an -OCH₂CH₂O- group, a -CH₂OCH₂CH₂OCH₂- group, and a -CH₂COCH₂- group, which are o bonding, and more preferred examples include a -CH₂CH₂- group, an -Si(CH₃)₂Si(CH₃)₂- group, an -Si(CH₃)₂OSi(CH₃)₂-group, and a -CH₂SCH₂- group. Bridging may be carried out using one or more types of these bridging groups.
By adjusting the distance and angle of the two aryl groups A and B of the compound of the present invention, the distance and the angle of the two imidazole rings, the flexibility of the molecule, etc. by means of the number, the type, and the length of these bridging groups, it is possible to adjust photochromic properties such as color switching reaction rate or color density as appropriate according to the intended application of the compound of the present invention.

In general formula (3) above, a number α of structural units containing one bisimidazole skeleton that are bound is an integer of 1 to 9; from the viewpoint of organic synthetic route design, steric hindrance of the compound, etc. it is preferably an integer of 1 to 6, and more preferably an integer of 1 to 4. Furthermore, from the viewpoint of color density per unit molarity, etc., it is preferably an integer of 2 to 8, more preferably an integer of 2 to 6, and yet more preferably an integer of 2 to 4.
Moreover, the linking group L is a monocyclic or polycyclic aromatic compound containing 1 to 12 aromatic rings having 5 to 8 carbon atoms per ring structure, and from the viewpoint of organic synthetic route design, steric hindrance of the compound, etc. the number of carbon atoms per ring structure is preferably 6 and the number of aromatic rings is preferably 1 to 8, and more preferably 1 to 4.
Provided that there is at least one carbon atom as a ring member, the aromatic ring may be a heterocycle in which one or more carbon atoms are replaced by an oxygen atom, a nitrogen atom, a sulfur atom, etc. or may be an aliphatic ring having 5 to 8 carbon atoms instead of the aromatic ring, and an atom that is not bonded to the structural unit containing one bisimidazole skeleton may be substituted with one or more of any substituent R_{L}. Said any substituent R_{L} has the same meaning as that of the substituents Rc to R_{F}.

In general formulae (3), (3b), and (3c) and the partial structural formulae (vi) and (vii), carbon atoms to which the bridging group X of the two aryl groups A and B are not bonded in the bisimidazole skeleton-containing structural unit may mutually independently have substituents R_{A} and R_{B}, subscripts m and n are mutually independently an integer of 0 to 4, the other three aryl groups C to E in the structural unit may mutually independently have substituents Rc to R_{E}, and subscripts o to q are mutually independently an integer of 0 to 5.
Furthermore, carbon atoms on an aromatic ring of the linking group L to which the structural unit is not bonded, the linking group L being a monocyclic or polycyclic aromatic compound, may have one or more substituents R_{L} depending on the molecular structure of the compound of the present invention, for example, a substituent R_{F}, and subscripts r are mutually independently an integer of 0 to 4.
Moreover, the carbon atom, silicon atom, etc. of the bridging group X may have one or more substituents Rx depending on the molecular structure of the compound of the present invention.

All of the substituents R_{A}, R_{B}, and Rx (including the difference in symbol subscripts) of the aryl groups A and B and the bridging group X in the bisimidazole skeleton-containing structural unit may be mutually independently selected from identical or different substituents in the range defined above (hydrogen atom being included for partial structural formula (vii)), and are more preferably selected from a methyl group (hydrogen atom or methyl group for partial structural formula (vii)). Substitution may be carried out using one or more types of these substituents.

Furthermore, the substituent may form, together with the carbon atom to which the substituent is bonded, the above other substituent, and the carbon atom to which said other substituent is bonded, an aliphatic ring, an aromatic ring, or a hetero ring, and the ring may further have another substituent. This substituent preferably has the same meaning as that of Rc to R_{F} of general formula (2).
Moreover, the structure of the triarylimidazole moiety containing the aryl group A and the structure of the triarylimidazole moiety containing the aryl group B contained in the compound of the present embodiment may be identical or different due to different substituents being bonded. By combining two TAIRs having different structures and different absorption wavelengths, etc., according to the intended application of the compound of the present invention, it is also possible to precisely control photochromic properties such as tone and density of coloration.
Furthermore, by adjusting the distance and angle of the two aryl groups A and B, the distance and angle of the two imidazole rings, the flexibility of the molecule, the absorption wavelength of the molecule, the distance between hexaarylimidazolyl radical (HAIR) structures contained in the compound of the present embodiment, and the overall structure of a multimer molecule by means of the number and type of substituents on the linking group L and the five aryl groups A to E in the HAIR structure and the structure of the aromatic ring, etc. formed by the substituents, it is possible to adjust photochromic properties such as color switching reaction rate or color density as appropriate according to the intended application of the compound of the present embodiment.

Specific preferred examples of the compounds represented by general formulae (3), (3b), and (3c) above include a multimer compound such as a dimer to a nonamer of *pseudogem*-bisDPI[2.2]paracyclophane, 1,3-bis(triphenylimidazole)-1,1,3,3-tetramethyldisiloxane (bisTPI-TMDS), etc.
Furthermore, the compound of the present invention may be formed by further polymerizing a plurality of multimer compounds represented by the above-mentioned general formulae, and examples thereof include a multimer compound such as a tetramer to a nonamer formed by polymerizing two or three dimers or trimers of *pseudogem*-bisDPI[2.2]paracyclophane.

Specific compounds represented by the above-mentioned general formulae, etc., where the effects of the present invention are anticipated are exemplified below, but the compounds of the invention of the present application are not limited to these compounds.

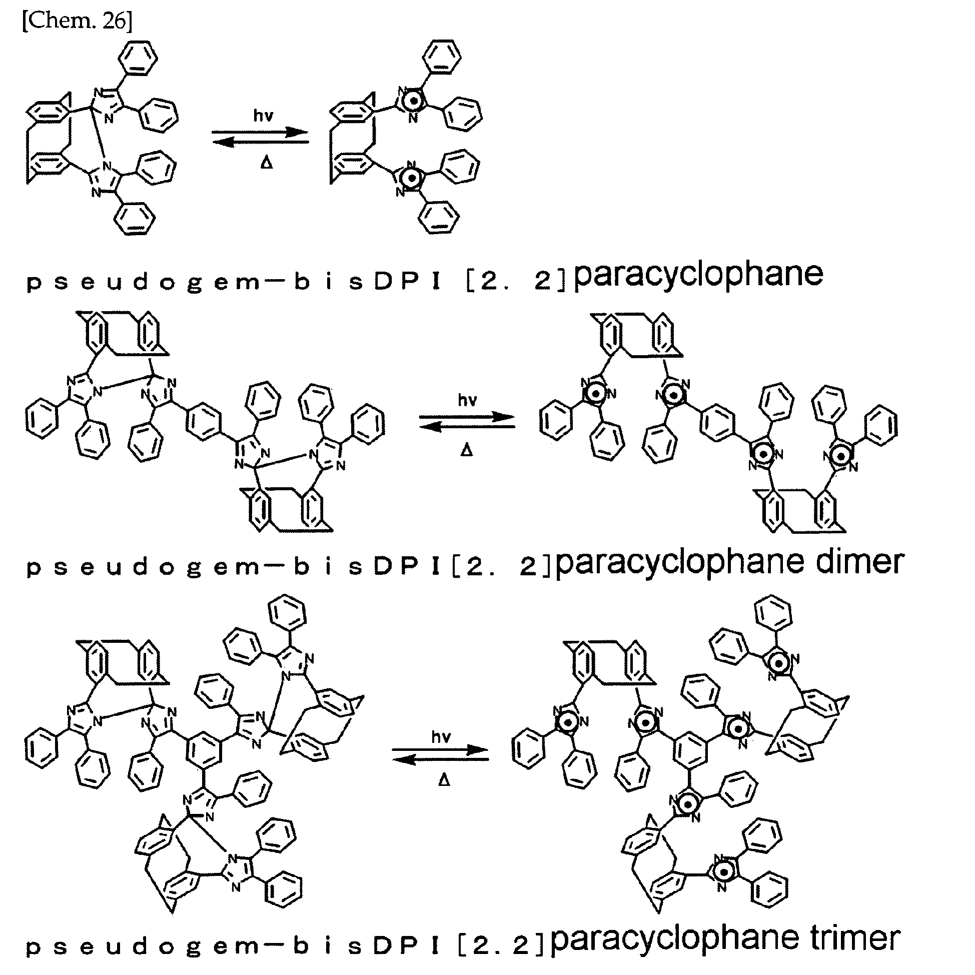

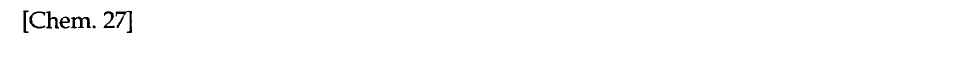

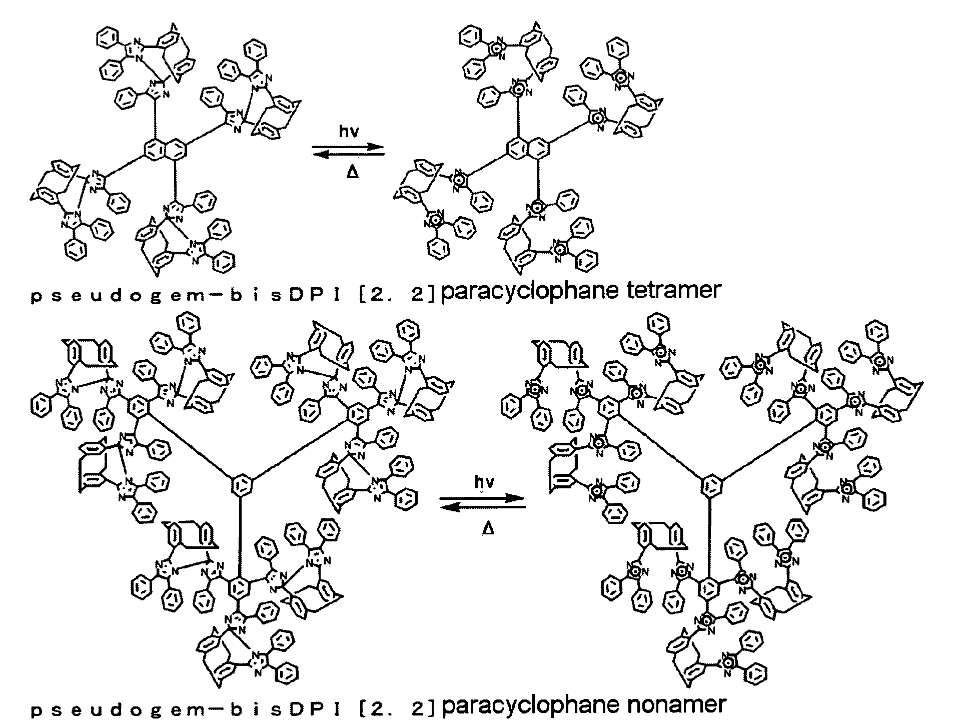

Furthermore, the monomer or multimer, which is the compound of the present invention, may be formed as a chain-like or net-like polymer compound by introducing the compound of the present invention as a functional moiety into a polymer compound by condensation-polymerization, etc. of two or one polymerizable substituents selected from the substituents Rc to R_{F} and two or one polymerizable functional groups contained in a polymer main chain and/or side chain of the polymer compound, or by carrying out radical polymerization, etc. of the compound of the present invention having two or more polymerizable substituents selected from the substituents Rc to R_{F}.

Preferred examples of the polymerizable substituents Rc to R_{F} in this case include substituents selected from a hydroxy group, an amino group, a carboxyl group, an isocyanate group, a halogen group, an azido group, a vinyl group, an ethynyl group, a norbornene group, and an acrylate or methacrylate ester such as a butyl methacrylate group, a butyl acrylate group, or a propoxy methacrylate group, represented by partial structural formulae (viii) below, 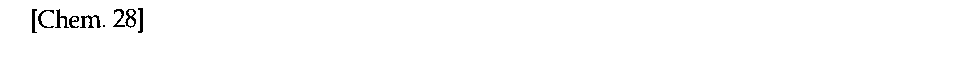 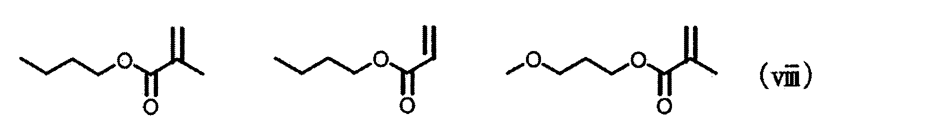 and more preferred examples include a substituent selected from a hydroxy group, a butyl methacrylate group, etc. Substitution may be carried out using one or more types of these substituents.

As hereinbefore described, the polymer compound of the present invention is a polymer compound having a repeating structural unit represented by partial structural formula (iv) below

[Chem. 29] -[(A)_{γ}-(B)_{δ}]_{ε}- (iv)

and/ or partial structural formula (v) below. 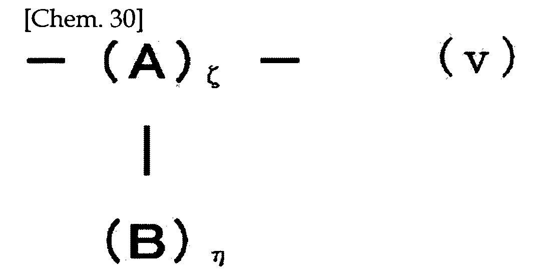 Specifically, A is any linking group containing one or more atoms selected from the group consisting of carbon, nitrogen, and oxygen atoms, and may be any linking group known to a person skilled in the art as a linking group that can be polymerized with the substituents R_{C} to R_{F}. It is preferably styrene, an methacrylate ester such as methyl methacrylate, or an acrylate ester such as methyl acrylate. Examples include a repeating structural unit in which B is a derivative of the bridged hexaarylimidazole of the present invention, A-B denotes a bond between the linking group and two or one substituents selected from the substituents R_{C} to R_{F} of the bridged hexaarylimidazole derivative, Υ is an integer of 0 or greater, and δ, ε, ζ, and η are mutually independently an integer of 1 or greater. The repeating unit may be formed by repeating a single partial structural formula (iv) or (v) or may be formed by repeating a combination as in for example partial structural formula (ix).

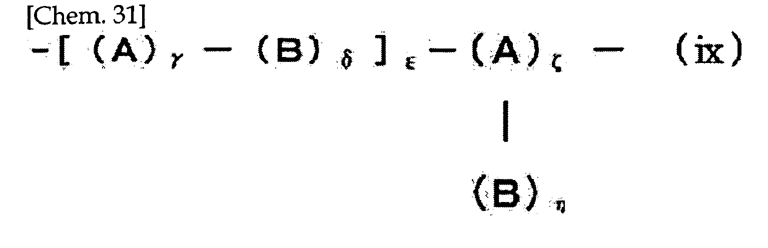

It is possible to introduce the compound of the present invention into a polymer compound as a functional moiety by condensation-polymerization, etc. of two or one polymerizable substituents such as a hydroxy group selected from the substituents R_{C} to R_{F} of the bridged hexaarylimidazole derivative and two or one polymerizable linking groups such as a carboxyl group contained in a polymer main chain or side chain of the polymer compound.

The photochromic material containing a compound represented by general formula (2) above, preferably (2a), or (3), preferably (3b) and (3c), of the present invention is a material that contains the bridged hexaarylbisimidazole compound of the present invention, the asymmetric bridged hexaarylbisimidazole compound, and/or the multimer compound such as a dimer, a trimer, or a tetramer of these compounds and that reversibly changes color by heating or irradiation with electromagnetic waves such as UV light or visible light, and is preferably a material that changes color by irradiation with UV light and/or visible light. Here, 'containing' means both a case in which the compound of the present invention is contained in the composition as an added component and a case in which the compound of the present invention is chemically bonded to a molecular structure of a solvent, a resin, etc. by means of a covalent bond, a coordination bond, etc.

The compound of the present invention may be mixed with a predetermined solvent since it has rapid color switching characteristics and high color density in a solvent. Preferred examples of the solvent that is mixed include benzene, toluene, chloroform, and methylene chloride, and among them, from the viewpoint of stability of the colored form, benzene and toluene are more preferable. These solvents may be used as a mixture of two or more types.

The compound of the present invention may be mixed with a solid such as a predetermined resin or glass since it has rapid color switching characteristics and high color density even in a solid phase such as a glass or a resin such as a plastic material, or may be chemically bonded to a resin main chain, etc. as a functional moiety. Preferred examples of the resin that is mixed, etc. include polymethyl methacrylate, polystyrene, polyimide, Teflon (registered trademark), and polycarbonate, and among them, from the viewpoint of stability of a colored form, polymethyl methacrylate, Teflon (registered trademark), and polycarbonate are more preferable.

Examples of applications of the compound of the present invention and the photochromic material, solvent, and resin containing the compound include light control materials for sunglasses whose lens color changes in response to sunlight, sun visors, T-shirts, and accessories and, as well as UV ray checkers, holographic materials, ink materials such as security inks, optical information display devices, optical switch elements, and photoresist materials.

The compound of the present invention in one embodiment is a photochromic compound that has in particular the merit of rapid decoloration properties, and enables the photochromic property of color visually disappearing at the same time as irradiation with light stops to be realized.
With regard to the decoloration speed of the compound of the present invention, for example, a solution (concentration 2.1 × 10⁻⁴ mol/L) using benzene as a solvent is measured by a nanosecond laser flash photolysis measurement method, which is described later, and the half life of the colored form is preferably in the range of 1 to 200 ms, more preferably 1 to 100 ms, and yet more preferably 1 to 40 ms.

Furthermore, the compound of the present invention exhibits high color density compared with a conventional photochromic compound. In particular, a multimer compound such as a dimer, trimer, or tetramer in which a plurality of bisimidazole skeleton-containing structural units are polymerized has very high color density per unit molarity.
With regard to the color density of the compound of the present invention, for example, a solution (concentration 2.1 × 10⁻⁴ mol/L) using benzene as a solvent is measured by a nanosecond laser flash photolysis measurement method, which is described later, and the optical density (ΔO.D.) in the visible light region is a value of 0.01 or greater, preferably in the range of 0.01 to 1.0, more preferably 0.1 to 1.0, and yet more preferably 0.5 to 1.0.

The method for producing the compound of the present invention is not particularly limited, and synthesis may be carried out using a known method as appropriate.
For example, as a synthetic route for the above *pseudogem-*bisDPI[2.2]paracyclophane, a synthetic route in which [2.2]paracyclophane-4,13-dicarbaldehyde, etc., which is a dialdehyde, is prepared and reacted with any 1,2-diketone-containing benzil derivative, can be considered (ref. Psiorz, M. et al. Chem. Ber., 1987, 120, 1825.; Hopf, H. et al. Eur. J. Org. Chem., 2002, 2298. Hopf, H. et al. Eur. J., 2005, 11, 6944., etc.).

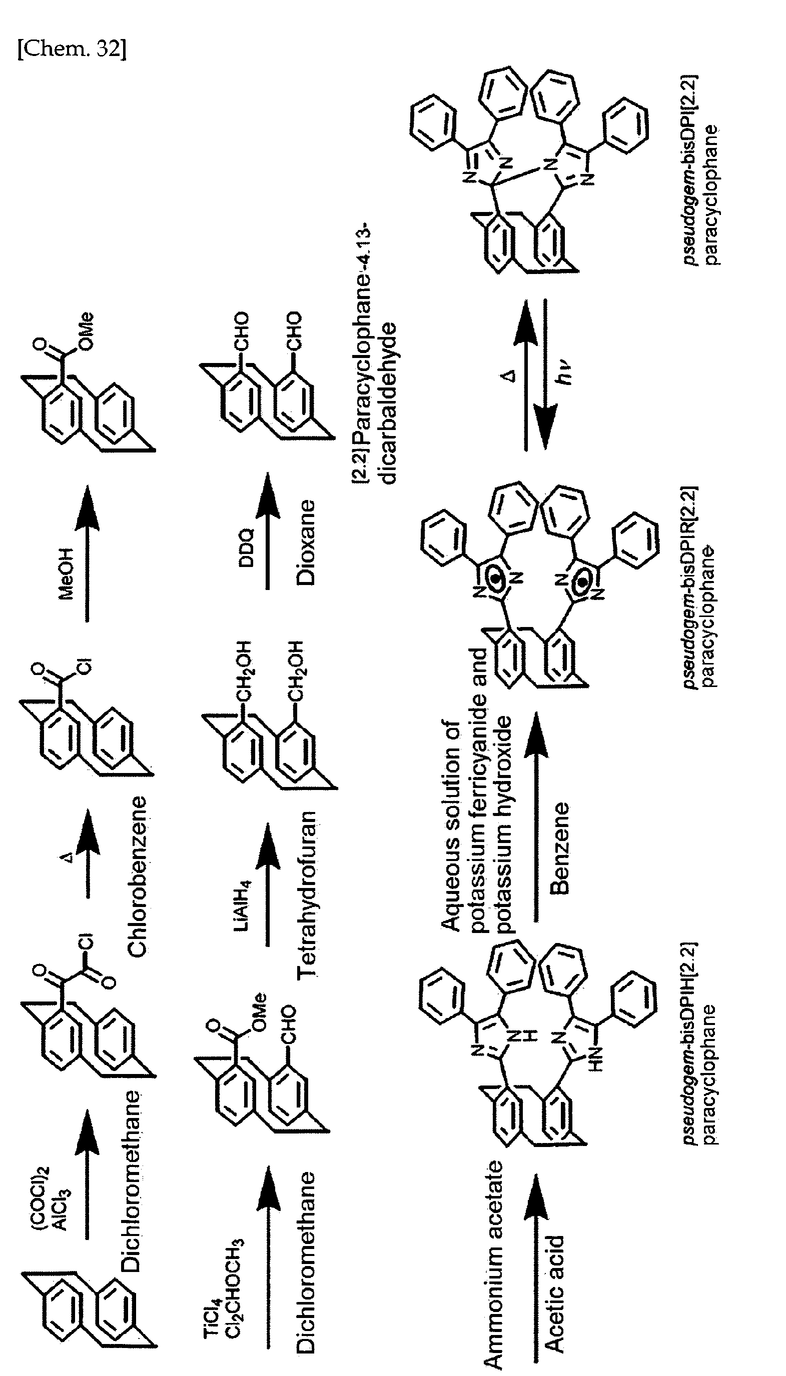

Conventionally, the compound of the present invention is synthesized using the method above or a modified method thereof, and since such a synthetic method employs a target dicarbaldehyde compound such as for example [2.2]paracyclophane-4,13-dicarbaldehyde as an intermediate, it is only possible to synthesize a symmetrical compound in which the structures of triarylimidazoles containing aryl groups A and B are the same for the compound of the present application, which is the final product. While carrying out an intensive investigation, the present inventors have succeeded in synthesizing a novel compound represented by general formula (1) above, and have also succeeded in synthesizing an asymmetric compound of the present application by use of the above compound in synthesis of the compound of the present application. The present invention therefore includes a compound represented by general formula (1) above.
In general formula (1) above, the bridging group X, substituents R_{A} to R_{D}, and subscripts m to p respectively have the same meanings as those of the bridging group X, the substituents R_{A} to R_{D}, and the subscripts m to r defined for general formula (2), etc. above.

The compound of the present invention may be produced, other than by the above-mentioned known method, by using as a key compound an imidazole skeleton-containing precursor compound, which is a monoaldehyde represented by general formula (1) above, and reacting the compound with any 1,2-diketone-containing benzil derivative represented by general formula (4) above. In general formula (4) above, the substituents R_{E} and R_{F} and subscripts q and r of said any 1,2-diketone-containing benzil derivative respectively have the same meanings as those of the substituents R_{A} to R_{F} and the subscripts m to r defined for general formula (2), etc. above.

In accordance with this method, compared with a conventional synthetic method, etc. in which [2.2]paracyclophane-4,13-dicarbaldehyde, etc., which is a dialdehyde, is prepared and reacted with any 1,2-diketone-containing benzil derivative, the degree of freedom of molecular structure design and synthesis of a photochromic compound can be increased greatly, and it becomes possible to synthesize photochromic compounds having various structures such as a bridged hexaarylbisimidazole compound, a bridged hexaarylbisimidazole compound having an asymmetric structure, and multimer compounds of these compounds formed by polymerizing a plurality of bisimidazole skeleton-containing structural units.

Furthermore, the method for producing a multimer compound of the present invention is a production method in which an imidazole skeleton-containing precursor compound, which is a monoaldehyde represented by general formula (1) above, is reacted as a key compound with any benzil derivative formed by polymerization of a plurality of 1,2-diketone-containing structural units represented by general formula (5) above. In general formula (5) above, the bonding number β, the linking group M, the substituent R_{E}, and the subscript q of 1,2-diketone-containing structural units respectively have the same meanings as those of the bonding number α, the linking group L, the substituent R_{E}, and the subscript q defined for general formula (3), etc. above.

This enables the degree of freedom of molecular structure design and synthesis of a photochromic compound to be increased greatly compared with a conventional synthetic method, etc. in which [2.2]paracyclophane-4,13-dicarbaldehyde, etc., which is a dialdehyde, is prepared and then reacted with any 1,2-diketone-containing benzil derivative, and it is possible to synthesize photochromic compounds having various structures such as a multimer compound of the above compound formed by polymerizing a plurality of bisimidazole skeleton-containing structural units.

### [Examples]

The present invention is more specifically explained below by reference to Examples and Comparative Examples, but the present invention is not limited to these Examples and may be modified in a variety of ways as long as the modifications do not depart from the technical spirit and scope of the present invention.

### [Example 1]

### Synthesis of pseudogem-bisDPI[2.2]paracyclophane

[2.2]Paracyclophane-4,13-(4,5-diphenyl-1H-imidazol-yl) (hereinafter, also called '*pseudogem*-bisDPIH[2.2]paracyclophane'), which is a precursor of *pseudogem-*bisDPI[2.2]paracyclophane, which is a photochromic compound of the present invention, can be produced by heating and stirring [2.2]paracyclophane-4,13-dicarbaldehyde and any benzil derivative in acetic acid in the presence of ammonium acetate. The reaction formula is shown below.

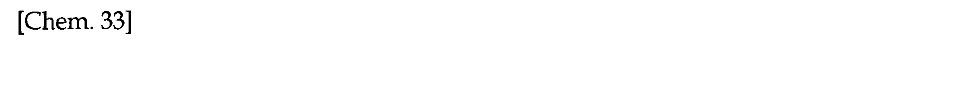

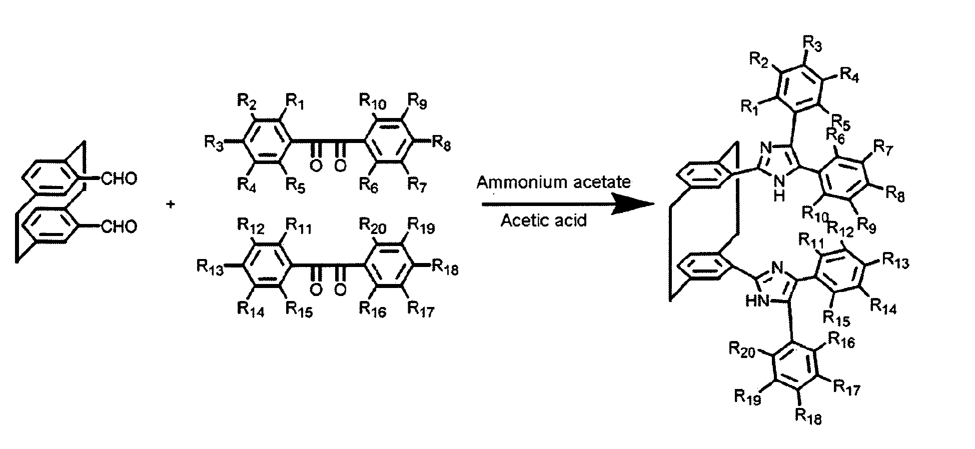

To a 25 mL recovery flask were added 56.1 mg (0.212 mmol) of [2.2]paracyclophane-4,13-dicarbaldehyde, 90 mg (0.428 mmol) of benzil, 412 mg (5.35 mmol) of ammonium acetate, and 2 mL of acetic acid, and heating at 90°C while stirring was carried out for 2 days. After the heating and stirring was completed, the interior of the system was cooled to 0°C, and aqueous ammonia was added until the pH became about 6. When aqueous ammonia was added, a white precipitate was formed together with white smoke. The white precipitate thus obtained was filtered and washed with ion exchanged water. After drying, recrystallization was carried out using ethanol, and 82.9 mg (0.129 mmol) of *pseudogem-*bisDPIH[2.2]paracyclophane was obtained as white acicular crystals at a yield of 60.8%. The results of NMR measurement are shown below.

¹H NMR (500 MHz, DMSO-d₆): δ = 11.65 (s, 2H), 7.30-7.00 (m, 22H), 6.71 (d, 2H), 6.64-6.59 (dd, 2H), 4.59-4.50 (m, 2H), 3.16-3.01 (m, 6H)

Subsequently, to a 100 mL nitrogen-flushed recovery flask was added 44.0 mg (0.0682 mmol) of *pseudogem*-bisDPIH[2.2]paracyclophane, which was dissolved in 40 mL of benzene. An aqueous solution of 1.26g (22.5 mmol) of potassium hydroxide and 2.80 g (11.1 mmol) of potassium ferricyanide in 30 mL of ion exchanged water was added dropwise thereto over 10 minutes, and stirring was carried out at room temperature for 2 hours. After stirring was completed, extraction was carried out using benzene, washing was carried out using ion exchanged water, and the solution was dried using sodium sulfate. Recrystallization was carried out using ethanol, and 40.0 mg of *pseudogem*-bisDPI[2.2]paracyclophane was obtained as white acicular crystals. The results of NMR measurement are shown below.

¹H NMR (500 MHz, CD₃CN): δ = 7.57-7.49 (m, 2H), 7.45-7.36 (m, 3H), 7.32-7.17 (m, 9H), 7.14-7.02 (m, 7H), 6.80 (s, 1H), 6.71 (d, 2H), 6.56-6.48 (m, 2H), 4.49-4.37 (m, 1H), 3.35-2.91 (m, 7H)

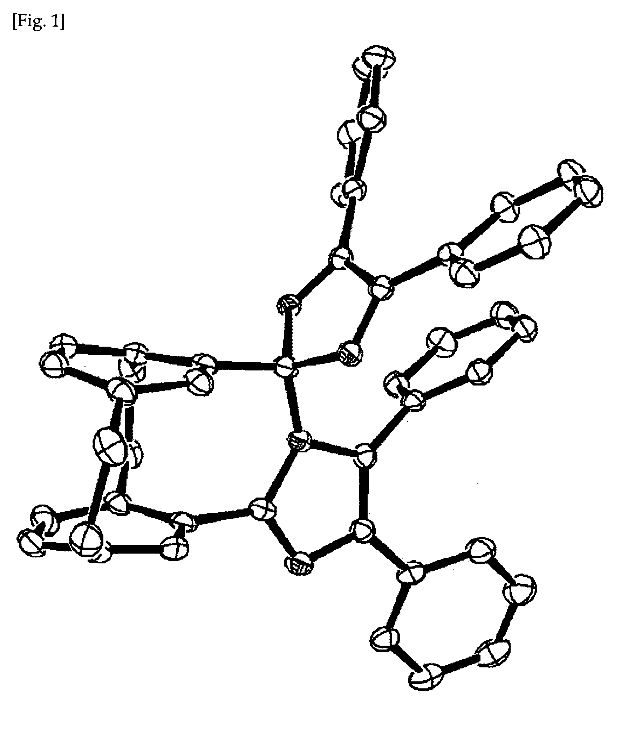

Crystal structural analysis of the *pseudogem*-bisDPI[2.2]paracyclophane thus synthesized was carried out using CCD-equipped single crystal X-ray structural analysis equipment (SMART APEX II, Bruker AXS K.K.). The molecular structure elucidated by the analysis is shown in FIG.1.

### Nanosecond laser flash photolysis measurement of pseudogem-bisDPI[2.2]paracyclophane

Laser flash photolysis measurement of the *pseudogem-*bisDPI[2.2]paracyclophane thus synthesized and 1,8-NDPI-TPI-naphthalene, which was prepared as Comparative Example 1 by introducing two TAIRs at the 1- and 8-positions of naphthalene, was carried out using a time-resolved spectrometer (model TSP-1000, UNISOKU Co., Ltd.). Nanosecond laser flash photolysis measurement of a *pseudogem*-bisDPI[2.2]paracyclophane benzene solution (concentration 2.1 × 10⁻⁴ mol/L) was carried out using a quartz spectroscopic cell having an optical path length of 10 mm under an atmosphere of argon at 25°C.

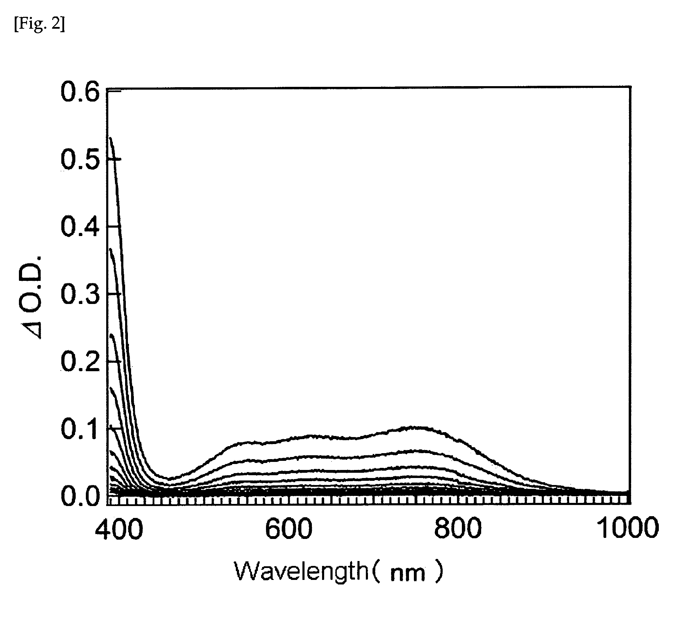

FIG. 2 shows the results of measurement of visible/near-IR absorption spectra in which measurement was carried out at 20 ms intervals by a time-resolved spectrometer immediately after a benzene solution of *pseudogem-*bisDPI[2.2]paracyclophane was irradiated with a nanosecond UV laser having a wavelength of 355 nm (pulse width: 5 ns, output: 8 mJ). It was confirmed from the results of FIG. 2 that, with regard to the *pseudogem*-bisDPI[2.2]paracyclophane of Example 1, a strong absorption band at around 400 nm and a weak broad absorption band in a wide range from 500 nm to 800 nm reversibly appeared on irradiation with UV light.

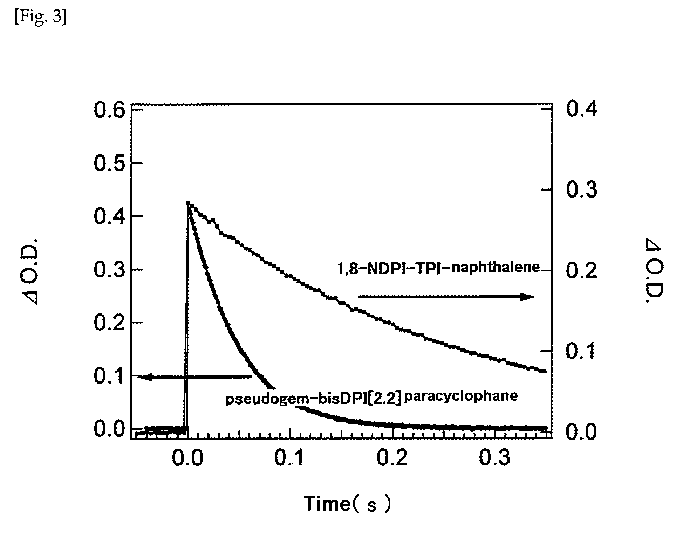

Furthermore, FIG. 3 shows the results of measurement of decay over time of the absorption band at 400 nm that appeared at the same time as the benzene solution of *pseudogem*-bisDPI[2.2]paracyclophane was irradiated with a nanosecond UV laser having a wavelength of 355 nm (pulse width: 5 ns, output: 8 mJ). It was confirmed from the results of FIG. 3 that, with regard to the *pseudogem-*bisDPI[2.2]paracyclophane of Example 1, the absorption band that had appeared on irradiation with the nanosecond UV laser decayed quickly after stopping irradiation with the nanosecond UV laser with a half life of 33 ms at 25°C.

Moreover, in order to examine photochromic behavior in a polar solvent, laser flash photolysis measurement of a dichloromethane solution of *pseudogem-*bisDPI[2.2]paracyclophane (concentration 2.1 × 10⁻⁴ mol/L) was carried out in the same manner.

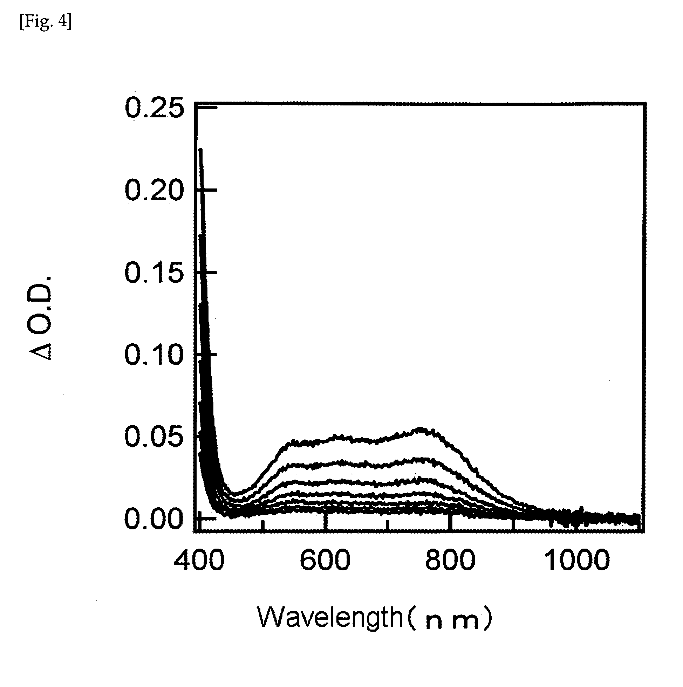

FIG. 4 shows the result of measurement of visible/near-IR absorption spectra in which measurement was carried out at 6 ms intervals by a time-resolved spectrometer immediately after a dichloromethane solution of *pseudogem-*bisDPI[2.2]paracyclophane was irradiated with a nanosecond UV laser having a wavelength of 355 nm (pulse width: 5 ns, output: 8 mJ). It was confirmed from the results of FIG. 4 that in the solution in dichloromethane, which is a polar solvent, in the same way as for the benzene solution above, a strong absorption band at around 400 nm and a weak broad absorption band in a wide range from 500 nm to 800 nm also reversibly appeared on irradiation with UV light.

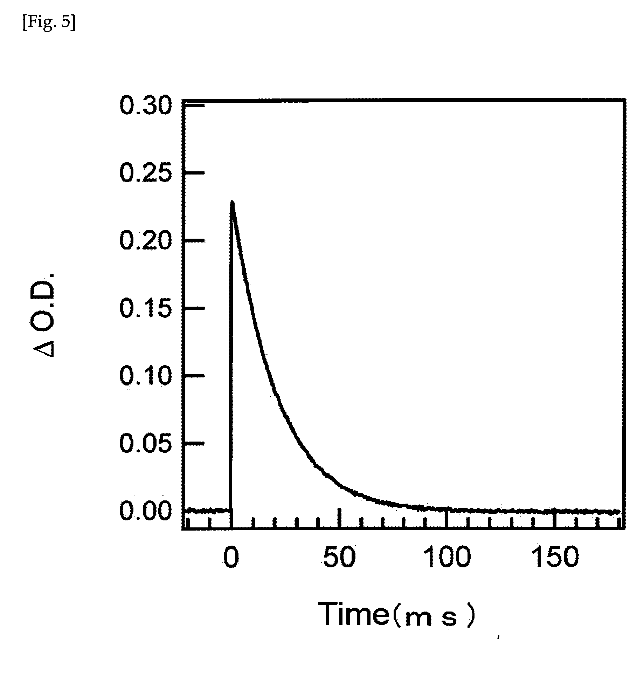

Furthermore, FIG. 5 shows the results of measurement of decay over time of the absorption band at 400 nm that appeared at the same time as the dichloromethane solution of *pseudogem*-bisDPI[2.2]paracyclophane was irradiated with a nanosecond UV laser having a wavelength of 355 nm (pulse width: 5 ns, output: 8 mJ). It was confirmed from the results of FIG. 5 that, in the solution in dichloromethane, which is a polar solvent, the absorption band that had appeared on irradiation with the nanosecond UV laser decayed quickly after stopping irradiation with the nanosecond UV laser with a half life of 15 ms at 25°C.

On the other hand, with regard to a benzene solution of the 1,8-NDPI-TPI-naphthalene of Comparative Example 1 (concentration 9.2 × 10⁻⁵ mol/L), the half life at 25°C was about 179 ms, and it was thus found that the compound of the present invention of Example 1 had a very high decoloration speed that was about 5 times that of the compound of Comparative Example 1. That is, this shows that *pseudogem-*bisDPI[2.2]paracyclophane of the present invention is a photochromic compound having rapid color switching characteristics since the two TAIRs generated are restrained by a bridging group, thus preventing diffusion in a medium, and the two TAIRs and the bridging group do not form a resonant structure that is more stable than necessary.

### [Example 2]

### Nanosecond laser flash photolysis measurement of pseudogem-bisDPI[2.2]paracyclophane-containing PMMA

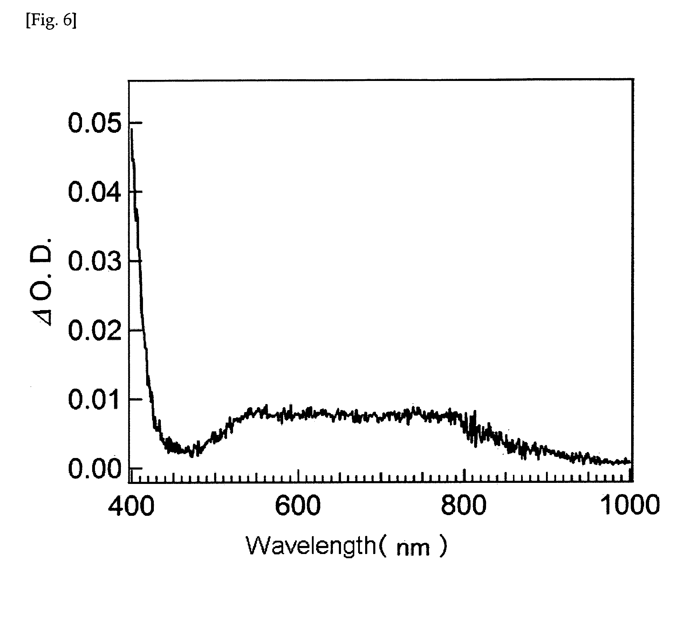

A solution (concentration 20 wt%) was prepared by dissolving 19.8 mg of polymethyl methacrylate (PMMA) (Aldrich, molecular weight 350,000) in 0.4 mL of chloroform solvent and dissolving therein 4.0 mg of the synthesized *pseudogem-*bisDPI[2.2]paracyclophane. A 200 µm thick PMMA thin film containing *pseudogem-*bisDPI[2.2]paracyclophane was prepared by a casting method using the above solution. Nanosecond laser flash photolysis measurement of the PMMA thin film was carried out at 25°C using a nanosecond UV laser having a wavelength of 355 nm (pulse width: 5 ns, output: 4 mJ). The result of measurement of a visible/near-IR absorption spectrum immediately after irradiation with the nanosecond UV laser is shown in FIG. 6.

It was confirmed from the result of FIG. 6 that, with regard to *pseudogem-*bisDPI[2.2]paracyclophane of the present invention, a strong absorption band at around 400 nm and a weak broad absorption band in a wide range from 500 nm to 800 nm also reversibly appeared in a solid-phase PMMA resin in the same way as for the benzene solution. Furthermore, it was confirmed from this nanosecond laser flash photolysis measurement that, with regard to *pseudogem-*bisDPI[2.2]paracyclophane of the present invention, the absorption band thus formed also decayed quickly in the solid-phase PMMA resin with a half life of about 13 ms at 25°C.

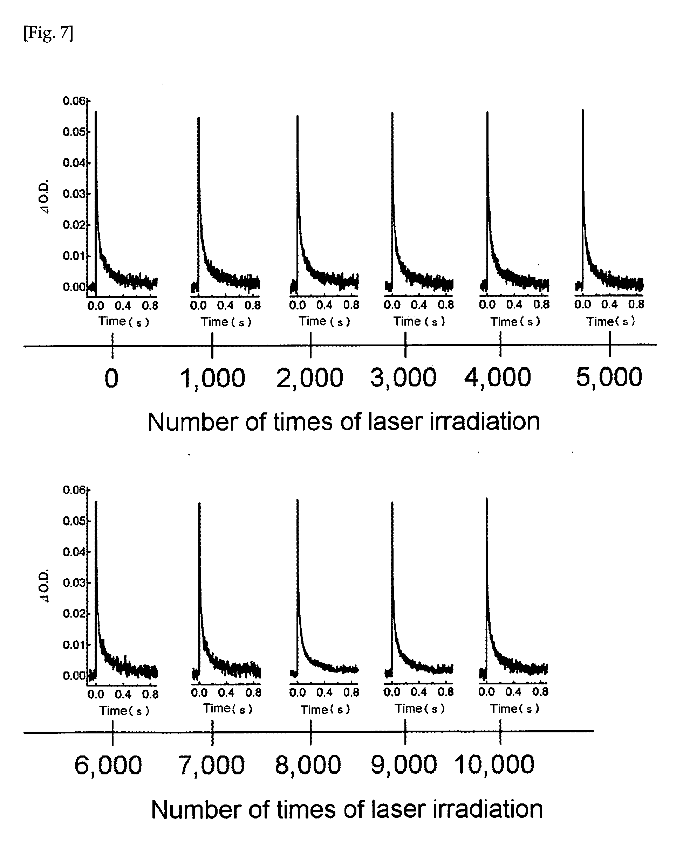

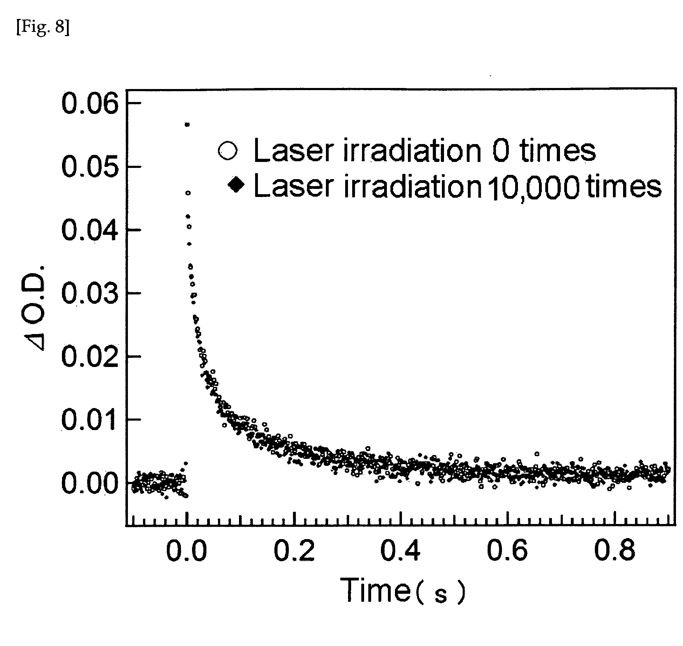

Subsequently, a durability test in which the same portion of the PMMA thin film was irradiated with a nanosecond UV laser having a wavelength of 355 nm (pulse width: 5 ns, output: 4 mJ) 10,000 times at intervals of 1 second was carried out at 25°C. FIG. 7 shows the results of measurement of decay over time of the absorption band at 400 nm measured before irradiation with the nanosecond UV laser and after every 1,000 times of irradiation with the nanosecond UV laser. FIG. 8 shows a comparison of the results of measurement of decay over time before irradiation with a nanosecond UV laser (0^{th} time of laser irradiation) and after irradiation with a nanosecond UV laser 10,000 times.

It was found from the results of FIG. 7 and FIG. 8 that the way in which the absorption band decayed over time did not change even upon irradiating 10,000 times with the nanosecond UV laser, and the measurement sample was not degraded. That is, it is shown that *pseudogem*-bisDPI[2.2]paracyclophane of the present invention is a photochromic compound having very high repetition durability since the two TAIRs generated are restrained by the bridging group, thus preventing diffusion in a medium.

### [Example 3]

### Synthesis of 1,3-bis(triphenylimidazole)-1,1,3,3-tetramethyldisiloxane

1.3-BisTPIH-1,1,3,3-tetramethyldisfloxane (hereinafter, also called 'bisTPIH-TMDS'), which is a precursor of 1,3-bis(triphenylimidazole)-1,1,3,3-tetramethyldisiloxane (hereinafter, also called 'bisTPI-TMDS'), which is a photochromic compound of the present invention, was synthesized by the synthetic route below using 2-(4-bromophenyl)-1,3-dioxolane as a starting material.

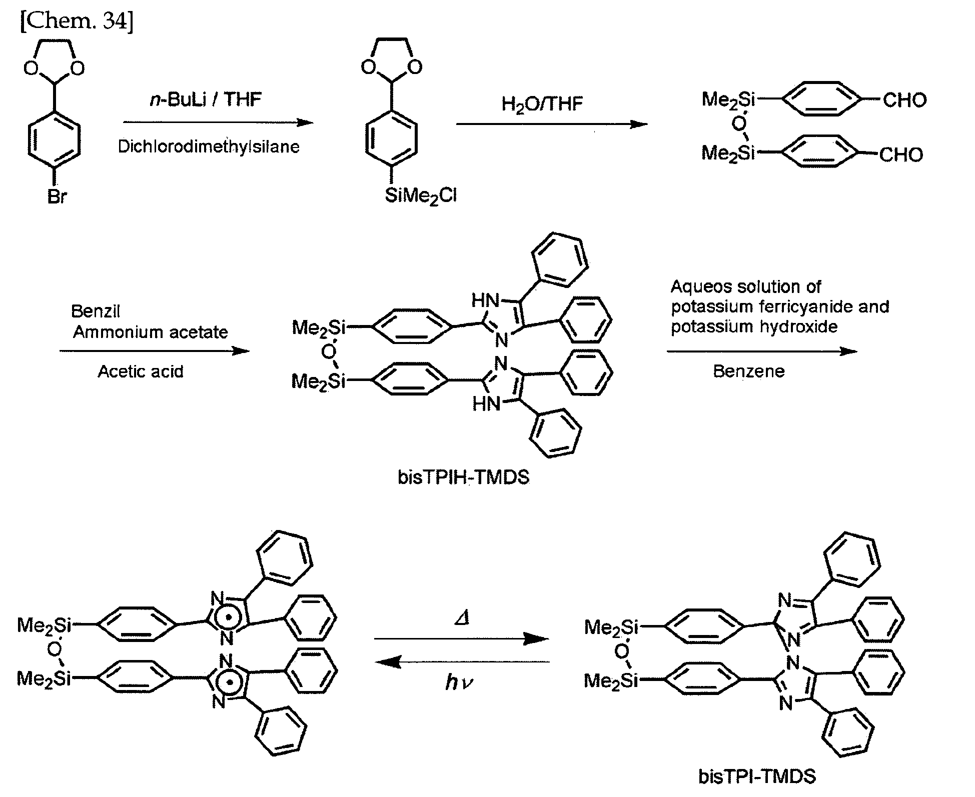

In a 100 mL capacity recovery flask, 2.00 g (8.72 mmol) of 2-(4-bromophenyl)-1,3-dioxolane was dissolved in 30 mL of THF and stirred at -78°C for 30 minutes. 6.50 mL (10.4 mmol) of a 1.66 M hexane solution of n-BuLi was added dropwise gradually at the same temperature, and stirring was carried out for 2 hours (reaction solution A).
Subsequently, in a 100 mL capacity recovery flask, 4.56 g (0.035 mmol) of dichlorodimethylsilane was diluted with 10 mL of THF, the reaction solution A was added dropwise thereto using a transfer tube, and stirring was carried out at room temperature for 12 hours. After unreacted dichlorodimethylsilane and THF were removed by distilling the reaction solution under reduced pressure, soluble components were separated using 40 mL of diethyl ether. 2 mL of ion exchanged water was added to this solution, stirring was carried out for 2 hours, a further 4 mL of ion exchanged water was added, and stirring was carried out for 30 minutes. After stirring, the diethyl ether layer was distilled under reduced pressure, thus giving a colorless transparent oil.

Moreover, in a 50 mL capacity recovery flask, the above colorless transparent oil, 2.02 g (9.60 mmol) of benzil, and 3.67 g (0.0476 mmol) of ammonium acetate were dissolved in 4 mL of acetic acid, and stirring was carried out at 90°C for 4 hours. After stirring, the acetic acid was neutralized with diluted aqueous ammonia solution, and a powder thus precipitated was filtered and washed with ion exchanged water, thus giving a yellow powder. This yellow powder was purified by column chromatography and a recrystallization method, and bisTPIH-TMDS was obtained as a white powder; the amount collected was 120 mg and the yield was 3.8%. The results of NMR measurement are shown below.

¹H NMR (500 MHz, DMSO-d₆): δ = 0.357 (s, 12H), 7.301-7.652 (m, 20H), 8.079 (d, 4H), 8.093 (d, 4H), 12.717 (s, 2H). Mass (m/e): 723(M⁺)

Subsequently, to a 100 mL nitrogen-flushed recovery flask was added 70 mg (0.096 mmol) of bisTPIH-TMDS, and it was dissolved in 40 mL of benzene. An aqueous solution of 1.00 g (0.0178 mmol) of potassium hydroxide and 1.00 g (3.03 mmol) of potassium ferricyanide in 30 mL of ion exchanged water was added dropwise thereto over 10 minutes, and stirring was carried out for 2 hours at room temperature. After stirring was completed, extraction was carried out using benzene, washing was carried out using ion exchanged water, and distillation under reduced pressure was carried out, thus giving 55 mg of bisTPI-TMDS as a pale yellow powder, yield 76%.

### Measurement of photochromic properties of 1,3-bis(triphenylimidazole)-1,1,3,3-tetramethyldisiloxane

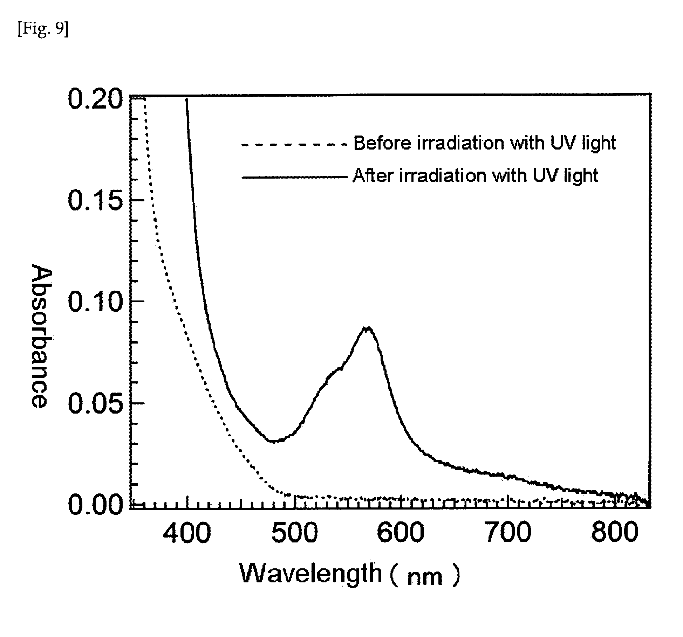

A benzene solution (concentration 3.5 × 10⁻⁴ mol/L) of the bisTPI-TMDS synthesized was subjected to measurement of photochromic properties at a wavelength of 360 nm to 830 nm under an atmosphere of argon at 25°C. Photochromism was shown in that the color of the solution instantly changed from being colorless to forming a reddish violet color on irradiation with UV light having a wavelength of 360 nm, and a new absorption band was observed from 500 nm to 800 nm. The result of measurement of a visible absorption spectrum is shown in FIG. 9.

### [Example 4]

### Synthesis of [2.2]paracyclophane-4-diphenylimidazole-13-carbaldehyde

The precursor compound [2.2]paracyclophane-4-diphenylimidazole-13-carbaldehyde, which is a key compound in the production method of the present invention, was synthesized via the synthetic route below.

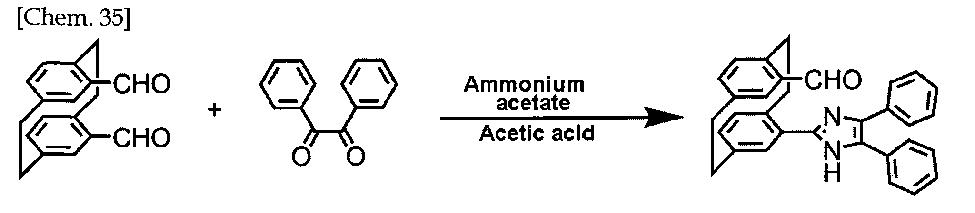

To a 30 mL flask were added [2.2]paracyclophane-4,13-dicarbaldehyde (0.50 g,1.9 mmol), benzil (0.40 g, 1.9 mmol), ammonium acetate (2.2 g, 28 mmol), and 5 mL of acetic acid, and they were heated and refluxed for 5 hours. After allowing to cool to room temperature, neutralization with aqueous ammonia and filtration under reduced pressure were carried out. The filtrate was washed with water and dried, and components at the origin were removed by silica gel column chromatography (solvent: dichloromethane). This mixture was dissolved in dichloromethane and stirred with 1M hydrochloric acid, thus forming a colorless precipitate (hydrochloride salt of target compound). This was filtered off by Celite filtration, washed well with dichloromethane, then suspended again in dichloromethane, and deprotonated by adding aqueous ammonia. This was extracted with dichloromethane, and [2.2]paracyclophane-4-diphenylimidazole-13-carbaldehyde, which is the target key compound, was thus isolated (0.56 g, 67%). The results of NMR measurement are shown below.

¹H-NMR (500 MHz, DMSO-d₆) δ: 12.14 (s, 1H), 9.55 (s, 1H), 7.58 (d, J = 7.5 Hz, 2H), 7.51 (d, J = 7.0 Hz, 2H), 7.47 (dd, J = 7.5 Hz, 2H), 7.40 (t, J = 7.0 Hz, 1H), 7.31 (dd, J = 7.0 Hz, 2H), 7.16 (t, J = 7.0 Hz, 1H), 7.10 (s, 1H), 6.96 (s, 1H), 6.84 (d, J = 7.5 Hz, 1H), 6.71 (d, J = 8.0 Hz, 1H), 6.66 (d, J = 7.5 Hz, 2H), 4.49-4.45 (m, 1H), 3.95-3.92 (m, 1H), 3.15-2.97 (m, 6H).

### [Example 5]

### Synthesis of [2.2]paracyclophane-4-bis(para-methoxyphenyl)imidazole-13-diphenylimidazole (pseudogem-BMPIH-DPIH[2.2]paracyclophane)

*pseudogem*-BMPIH-DPIH[2.2]paracyclophane was synthesized in accordance with the synthetic route below using [2.2]paracyclophane-4-diphenylimidazole-13-carbaldehyde obtained in the synthesis of Example 4 above. The synthetic route is shown below.

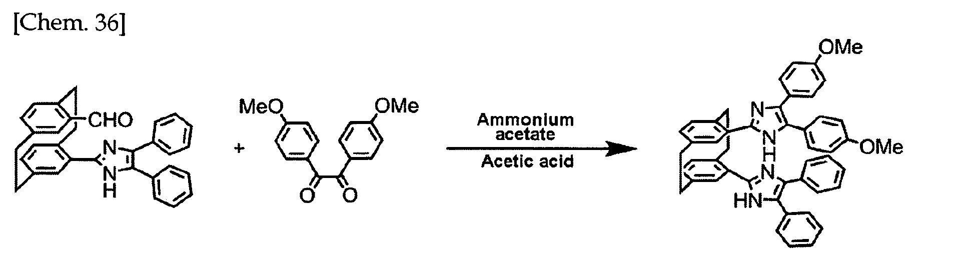

To a 30 mL flask were added [2.2]paracyclophane-4-diphenylimidazole-13-carbaldehyde (0.15 g, 0.33 mmol), *p*-anisil (0.108 g, 0.40 mmol), ammonium acetate (0.38 g, 5.0 mmol), and 5 mL of acetic acid, 18 hours thereafter 0.4 g of ammonium acetate and 24 hours thereafter 0.1 g of *p*-anisil were added, and stirring was carried out for 36 hours. After allowing to cool to room temperature, while cooling with ice neutralization with aqueous ammonia and extraction with dichloromethane were carried out. The organic layer was washed with saturated brine and vacuum concentrated. This mixture was purified by silica gel column chromatography (solvent: hexane/THF = 2/1) and GPC (solvent: THF), thus giving *pseudogem-*BMPIH-DPIH[2.2]paracyclophane (0.011 g, 45%). The results of NMR measurement are shown below.

¹H-NMR (500 MHz, DMSO-d₆) δ: 11.62 (s, 1H), 11.47 (s, 1H), 7.29-6.98 (m, 16H), 6.70-6.68 (m, 4H), 6.61-6.58 (m, 4H), 4.59-4.49 (m, 2H), 3.73 (s, 3H), 3.66 (s, 3H), 3.12-3.02 (m, 6H).

Subsequently, *pseudogem*-BMPI-DPI[2.2]paracyclophane, which is a compound of the present invention for the purpose of precise control of photochromic properties, was synthesized in accordance with the synthetic route below.

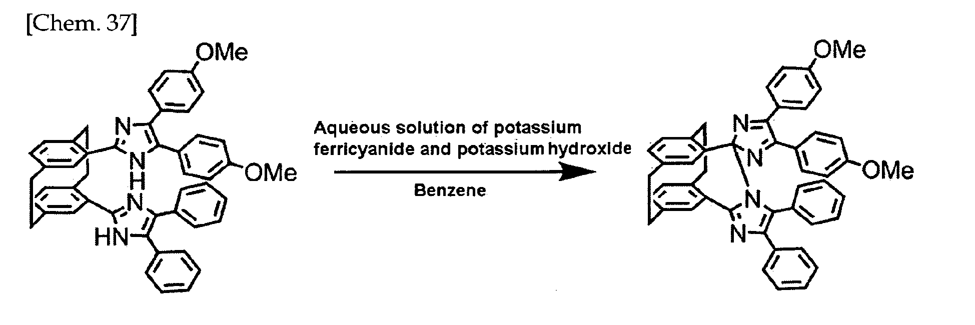

All solvents used in the reaction were degassed by bubbling nitrogen for 30 minutes, and all of the operations were carried out in the dark. In a 200 mL flask, *pseudogem*-BMPIH-DPIH[2.2]paracyclophane (0.050 g, 0.071 mmol) obtained by the above synthesis was dissolved in 20 mL of degassed benzene, an aqueous solution (7.4 mL) of potassium ferricyanide (1.2 g, 3.5 mmol) and potassium hydroxide (0.48 g, 8.5 mmol) was added, and vigorous stirring was carried out for 30 minutes. The benzene layer was extracted, washed well with water, and then vacuum concentrated, thus giving the target *pseudogem*-BMPI-DPI[2.2]paracyclophane (0.050 g, quant.). Since the oxidized form is a complicated mixture of isomers, clear assignment by ¹H-NMR could not be carried out.

### Measurement of photochromic properties of pseudogem-BMPI-DPI[2.2]paracyclophane

The photochromic properties of the *pseudogem*-BMPI-DPI[2.2]paracyclophane obtained by the above synthesis were confirmed by nanosecond laser flash photolysis measurement under the same conditions as in Example 1. The *pseudogem-*BMPI-DPI[2.2]paracyclophane was dissolved in degassed benzene (concentration: 1.57 × 10⁻⁴ mol/L), and measurement was carried out at 25°C under an atmosphere of argon.

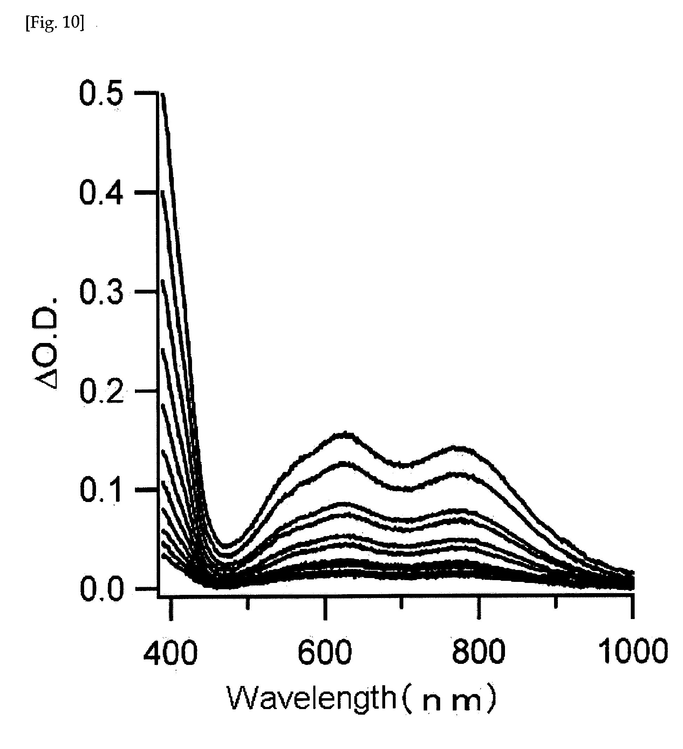

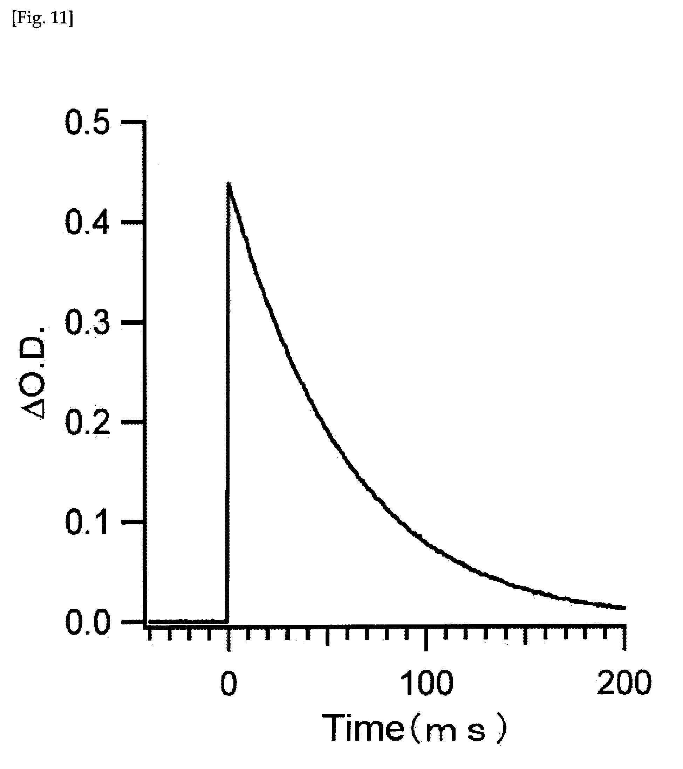

FIG. 10 shows the result of measurement of visible/near-IR absorption spectra under the same conditions as in Example 1. It was confirmed from the result of FIG. 10 that, compared with the absorption spectrum of the above-mentioned unsubstituted form *pseudogem*-bisDPI[2.2]paracyclophane, the absorption spectrum of the photocolored form of *pseudogem*-BMPI-DPI[2.2]paracyclophane resulting from irradiation with UV light shifted overall to longer wavelength, and the shape of the spectrum changed. Furthermore, it was confirmed from visual examination that a slightly darker blue coloration developed. That is, the possibility of precise control of photochromic properties such as tone and density of coloration by combination of two triarylimidazolyl radicals (TAIRs) having different structures and different energy levels, absorption wavelengths, etc. is suggested.
Furthermore, FIG. 11 shows the result of measurement of decay over time of the absorption band at 400 nm under the same conditions as in Example 1. It was confirmed from the result of FIG. 11 that, with regard to change over time of the absorbance at 400 nm of *pseudogem*-BMPI-DPI[2.2]paracyclophane, it decayed with a half life (about 50 ms) comparable to that of the above-mentioned unsubstituted form *pseudogem*-bisDPI[2.2]paracyclophane.

### [Example 6]

### Synthesis of [2.2]paracyclophane-4-bis(para-hydroxyphenyl)imidazole-13-diphenylimidazole (pseudoem-BHPIH-DPIH[2.2]paracyclophane)

*pseudogem*-BHPIH-DPIH[2.2]paracyclophane, which is a precursor of the photochromic compound *pseudogem*-BHPI-DPI[2.2]paracyclophane of the present invention, was synthesized by heating and stirring [2.2]paracyclophane-4-diphenylimidazole-13-carbaldehyde obtained in the synthesis of Example 4 above and dihydroxybenzil, which is a benzil derivative having a target structure, in acetic acid in the presence of ammonium acetate. The synthetic route is shown below.

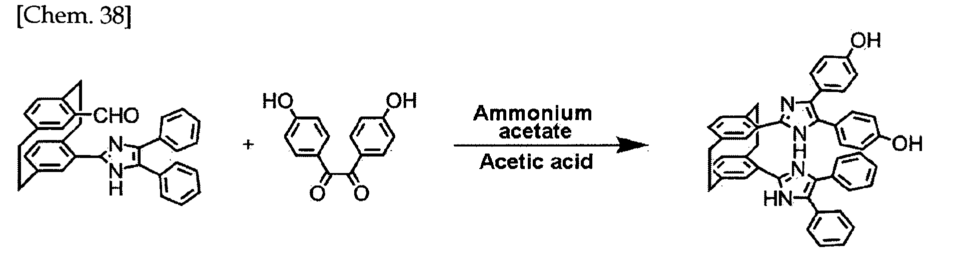

To a 50 mL flask were added [2.2]paracyclophane-4-diphenylimidazole-13-carbaldehyde (0.22 g, 0.64 mmol), dihydroxybenzil (0.16 g, 0.67 mmol), ammonium acetate (0.74 g, 9.6 mmol), and 5 mL of acetic acid, and heating and refluxing were carried out for 10 hours. After allowing to cool to room temperature, while cooling with ice, neutralization with aqueous ammonia and filtration under reduced pressure were carried out. The filtrate was washed with water and then dried under vacuum. This reaction mixture was subjected to silica gel column chromatography (solvent: hexane/THF = 2/1), thus giving *pseudogem-BHPIH-*DPIH[2.2]paracyclophane (0.30 g, quant.). The results of NMR measurement are shown below.

¹H-NMR (500 MHz, DMSO-d₆) δ: 11.60 (s,1H),11.32 (s,1H), 9.36 (s,1H), 9.10 (s,1H), 7.27-7.05 (m, 13H), 6.89-6.86 (m, 3H), 6.70-6.44 (m, 8H), 4.33-4.32 (m, 2H), 3.10-3.01 (m, 6H).

### [Example 7]

### Synthesis of paracyclophane-4-bis(3,4-dimethoxyphenyl)imidazole-13-diphenylimidazole (pseudogem-DMIH-DPIH[2.2]paracyclophane)

*pseudogem*-DMIH-DPIH[2.2]paracyclophane was synthesized in accordance with the synthetic route below using [2.2]paracyclophane-4-diphenylimidazole-13-carbaldehyde obtained in the synthesis of Example 4 above. The synthetic route is shown below.

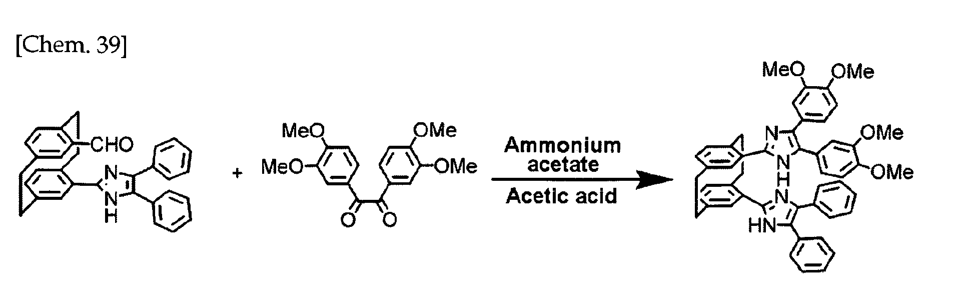

To a 30 mL flask were added [2.2]paracyclophane-4-diphenylimidazole-13-carbaldehyde (0.18 g, 0.39 mmol), 3,3',4,4'-tetramethoxybenzil (0.16 g, 0.47 mmol), ammonium acetate (1.1 g, 14 mmol), and 7.5 mL of acetic acid, and refluxing was carried out for 30 hours. After allowing to cool to room temperature, while cooling with ice, neutralization with aqueous ammonia and extraction with dichloromethane were carried out. The organic layer was washed with saturated brine and vacuum concentrated. This mixture was purified by silica gel column chromatography (solvent: hexane/THF = 2/1), thus giving *pseudogem-*DMIH-DPIH[2.2]paracyclophane (0.16 g, 56%). The results of NMR measurement are shown below.

¹H-NMR (500 MHz, DMSO-d₆) δ =11.61 (s,1H),11.51 (s,1H), 7.20 (d, J = 5.1 Hz, 2H), 7.13-7.11 (m, 3H), 7.06-7.04 (m, 3H), 7.01-6.97 (m, 5H), 6.85 (d, J = 2.0 Hz, 1H), 6.79-6.77 (m, 3H), 6.71-6.69 (m, 2H), 6.64-6.61 (m, 3H), 3.74 (s, 3H), 3.67 (s, 3H), 3.07-3.03 (m, 6H)

Subsequently, *pseudogem*-DMI-DPI[2.2]paracyclophane, which is a compound of the present invention for the purpose of control of photochromic properties, was synthesized in accordance with the synthetic route below. 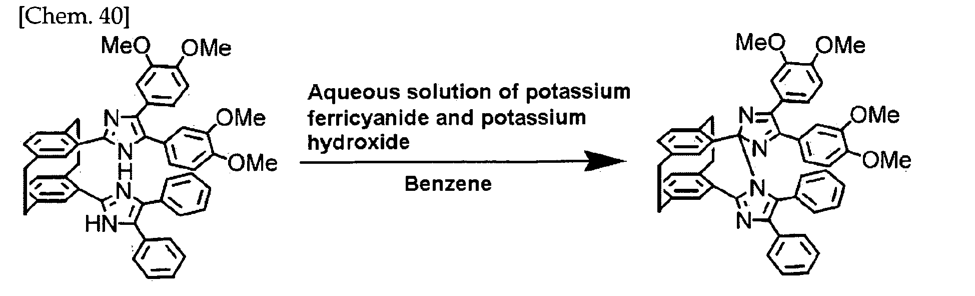 All solvents used in the reaction were degassed by bubbling nitrogen for 30 minutes, and all of the operations were carried out in the dark. In a 200 mL flask, *pseudogem*-DMIH-DPIH[2.2]paracyclophane (0.10 g, 0.13 mmol) obtained by the above synthesis was dissolved in 20 mL of degassed benzene, an aqueous solution (12 mL) of potassium ferricyanide (2.3 g, 6.9 mmol) and potassium hydroxide (0.78 g, 14 mmol) was added, and vigorous stirring was carried out for 60 minutes. The benzene layer was extracted, washed well with water, and then vacuum concentrated, thus giving the target *pseudogem*-DMI-DPI[2.2]paracyclophane (0.094 g, 94%). The results of NMR measurement are shown below.

¹H-NMR (500 MHz, DMSO-d₆) δ = 7.42 (d, J = 7.3 Hz, 1H), 7.21-7.19 (m, 2H), 7.18-7.15 (m, 2H), 7.13-7.11 (m, 2H), 7.09-7.04 (m, 3H), 6.98 (d, J = 8.6 Hz, 1H), 6.88-6.84 (m,1H), 6.80 (s, 1H), 6.77 (s,1H), 6.68-6.66 (m, 5H), 6.55-6.46 (m, 3H), 3.79 (s, 3H), 3.74 (s, 3H), 3.64 (s, 3H), 3.57 (s, 3H), 3.19-3.01 (m, 6H)

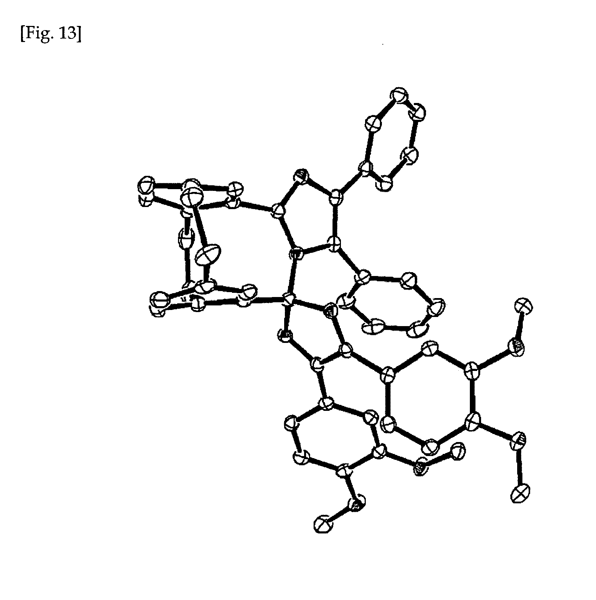

The synthesized *pseudogem*-DMI-DPI[2.2]paracyclophane is subjected to a crystal structural analysis in the same manner as in Example 1. The molecular structure elucidated by the analysis is shown in FIG. 13.

### Absorption spectrum characteristics of decolored form of , pseudogem-DMI-DPI[2.2]paracyclophane

The absorption spectrum of *pseudogem*-DMI-DPI[2.2]paracyclophane obtained by the above synthesis was confirmed by UV-visible absorption spectrum measurement. *pseudogem*-DMI-DPI[2.2]paracyclophane was dissolved in benzene (concentration: 2.1 × 10⁻⁵ M) and measured at 25°C.

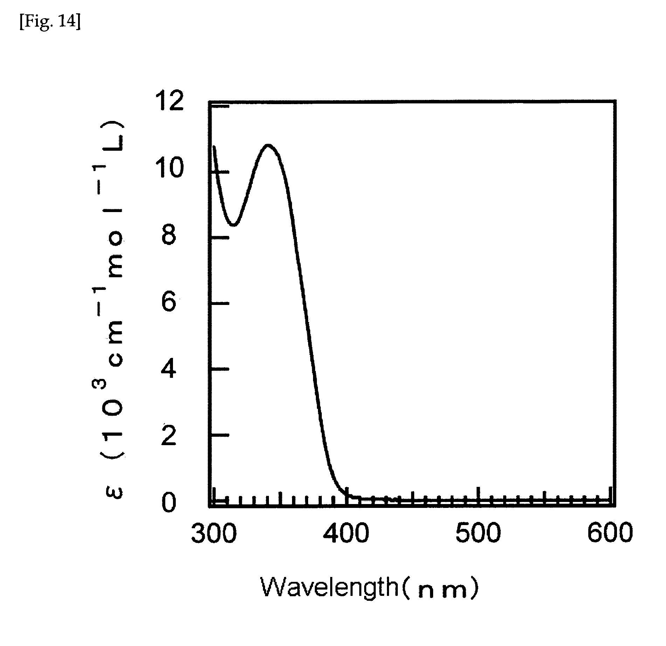

FIG. 14 shows the result of measurement of the UV-visible absorption spectrum. From the result of FIG. 14, *pseudogem*-DMI-DPI[2.2]paracyclophane had an absorption band in the visible light region at 400 nm and above. The molar extinction coefficient ε at 400 nm was 200. It was confirmed that, compared with the molar extinction coefficient at 400 nm of the above-mentioned unsubstituted form *pseudogem*-bisDPI[2.2]paracyclophane, the absorption in the visible light region was increased by the introduction of a methoxy group.

### Measurement of photochromic properties of pseudogem-DMI-DPI[2.2]paracyclophane

The photochromic properties of *pseudogem*-DMI-DPI[2.2]paracyclophane obtained by the above synthesis were confirmed by a nanosecond laser flash photolysis measurement under the same conditions as in Example 1. *pseudogem-*DMI-DPI[2.2]paracyclophane was dissolved in degassed benzene (concentration: 2.1 × 10⁻⁴ M), and measurement was carried out at 25°C under an atmosphere of argon.

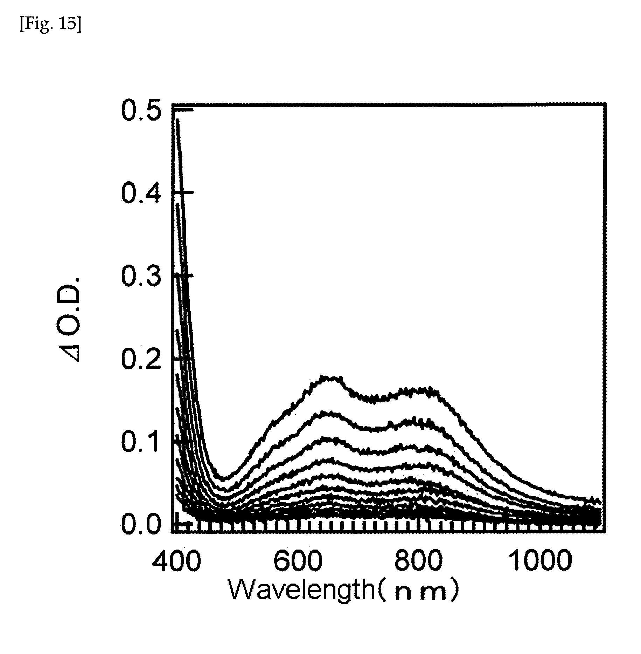

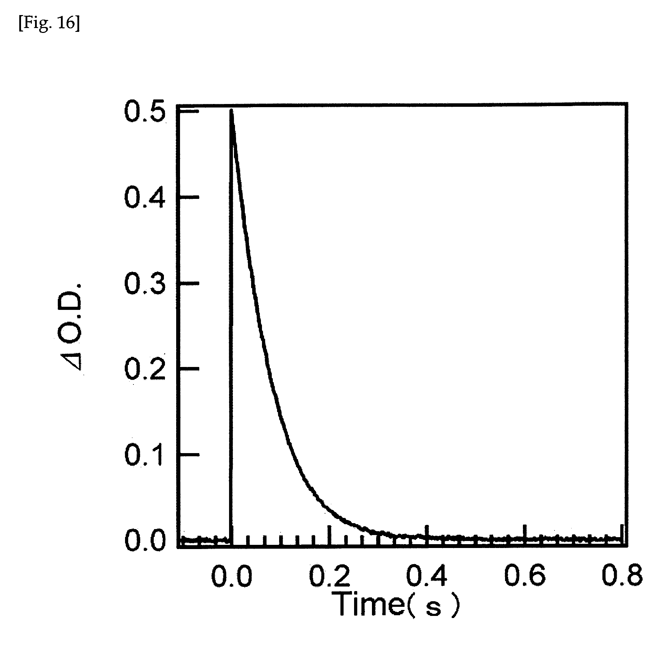

FIG. 15 shows the result of measurement of visible/near-IR absorption spectra under the same conditions as in Example 1. It was confirmed from the result of FIG. 15 that, compared with the absorption spectrum of the above-mentioned unsubstituted form *pseudogem*-bisDPI[2.2]paracyclophane, the absorption spectrum of the photocolored form of *pseudogem*-DMI-DPI[2.2]paracyclophane resulting from irradiation with UV light shifted slightly to longer wavelength, and the shape of the spectrum changed. As a result, the photocolored form looked bluish green to the naked eye.
Furthermore, FIG. 16 shows the result of measurement of decay over time of the absorption band at 400 nm under the same conditions as in Example 1. It was confirmed from the result of FIG. 16 that, with regard to change over time of the absorbance at 400 nm of *pseudogem*-DMI-DPI[2.2]paracyclophane, it decayed with a half life (56 ms) comparable to the half life (33 ms) of the above-mentioned unsubstituted form *pseudogem*-bisDPI[2.2]paracyclophane.

### [Example 8]

### Synthesis of paracyclophane-4,13-bis(3,4-dimethoxyphenyl)imidazole (pseudogem-bisDMIH[2.2]paracyclophane)

*pseudogem*-BisDMIH[2.2]paracyclophane was synthesized using [2.2]paracyclophane-4,13-dicarbaldehyde in accordance with the synthetic route below. The synthetic route is shown below.

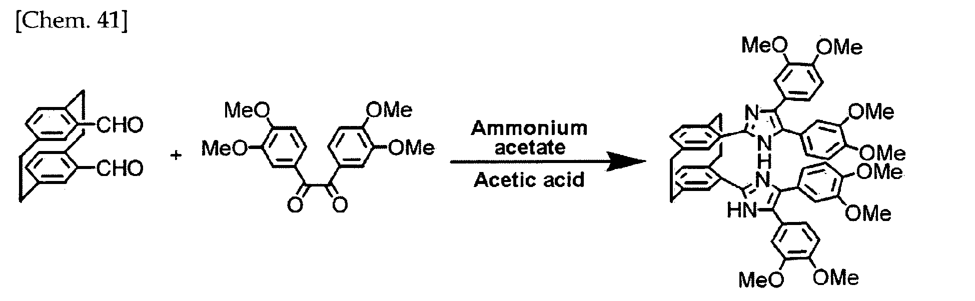

To a 25 mL recovery flask were added [2.2]paracyclophane-4,13-dicarbaldehyde (100 mg, 0.38 mmol), 3,3',4,4'-tetramethoxybenzil (250 mg, 0.76 mmol), ammonium acetate (1.8 g, 24 mmol), and 15 mL of acetic acid, and refluxing was carried out for 22 hours. After allowing to cool to room temperature, while cooling with ice, neutralization with aqueous ammonia and extraction with dichloromethane were carried out. The organic layer was washed with saturated brine and vacuum concentrated. This mixture was purified by silica gel column chromatography (solvent: hexane/THF = 2/1), thus giving *pseudogem-*bisDMIH[2.2]paracyclophane (0.12 g, 35%). The results of NMR measurement are shown below.

¹H-NMR (500 MHz, DMSO-d₆) δ = 11.49 (s, 2H), 7.14 (s, 2H), 6.95 (d, J = 7.9 Hz, 2H), 6.84 (d, J = 1.8 Hz, 2H), 6.75-6.57 (m, 12H), 3.72 (s, 6H), 3.66 (s, 6H), 3.42 (s, 6H), 3.36 (s, 6H), 3.05-3.04 (m, 6H)

Subsequently, *pseudogem*-bisDMI[2.2]paracyclophane, which is a compound of the present invention for the purpose of control of photochromic properties, was synthesized in accordance with the synthetic route below.

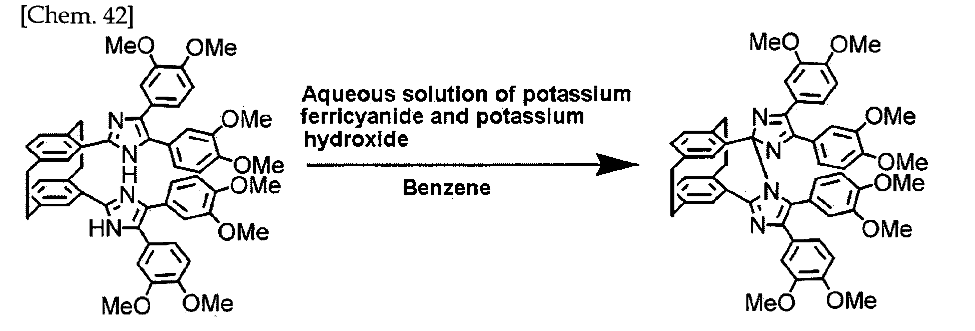

All solvents used in the reaction were degassed by bubbling nitrogen for 30 minutes, and all of the operations were carried out in the dark. In a 200 mL flask, *pseudogem*-bisDMIH[2.2]paracyclophane (0.040 g, 0.045 mmol) obtained by the above synthesis was dissolved in 10 mL of degassed benzene, an aqueous solution (8 mL) of potassium ferricyanide (0.75 g, 2.3 mmol) and potassium hydroxide (0.39 g, 7.0 mmol) was added, and vigorous stirring was carried out for 20 minutes. The benzene layer was extracted, washed well with water, and then vacuum concentrated, thus giving the target *pseudogem*-bisDMI[2.2]paracyclophane (0.040 g, quant.). The results of NMR measurement are shown below.

¹H-NMR (500 MHz, DMSO-d₆) δ = 7.20 (s, 1H), 7.16 (s, 1H), 7.08 (s, 1H), 7.02 (d, J = 8.6 Hz, 1H), 6.92 (m, 2H), 6.85 (s, 1H), 6.79-6.67 (m, 8H), 6.52-6.46 (m, 3H), 3.72-3.36 (m, 24H), 3.12-3.00 (m, 6H)

### Absorption spectrum characteristics of decolored form of pseudogem-bisDMI[2.2]paracyphane

The absorption spectrum of *pseudogem*-bisDMI[2.2]paracyclophane obtained by the above synthesis was confirmed by UV-visible absorption spectrum measurement. *pseudogem*-BisDMI[2.2]paracyclophane was dissolved in benzene (concentration: 2.1 × 10⁻⁵ M), and measurement was carried out at 25°C.

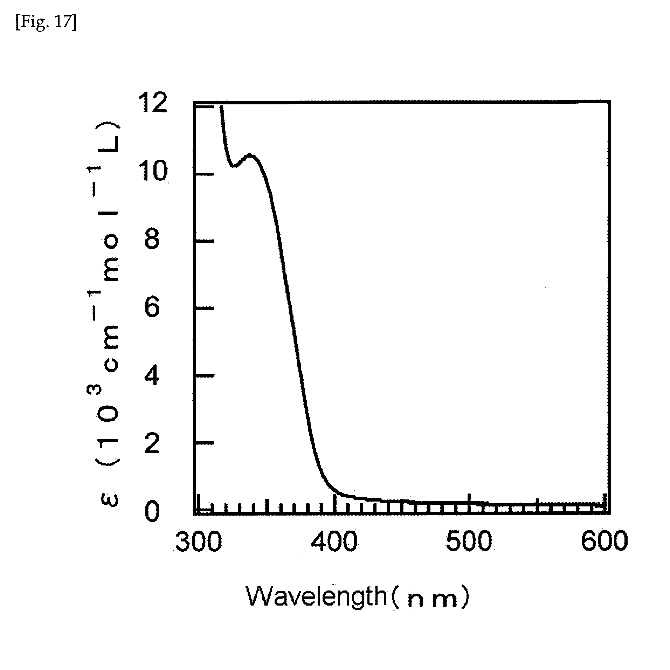

FIG. 17 shows the result of measurement of the UV-visible absorption spectrum. From the result of FIG. 17, *pseudogem*-bisDMI[2.2]paracyclophane also had an absorption band in the visible light region of 400 nm and above. The molar extinction coefficient ε at 400 nm was 600. It was confirmed that, compared with the molar extinction coefficient ε at 400 nm of the above-mentioned singly-substituted form (*pseudogem*-DMI-DPI[2.2]paracyclophane) and unsubstituted form (*pseudogem-*bisDPI[2.2]paracyclophane), the absorption in the visible light region increased.

### Measurement of photochromic properties of pseudogem-bisDMI[2.2]paracydophane

The photochromic properties of *pseudogem*-bisDMI[2.2]paracyclophane obtained by the above synthesis were confirmed by nanosecond laser flash photolysis measurement under the same conditions as in Example 1. *pseudogem-*BisDMI[2.2]paracyclophane was dissolved in degassed benzene (concentration: 2.1 × 10⁻⁴ M), and measurement was carried out at 25°C under an atmosphere of argon.

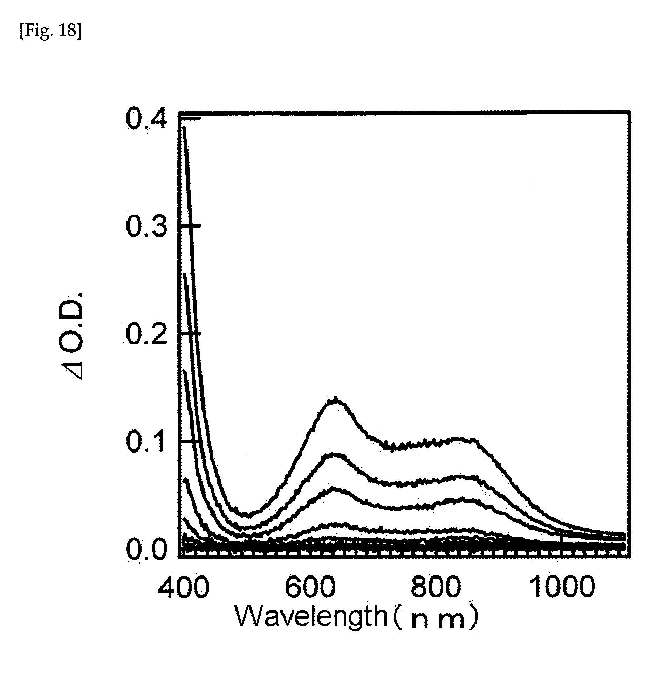

FIG. 18 shows the result of measurement of visible/near-IR absorption spectra under the same conditions as in Example 1. It was confirmed from the result of FIG. 18 that, compared with the absorption spectrum of the above-mentioned unsubstituted form *pseudogem*-bisDPI[2.2]paracyclophane, the absorption spectrum of the photocolored form of *pseudogem*-bisDMI[2.2]paracyclophane resulting from irradiation with UV light shifted to longer wavelength and the shape of the spectrum changed. As a result, the photocolored form looked green to the naked eye. Furthermore, as described above, since *pseudogem*-bisDMI[2.2]paracyclophane had an absorption in the visible light region of 400 nm and above, coloration could be confirmed under sunlight or room lighting.

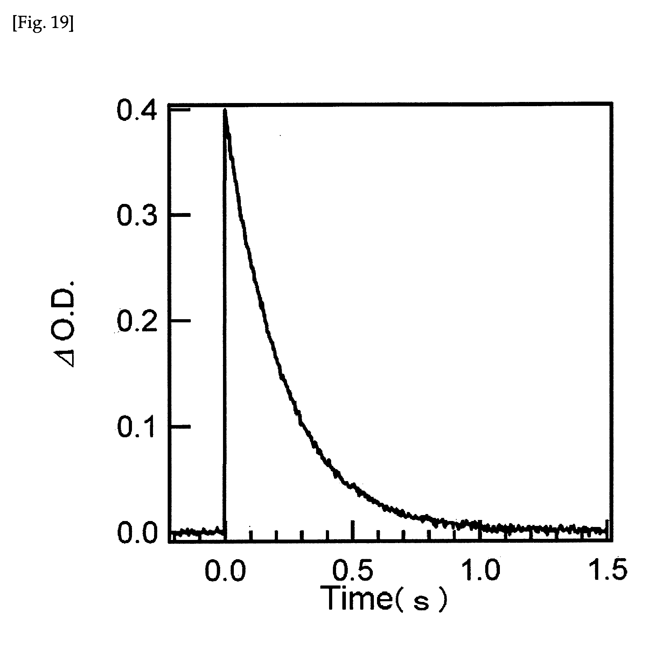

Furthermore, FIG. 19 shows the result of measurement of decay over time of the absorption band at 400 nm under the same conditions as in Example 1. It was confirmed from the result of FIG. 19 that, with regard to change over time of the absorbance at 400 nm of *pseudogem*-bisDMI[2.2]paracyclophane, it decayed with a half-life (not more than 200 ms) that was longer than the half life of the above-mentioned unsubstituted form *pseudogem*-bisDPI[2.2]paracyclophane.

### [Example 9]

### Synthesis of paracyclophane-4-(3-phenyl-4-pyrenyl)imidazole-13-diphenylimidazole (pseudogem-PYIH-DPIH[2.2]paracydophane)

*pseudogem*-PYIH-DPIH[2.2]paracyclophane was synthesized in accordance with the synthetic route below using [2.2]paracyclophane-4-diphenylimidazole-13-carbaldehyde obtained in the synthesis of Example 4 above. The synthetic route is shown below.

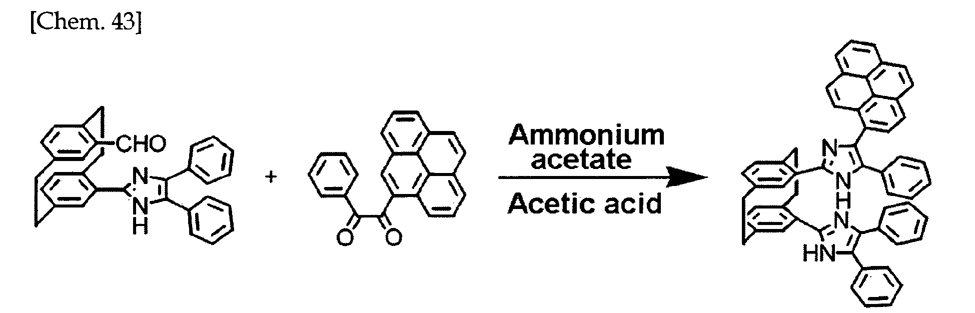

To a 30 mL flask were added [2.2]paracyclophane-4-diphenylimidazole-13-carbaldehyde (0.19 g, 0.42 mmol), a pyrene-substituted benzil derivative (0.14 g, 0.42 mmol), ammonium acetate (0.47 g, 6.1 mmol), and 5 mL of acetic acid, and a reaction was carried out at 110°C for 30 hours. After allowing to cool to room temperature, while cooling with ice, neutralization with aqueous ammonia and extraction with dichloromethane were carried out. The organic layer was washed with saturated brine and vacuum concentrated. This mixture was purified by silica gel column chromatography (solvent: hexane/THF = 2/1) and then purified by fractionation using gel filtration chromatography (solvent: THF), thus giving *pseudogem-PYIH-*DPIH[2.2]paracyclophane (0.27 g, 83%). The results of NMR measurement are shown below.

¹H-NMR (500 MHz, DMSO-d₆) δ = 12.03-11.69 (m, 2H), 8.20-6.64 (m, 30H), 4.34-4.14 (m, 2H), 3.16-3.05 (m, 6H)

Subsequently, *pseudogem*-PYI-DPI[2.2]paracyclophane, which is a compound of the present invention for the purpose of control of photochromic properties, was synthesized in accordance with the synthetic route below.

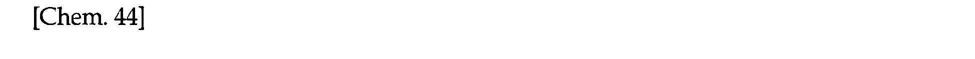

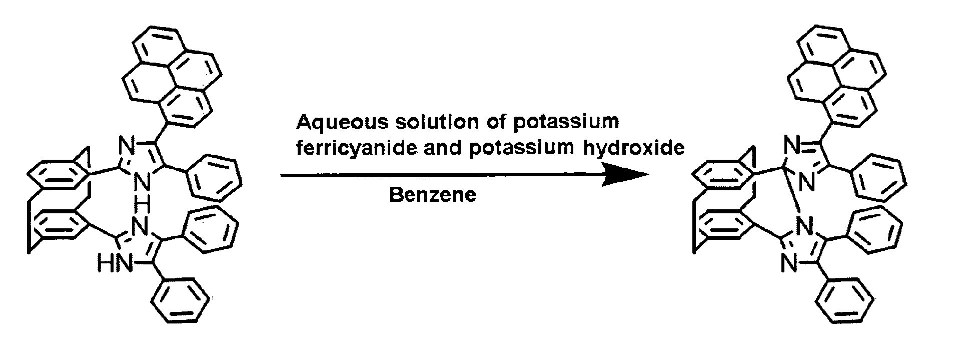

All solvents used in the reaction were degassed by bubbling nitrogen for 30 minutes, and all of the operations were carried out in the dark. In a 50 mL flask, *pseudogem*-PYIH-DPIH[2.2]paracyclophane (0.044 g, 0.057 mmol) obtained by the above synthesis was dissolved in 2 mL of degassed benzene, an aqueous solution (15 mL) of potassium ferricyanide (0.93 g, 2.8 mmol) and potassium hydroxide (0.32 g, 5.7 mmol) was added, and vigorous stirring was carried out for 2 hours. The benzene layer was extracted, washed well with water, and then vacuum concentrated, thus giving the target *pseudogem*-PYI-DPI[2.2]paracyclophane (0.038 g, 87%). Since the oxidized form is a complicated mixture of isomers, clear assignment by ¹H-NMR could not be carried out.

¹H-NMR (500 MHz, DMSO-d₆) δ = 8.34-5.80 (m, 30H), 4.56-4.52 (m, 2H), 3.30-2.90 (m, 6H)

### Absorption spectrum characteristics of decolored form of pseudogem-PYI-DPI[2.2]paracyclophane

The absorption spectrum of *pseudogem*-PYI-DPI[2.2]paracyclophane obtained by the above synthesis was confirmed by UV-visible absorption spectrum measurement. *pseudogem*-PYI-DPI[2.2]paracyclophane was dissolved in benzene (concentration: 2.0 × 10⁻⁵ M), and measurement was carried out at 25°C.

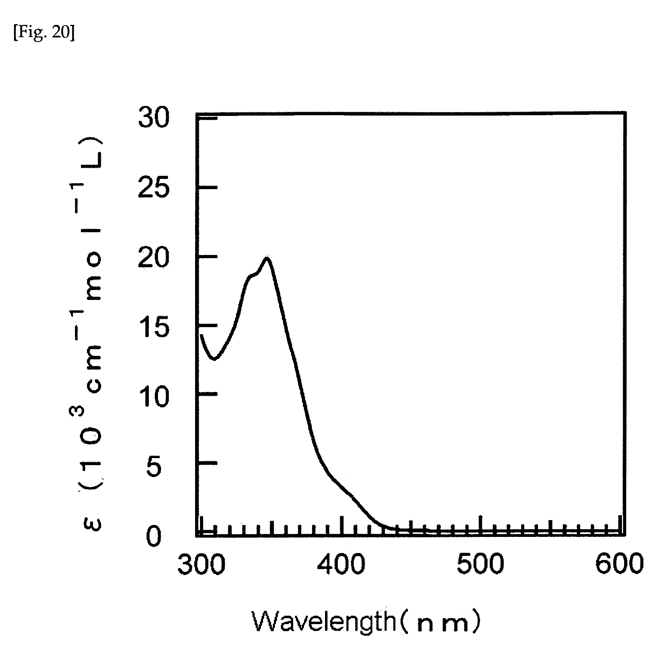

FIG. 20 shows the result of measurement of the UV-visible absorption spectrum. From the result of FIG. 20, *pseudogem*-PYI-DPI[2.2]paracyclophane had a strong absorption band in the visible light region of 400 nm and above. The molar extinction coefficient ε at 400 nm was 3200. The absorption in the visible light region increased greatly compared with the molar extinction coefficient ε at 400 nm of the above-mentioned tetramethoxy-substituted forms (*pseudogem*-DMI-DPI[2.2]paracyclophane, *pseudogem*-bisDMI[2.2]paracyclophane) and the unsubstituted form (*pseudogem*-bisDPI[2.2]paracyclophane).

### Measurement of photochromic properties of pseudogem-PYI-DPI[2.2]paracyclophane

The photochromic properties of *pseudogem*-PYI-DPI[2.2]paracyclophane obtained by the above synthesis were confirmed by nanosecond laser flash photolysis measurement under the same conditions as in Example 1. *µseudogem*-PYI-DPI[2.2]paracyclophane was dissolved in degassed benzene (concentration: 2.0 × 10⁻⁵ M), and measurement was carried out at 25°C under an atmosphere of argon.

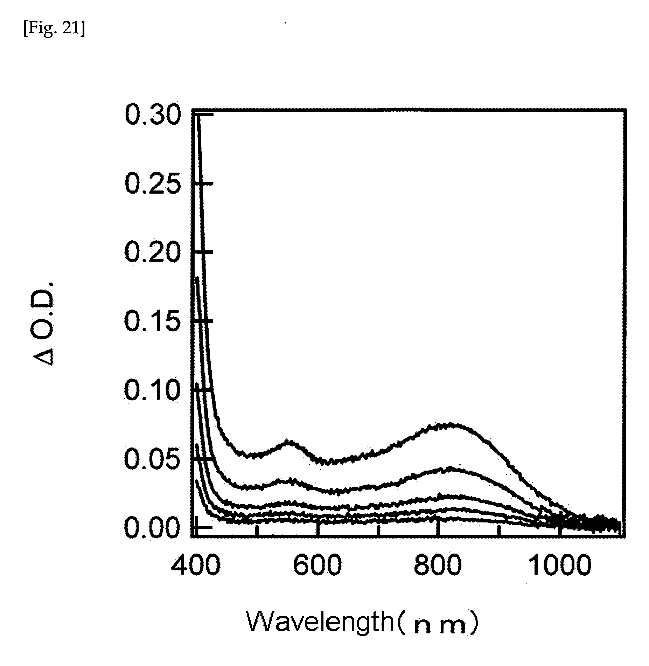

FIG. 21 shows the result of measurement of visible/near-IR absorption spectra under the same conditions as in Example 1. It was confirmed from the result of FIG. 21 that, compared with the absorption spectrum of the above-mentioned unsubstituted form *pseudogem*-bisDPI[2.2]paracyclophane, the absorption spectrum of the photocolored form of *pseudogem*-PYI-DPI[2.2]paracyclophane resulting from irradiation with UV light shifted slightly to longer wavelength and the shape of the spectrum changed.

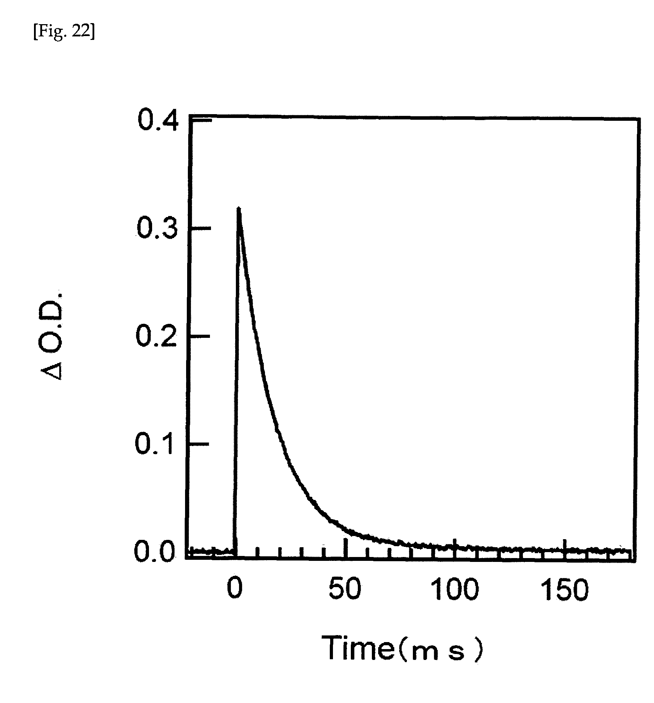

FIG. 22 shows the result of measurement of decay over time of the absorption band at 400 nm under the same conditions as in Example 1. It was confirmed from the result of FIG. 22 that, with regard to change over time of the absorbance at 400 nm of *pseudogem*-PYI-DPI[2.2]paracyclophane, it decayed with a shorter half life (about 15 ms) than that of the above-mentioned unsubstituted form *pseudogem-*bisDPI[2.2]paracyclophane.

### [Example 10]

### Synthesis of paracydophane-4-(3-phenyl-4-pyrenyl)imidazole-13-diphenylimidazole (pseudogem-bisPYIH[2.2]paracydophane)

*pseudogem*-BisPYIH[2.2]paracyclophane was synthesized in accordance with the synthetic route below using [2.2]paracyclophane-4-diphenylimidazole-13-carbaldehyde obtained in the synthesis of Example 4 above. The synthetic route is shown below.

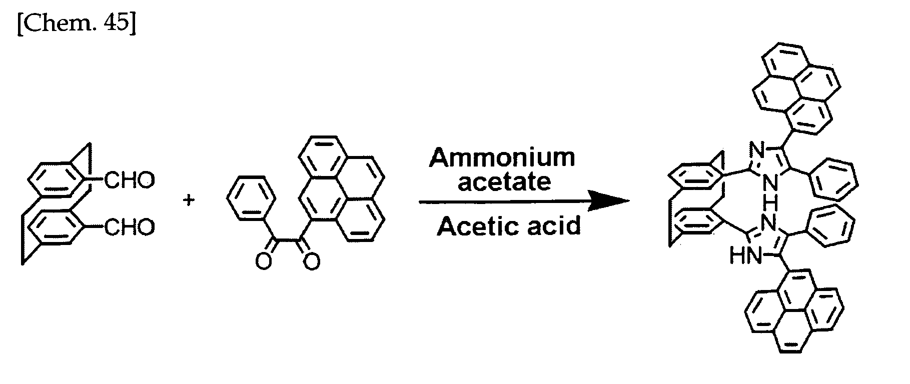

To a 50 mL flask were added [2.2]paracyclophane-4,13-dicarbaldehyde (87 mg, 0.33 mmol), a pyrene-substituted benzil derivative (0.28 g, 0.83 mmol), ammonium acetate (0.54 g, 7.0 mmol), and 10 mL of acetic acid, and a reaction was carried out at 110°C for 46 hours. After allowing to cool to room temperature, while cooling with ice, neutralization with aqueous ammonia and extraction with dichloromethane were carried out. The organic layer was washed with saturated brine and vacuum concentrated. This mixture was purified by silica gel column chromatography (solvent: hexane/THF = 2/1) and then purified by fractionation using gel filtration chromatography (solvent: THF), thus giving *pseudogem-*bisPYIH[2.2]paracyclophane (0.10 g, 35%). The results of NMR measurement are shown below.

¹H-NMR (500 MHz, DMSO-d₆) δ = 12.11-11.96 (m, 2H), 8.25-6.66 (m, 34H), 4.79-4.56 (m, 2H), 3.32-3.08 (m, 6H)

Subsequently, *pseudogem*-bisPYI[2.2]paracyclophane, which is a compound of the present invention for the purpose of control of photochromic properties, was synthesized in accordance with the synthetic route below.

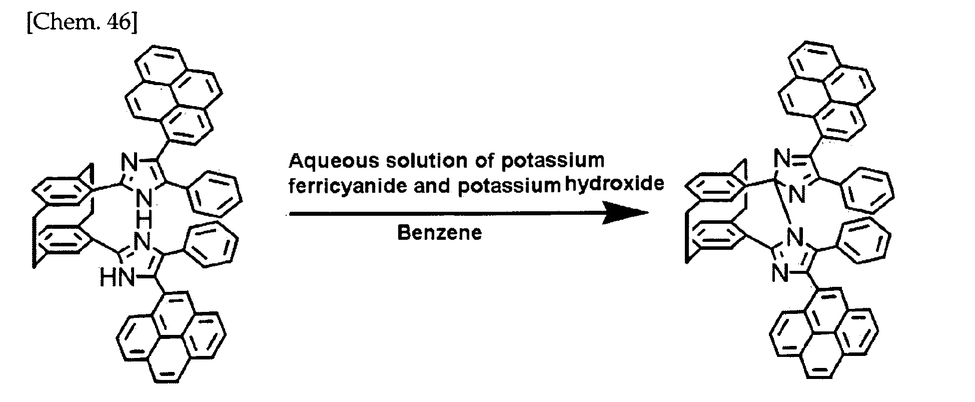

All solvents used in the reaction were degassed by bubbling nitrogen for 30 minutes, and all of the operations were carried out in the dark. In a 50 mL flask, *pseudogem*-bisPYIH[2.2]paracyclophane (0.075 g, 0.083 mmol) obtained by the above synthesis was dissolved in 5 mL of degassed benzene, an aqueous solution (10 mL) of potassium ferricyanide (1.4 g, 4.3 mmol) and potassium hydroxide (0.51 g, 9.0 mmol) was added, and vigorous stirring was carried out for 1 hour. The benzene layer was extracted, washed well with water, and then vacuum concentrated, thus giving the target *pseudogem*-bisPYI[2.2]paracyclophane (0.056 g, 75%). Since the oxidized form is a complicated mixture of isomers, clear assignment by ¹H-NMR could not be carried out.

¹H-NMR (500 MHz, DMSO-d₆) δ = 8.50-5.80 (m, 34H), 4.74-4.45 (m, 2H), 3.30-3.00 (m, 6H)

### Absorption spectrum characteristics of decolored form of pseudogem-bisPYI[2.2]paracyclophane

The absorption spectrum of *pseudogem*-bisPYI[2.2]paracyclophane obtained by the above synthesis was confirmed by UV-visible absorption spectrum measurement. *pseudogem*-BisPYI[2.2]paracyclophane was dissolved in benzene (concentration: 2.0 × 10⁻⁵ M), and measurement was carried out at 25°C.

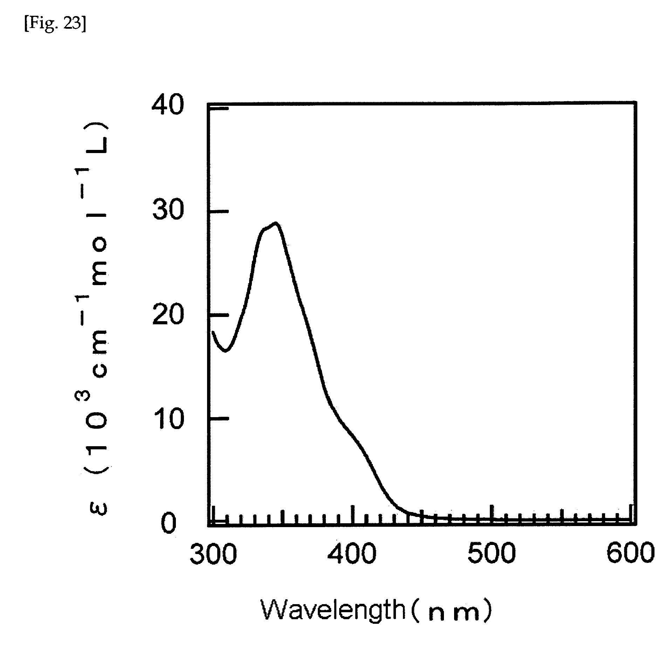

FIG. 23 shows the result of measurement of the UV-visible absorption spectrum. From the result of FIG. 23, *pseudogem*-bisPYI[2.2]paracyclophane also had a strong absorption band in the visible light region of 400 nm and above. The molar extinction coefficient ε at 400 nm was 8300. The absorption in the visible light region increased greatly compared with the molar extinction coefficient ε at 400 nm of the above-mentioned tetramethoxy substituted forms (*pseudogem*-DMI-DPI[2.2]paracyclophane, *pseudogem*-bisDMI[2.2]paracyclophane) and unsubstituted form (*pseudogem*-bisDPI[2.2]paracyclophane).

### Measurement of photochromic properties of pseudogem-bisPYI[2.2]paracyclophane

The photochromic properties of the *pseudogem*-bisPYI[2.2]paracyclophane obtained by the above synthesis were confirmed by nanosecond laser flash photolysis measurement under the same conditions as in Example 1. *pseudogem-*BisPYI[2.2]paracyclophane was dissolved in degassed benzene (concentration: 2.0 × 10⁻⁵ M), and measurement was carried out at 25°C under an atmosphere of argon.

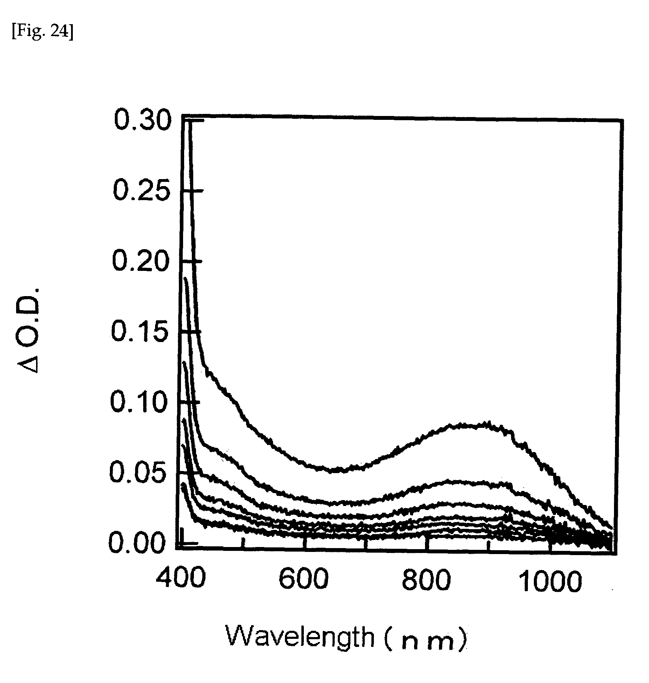

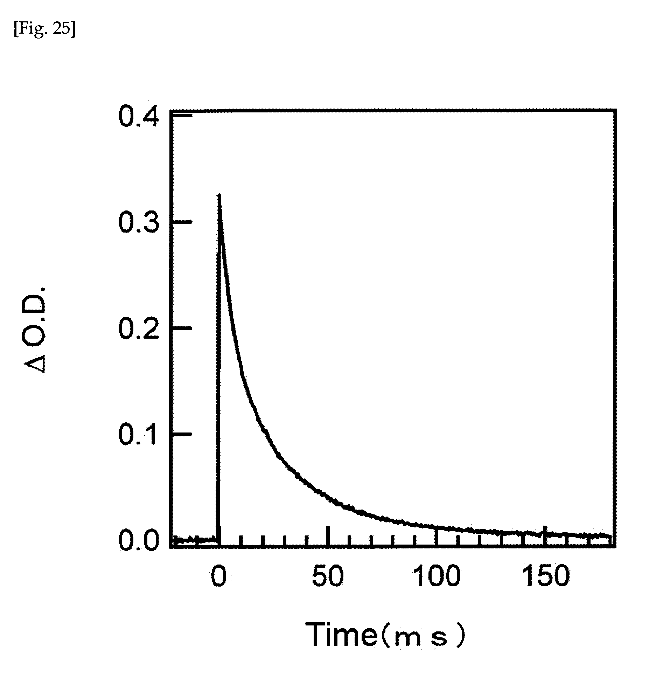

FIG. 24 shows the result of measurement of visible/near-IR absorption spectra under the same conditions as in Example 1. It was confirmed from the result of FIG. 24 that, compared with the absorption spectrum of the above-mentioned unsubstituted form *pseudogem*-bisDPI[2.2]paracyclophane, the absorption spectrum of the photocolored form of *pseudogem*-bisPYI[2.2]paracyclophane resulting from irradiation with UV light shifted slightly to longer wavelength, and the shape of the spectrum changed.
Furthermore, FIG. 25 shows the result of measurement of decay over time of the absorption band at 400 nm under the same conditions as in Example 1. It was confirmed from the result of FIG. 25 that, with regard to change over time of the absorbance at 400 nm of *pseudogem*-bisPYI[2.2]paracyclophane, it decayed with a shorter half life (about 20 ms) than that of the above-mentioned unsubstituted form *pseudogem*-bisDPI[2.2]paracyclophane.

### [Example 11]

### Synthesis of paracyclophane-4,13-bis(3-dimethoxyphenyl-4-chlorophenyl)imidazole (pseudogem-bisMCIH[2.2]paracyclophane)

*pseudogem*-BisMCIH[2.2]paracyclophane was synthesized in accordance with the synthetic route below using [2.2]paracyclophane-4,13-dicarbaldehyde. The synthetic route is shown below.

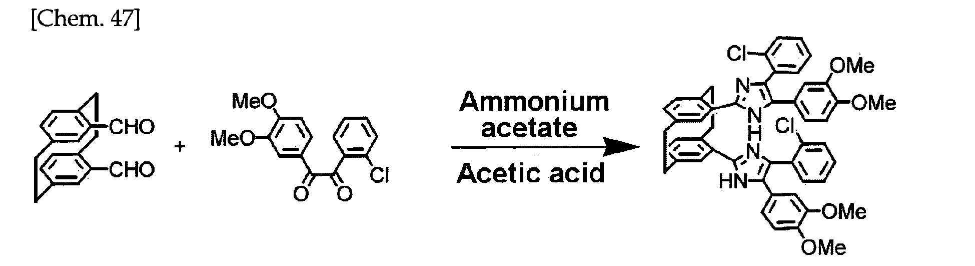

To a 25 mL recovery flask were added [2.2]paracyclophane-4,13-dicarbaldehyde (150 mg, 0.57 mmol), 2-chloro-3',4'-dimethoxybenzil (346 mg, 1.13 mmol), ammonium acetate (0.88 g, 11 mmol), and 6 mL of acetic acid, and a reaction was carried out at 80°C for 40 hours. After allowing to cool to room temperature, while cooling with ice, neutralization with aqueous ammonia and extraction with dichloromethane were carried out. The organic layer was washed with saturated brine and vacuum concentrated. This mixture was purified by silica gel column chromatography (solvent: hexane/THF = 2/1), thus giving *pseudogem-*bisMCIH[2.2]paracyclophane (0.28 g, 60%). The results of NMR measurement are shown below.

¹H-NMR (500 MHz, DMSO-d6) δ = 11.81-11.55 (m, 2H), 7.47-6.61 (m, 20H), 4.54 (br, 2H), 3.73-3.68 (m, 12H), 3.13-3.07 (m, 6H)

Subsequently, *pseudogem*-bisMCI[2.2]paracyclophane, which is a compound of the present invention for the purpose of control of photochromic properties, was synthesized in accordance with the synthetic route below.

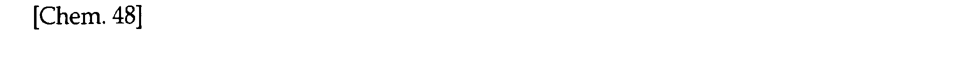

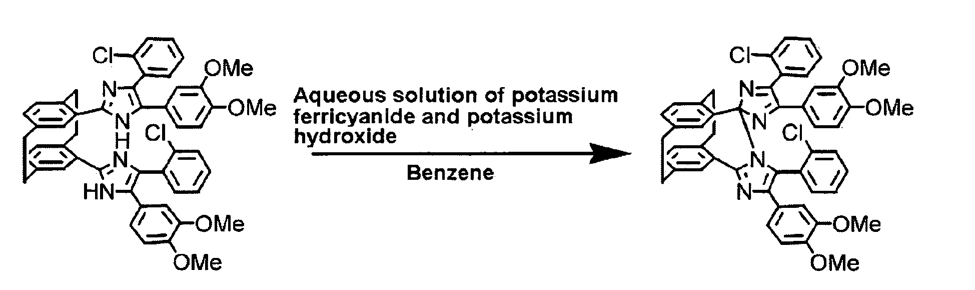

All solvents used in the reaction were degassed by bubbling nitrogen for 30 minutes, and all of the operations were carried out in the dark. In a 100 mL flask, *pseudogem*-bisMCIH[2.2]paracyclophane (0.10 g, 0.12 mmol) obtained by the above synthesis was dissolved in 18 mL of degassed benzene, an aqueous solution (12 mL) of potassium ferricyanide (2.0 g, 6.0 mmol) and potassium hydroxide (0.67 g, 12 mmol) was added, and vigorous stirring was carried out for 30 minutes. The benzene layer was extracted, washed well with water, and then vacuum concentrated, thus giving the target *pseudogem*-bisMCI[2.2]paracyclophane (0.088 g, 87%). Since the oxidized form is a complicated mixture of isomers, clear assignment by ¹H-NMR could not be carried out.

¹H-NMR (500 MHz, DMSO-d₆) δ = 7.48-6.47 (m, 20H), 3.79-3.14 (m, 20H)

### Absorption spectrum characteristics of pseudogem-bisMCI[2.2]paracyclophane decolored form

An absorption spectrum of *pseudogem*-bisMCI[2.2]paracyclophane obtained by the above synthesis was confirmed by UV-visible absorption spectrum measurement. *pseudogem*-BisMCI[2.2]paracyclophane was dissolved in benzene (concentration: 2.1 × 10⁻⁵ M), and measurement was carried out at 25°C.

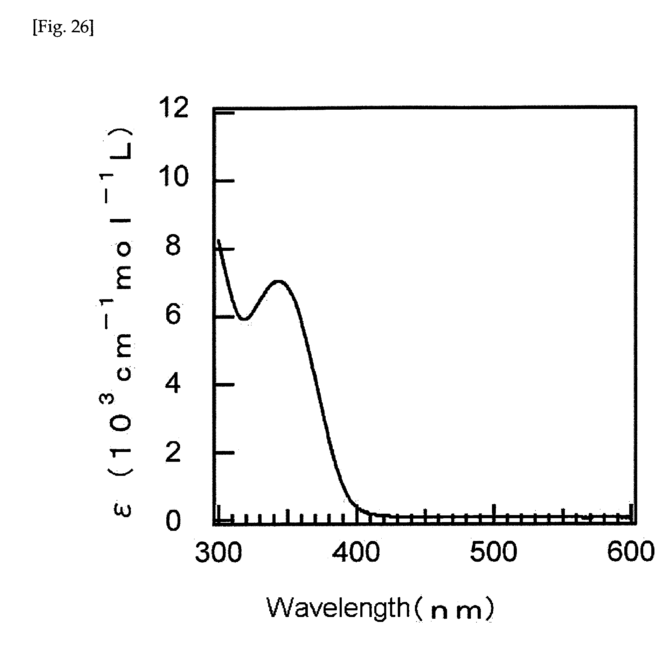

FIG. 26 shows the result of measurement of a visible absorption spectrum. From the result of FIG. 26, *pseudogem*-bisMCI[2.2]paracyclophane had an absorption band in the visible light region of 400 nm and above. The molar extinction coefficient ε at 400 nm was 400. It was confirmed that, compared with the molar extinction coefficient ε (= 0) at 400 nm of the above-mentioned unsubstituted form (*pseudogem-*bisDPI[2.2]paracyclophane), the absorption in the visible light region was increased by the introduction of a substituent.

### Measurement of photochromic properties of pseudogem-bisMCI[2.2]paracyclophane

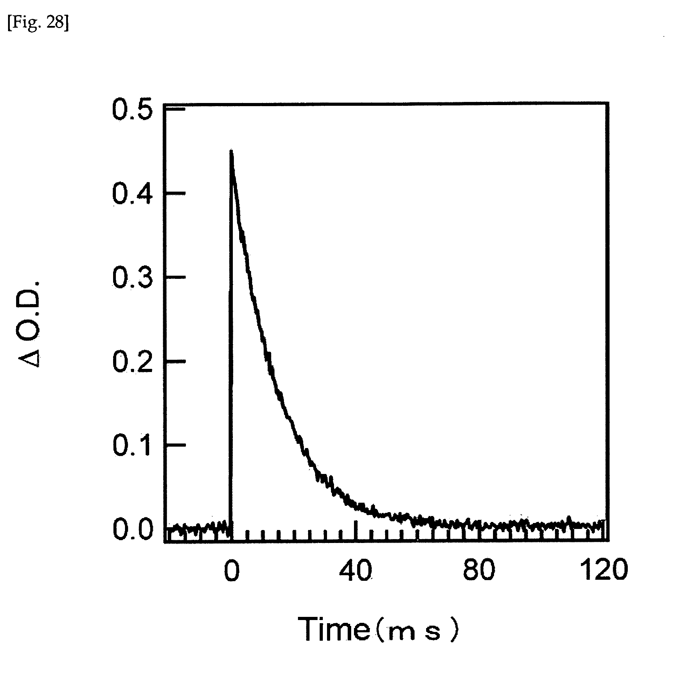

The photochromic properties of *pseudogem*-bisMCI[2.2]paracyclophane obtained by the above synthesis were confirmed by nanosecond laser flash photolysis measurement under the same conditions as in Example 1. *pseudogem-*BisMCI[2.2]paracydophane was dissolved in degassed benzene (concentration: 2.1 × 10⁻⁴ M), under an atmosphere of argon, and measurement was carried out at 25°C.

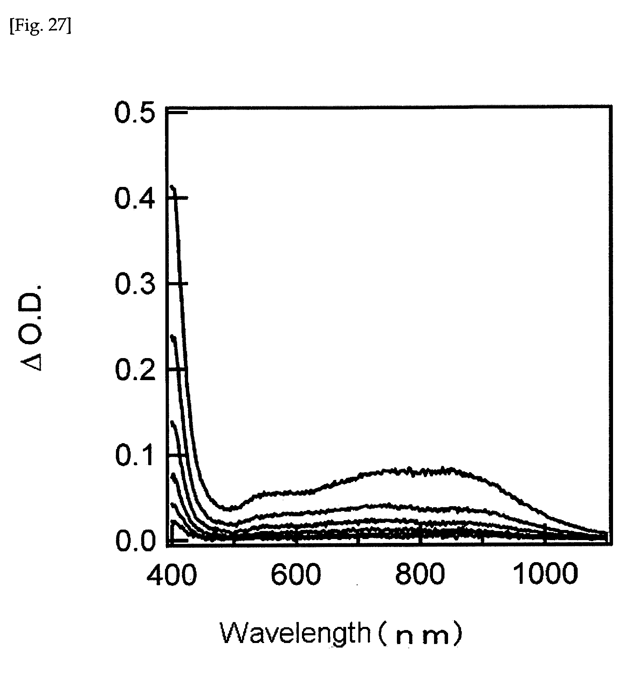

FIG. 27 shows the result of measurement of visible/near-IR absorption spectra under the same conditions as in Example 1. It was confirmed from the result of FIG. 27 that, compared with the absorption spectrum of the above-mentioned unsubstituted form *pseudogem*-bisDPI[2.2]paracyclophane, the absorption spectrum of the photocolored form of *pseudogem*-bisMCI[2.2]paracyclophane resulting from irradiation with UV light shifted to longer wavelength and the shape of the spectrum changed.
Furthermore, FIG. 28 shows the result of measurement of decay over time of the absorption band at 400 nm under the same conditions as in Example 1. It was confirmed from the result of FIG. 28 that, with regard to the change over time of the absorbance at 400 nm of *pseudogem*-bisMCI[2.2]paracyclophane, it decayed with a shorter half life (about 10 ms) than the half life of the above-mentioned unsubstituted form *pseudogem*-bisDPI[2.2]paracyclophane.

### [Example 12]

### Synthesis of paracyclophane-4-phenanthroin-tidazole-13-diphenylimidazole (pseudogem-PHIH-DPIH[2.2]paracyclophane)

*pseudogem*-PHIH-DPIH[2.2]paracyclophane was synthesized in accordance with the synthetic route below using [2.2]paracyclophane-4-diphenylimidazole-13-carbaldehyde obtained in the synthesis of Example 4 above. The synthetic route is shown below.

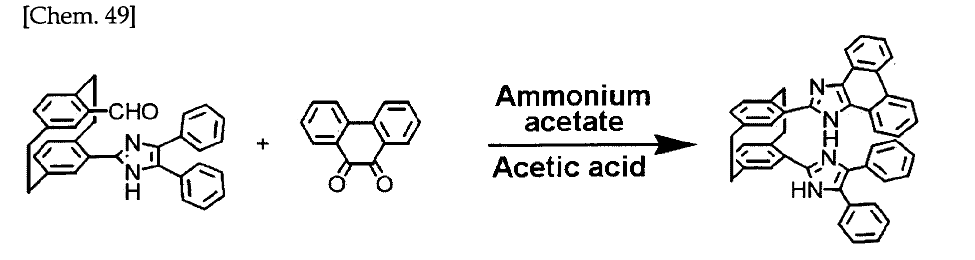

To a 30 mL flask were added [2.2]paracyclophane-4-diphenylimidazole-13-carbaldehyde (0.30 g, 0.66 mmol), 9,10-phenanthrenequinone (0.15 g, 0.33 mmol), ammonium acetate (0.76 g, 9.9 mmol), and 3 mL of acetic acid, and heating was carried out at 90°C for 10 hours. After allowing to cool to room temperature, while cooling with ice, neutralization with aqueous ammonia was carried out, and a precipitate was collected by filtration. This was purified by washing with cooled chloroform, thus giving *pseudogem*-PHIH-DPIH[2.2]paracyclophane (0.36 g, 84%). The results of NMR measurement are shown below.

¹H-NMR (500 MHz, DMSO-d₆) δ = 12.52 (s, 1H), 11.56 (s, 1H), 8.67-8.66 (m, 1H), 8.61 (d, J = 8.5 Hz,1H), 8.32-8.29 (m, 2H), 7.49-7.47 (m, 4H), 7.43-7.29 (m,1H), 7.19 (s, 1H), 6.95 (t, J = 7.5 Hz, 1H), 6.81-6.59 (m, 11H), 6.45 (d, J = 7.0 Hz, 2H), 4.77-4.68 (m, 2H), 3.19-3.14 (m, 6H)

Subsequently, *pseudogem*-PHI-DPI[2.2]paracyclophane, which is a compound of the present invention for the purpose of precise control of photochromic properties, was synthesized in accordance with the synthetic route below.

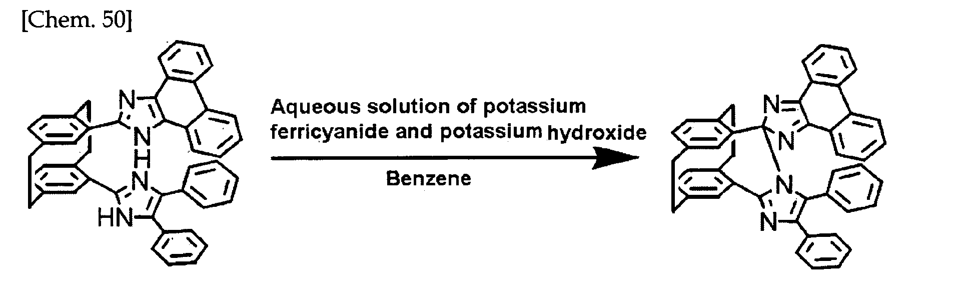

All solvents used in the reaction were degassed by bubbling nitrogen for 30 minutes, and all of the operations were carried out in the dark. In a 200 mL flask, *pseudogem*-PHIH-DPIH[2.2]paracyclophane (0.025 g, 0.06 mmol) obtained by the above synthesis was dissolved in 15 mL of degassed benzene and 10 mL of degassed ethanol, an aqueous solution (20 mL) of potassium ferricyanide (0.4 g, 1.2 mmol) and potassium hydroxide (0.27 g, 4.8 mmol) was added, and vigorous stirring was carried out for 2.5 hours. The benzene layer was extracted, washed well with water, and then vacuum concentrated, thus giving the target *pseudogem-*PHI-DPI[2.2]paracyclophane (0.022 g, 88%). Since the oxidized form is a complicated mixture of isomers, clear assignment by ¹H-NMR could not be carried out. The results of NMR measurement are shown below.

¹H-NMR (500 MHz, DMSO-d₆,) δ = 8.84-8.79 (m, J = 8.0 Hz, 2H), 8.52 (d, J = 8.0 Hz, 1H), 8.31 (d, J = 8.0 Hz, 1H), 7.74-7.67 (m, 3H), 7.56-7.31 (m, 11H), 7.23-7.20 (m, 2H), 6.97 (s,1H), 6.73 (s,1H), 6.65-6.55 (m, 2H), 4.77-4.68 (m, 2H), 3.19-3.14 (m, 6H)

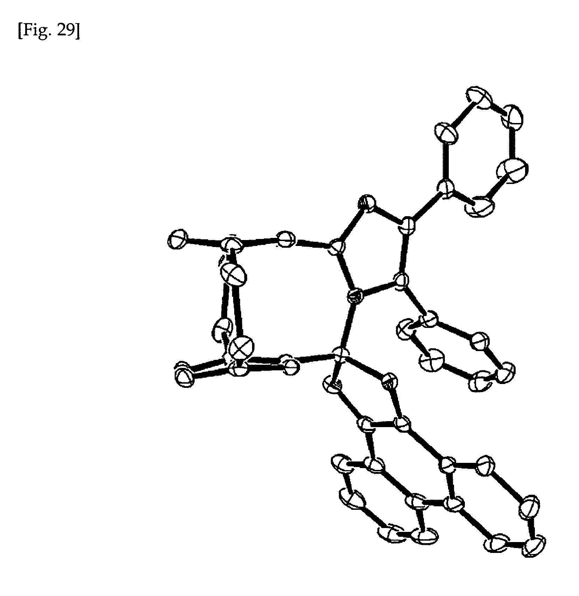

The synthesized *pseudogem*-PHI-DPI[2.2]paracyclophane is subjected to a crystal structural analysisin the same manner as in Example 1. The molecular structure elucidated by the analysis is shown in FIG. 29.

### Measurement of photochromic properties of pseudogem-PHI-DPI[2.2]paracyclophane

The photochromic properties of *pseudogem*-PHI-DPI[2.2]paracyclophane obtained by the above synthesis were confirmed by nanosecond laser flash photolysis measurement under the same conditions as in Example 1. *pseudogem-*PHI-DPI[2.2]paracyclophane was dissolved in degassed benzene (concentration: 10⁻⁴ M) under an atmosphere of argon, and measurement was carried out at 25°C.

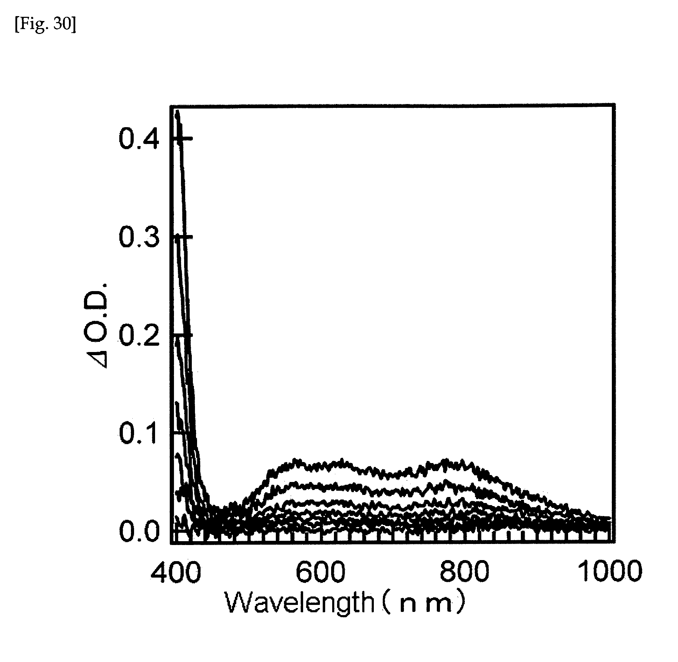

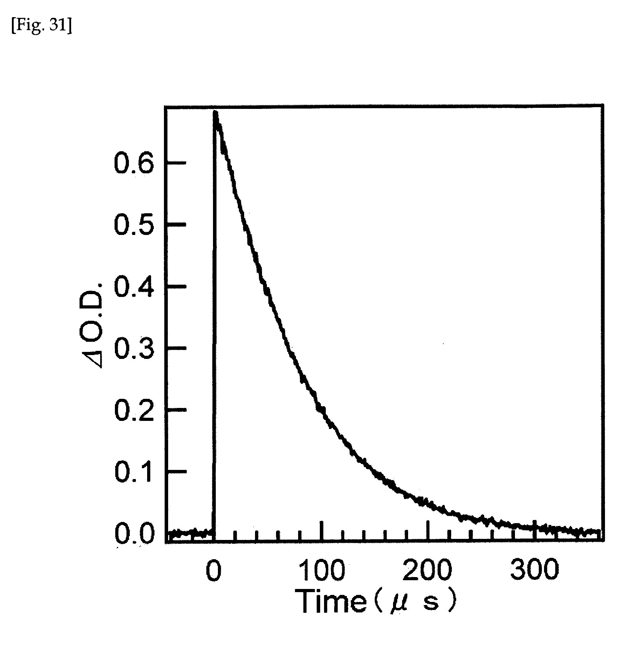

FIG. 30 shows the result of measurement of visible/near-IR absorption spectra under the same conditions as in Example 1. It was confirmed from the result of FIG. 30 that the absorption spectrum of the photocolored form of *pseudogem-*PHI-DPI[2.2]paracyclophane resulting from irradiation with UV light was very similar to the absorption spectrum of the above-mentioned unsubstituted form *pseudogem-*bisDPI[2.2]paracyclophane.
Furthermore, FIG. 31 shows the result of measurement of decay over time of the absorption band at 400 nm under the same conditions as in Example 1. It was confirmed from the result of FIG. 31 that, with regard to the change over time of the absorbance at 400 nm of *pseudogem*-PHI-DPI[2.2]paracyclophane, it decayed with a much shorter half life (about 80 µs) than that of the above-mentioned unsubstituted form *pseudogem*-bisDPI[2.2]paracydophane.

### [Example 13]

### Synthesis of paracyclophane-4-(3-p-dimethylaminophenyl-4-phenyl)imidazole-13-diphenylimidazole (pseudogem-DAIH-DPIH[2.2]paracyclophane)

*pseudogem-*DAIH-DPIH[2.2]paracyclophane was synthesized in accordance with the synthetic route below using [2.2]paracyclophane-4-diphenylimidazole-13-carbaldehyde obtained in the synthesis of Example 4 above. The synthetic route is shown below. 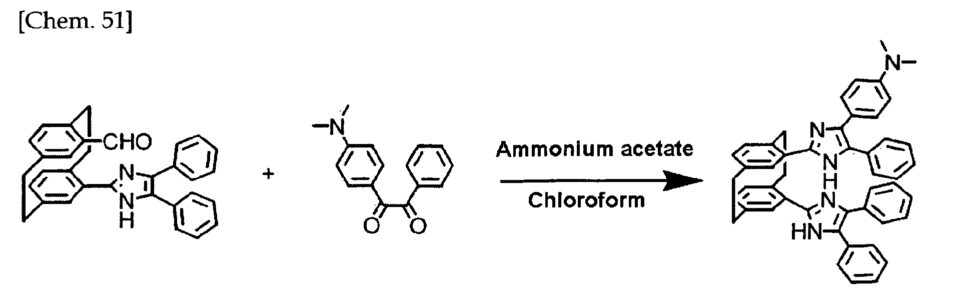 To a reaction vessel were added [2.2]paracyclophane-4-diphenylimidazole-13-carbaldehyde (0.095 g, 0.21 mmol), 4-dimethylaminobenzil (0.065 g, 0.26 mmol), ammonium acetate (0.085 g, 1.1 mmol), and 0.5 mL of chloroform, and refluxing was carried out for 18 hours. After allowing to cool to room temperature, hexane was added to the reaction mixture, and filtration by suction was carried out, thus giving the target *pseudogem*-DAIH-DPIH[2.2]paracyclophane (0.11 g, 77%). The results of NMR measurement are shown below.

¹H-NMR (500 MHz, DMSO-d₆) δ =11.62 (s,1H),11.46 (s,1H), 7.36-7.02 (m, 18H), 6.92 (d, J = 8.6 Hz, 1H), 6.71 (t, J = 6.1 Hz, 2H), 6.61 (t, J = 7.6 Hz, 2H), 6.48 (d, J = 8.6 Hz, 1H), 6.40 (d, J = 8.6 Hz, 1H), 4.59-4.50 (m, 2H), 3.13-3.02 (m, 6H), 2.89 (s, 3H), 2.82((s, 3H)

Subsequently, *pseudogem*-DAI-DPI[2.2]paracyclophane, which is a compound of the present invention for the purpose of control of photochromic properties, was synthesized in accordance with the synthetic route below. 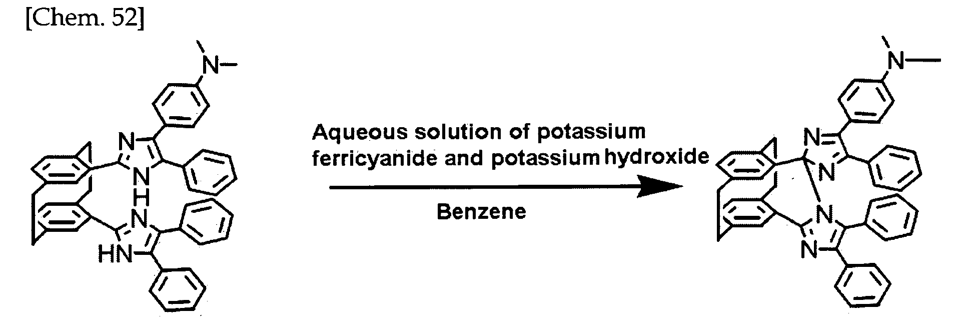 All solvents used in the reaction were degassed by bubbling nitrogen for 30 minutes, and all of the operations were carried out in the dark. In a 200 mL flask, *pseudogem*-DAIH-DPIH[2.2]paracyclophane (0.062 g, 0.091 mmol) obtained by the above synthesis was dissolved in 24 mL of degassed benzene, an aqueous solution (24 mL) of potassium ferricyanide (1.7 g, 5.1 mmol) and potassium hydroxide (0.44 g, 7.9 mmol) was added, and vigorous stirring was carried out for 20 minutes. The benzene layer was extracted, washed well with water, and then vacuum concentrated, thus giving the target *pseudogem*-DAI-DPI[2.2]paracyclophane (0.017 g, 26%). The results of NMR measurement are shown below.

¹H-NMR (500 MHz, DMSO-d₆) δ = 7.44-7.40 (m, 3H), 7.31-7.05 (m, 13H), 6.99 (d, J = 8.0 Hz, 1H), 6.93 (d, J = 9.5 Hz, 1H), 6.81 (d, J = 6.5 Hz, 1H), 6.68 (s, 2H), 6.64 (d, J = 0.5 Hz,1H), 6.53-6.45 (m, 3H), 4.45-4.30 (m, 2H), 3.30-3.05 (m, 7H), 2.97 (s, 3H), 2.92 (s, 3H)

### Absorption spectrum characteristics of pseudogem-DAI-DPI[2.2]paracyclophane decolored form

The absorption spectrum of *pseudogem*-DAI-DPI[2.2]paracyclophane obtained by the above synthesis was confirmed by UV-visible absorption spectrum measurement. *pseudogem*-DAI-DPI[2.2]paracyclophane was dissolved in benzene (concentration: 2.1 × 10⁻⁵ M), and measurement was carried out at 25°C.

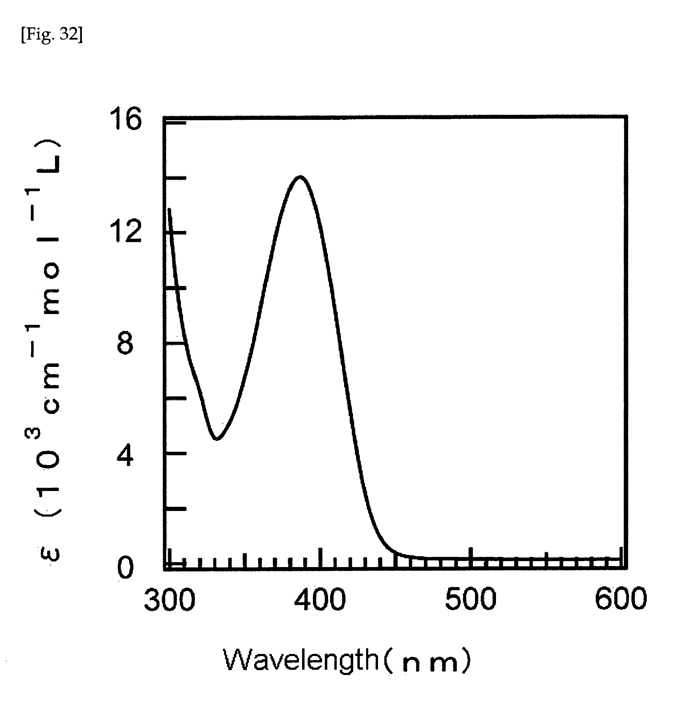

FIG. 32 shows the result of measurement of the visible/near-IR absorption spectrum. From the result of FIG. 32, *pseudogem*-DAI-DPI[2.2]paracyclophane had an absorption band in the visible light region of 400 nm and above. The molar extinction coefficient ε at 400 nm was 14000. It was confirmed that, compared with the molar extinction coefficient ε (= 0) at 400 nm of the above-mentioned unsubstituted form *pseudogem*-bisDPI[2.2]paracyclophane, the absorption in the visible light region was increased by the introduction of a dimethylamino group.

### Measurement of photochromic properties of pseudogem-DAI-DPI[2.2]paracyclophane

The photochromic properties of *pseudogem*-DAI-DPI[2.2]paracyclophane obtained by the above synthesis were confirmed by nanosecond laser flash photolysis measurement under the same conditions as in Example 1. *pseudogem*-DAI-DPI[2.2]paracyclophane was dissolved in degassed benzene (concentration: 2.1 × 10⁻⁵ M) under an atmosphere of argon, and measurement was carried out at 25°C.

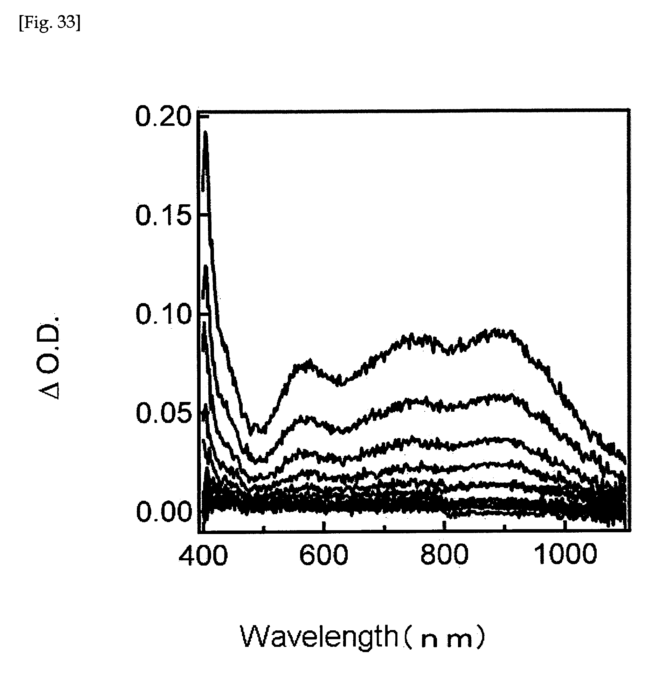

FIG. 33 shows the result of measurement of visible/near-IR absorption spectra under the same conditions as in Example 1. It was confirmed from the result of FIG. 33 that, with regard to the absorption spectrum of the photocolored form of *pseudogem*-DAI-DPI[2.2]paracyclophane resulting from irradiation with UV light, the shape of the spectrum changed compared with the absorption spectrum of the above-mentioned unsubstituted form *pseudogem*-bisDPI[2.2]paracyclophane.

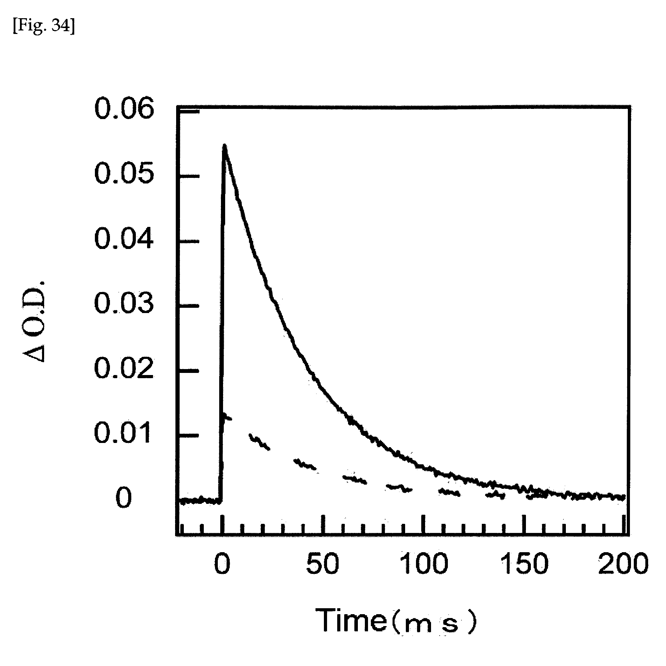

Furthermore, FIG. 34 shows the result of measurement of decay over time of the absorption band at 400 nm under the same conditions as in Example 1. It was confirmed from the result of FIG. 34 that, with regard to the change over time of the absorbance at 750 nm of *pseudogem*-DAI-DPI[2.2]paracyclophane, it decayed with a half life (31 ms) comparable to the half life (33 ms) of the above-mentioned unsubstituted form *pseudogem*-bisDPI[2.2]paracyclophane. When comparing the absorbance (ΔO.D., that is, color density) of the colored form under the same conditions, it was increased by about 4 times by the introduction of a dimethylamino group. This is due to the very large increase in the molar extinction coefficient in the UV region as described above.

### [Example 14]

### Synthesis of acrylate group-containing pseudogem-bisDPIH[2.2]paracyclophane (Ac-pseudogem-bisDPIH[2.2]paracyclophane)

Ac-*pseudogem*-bisDPIH[2.2]paracyclophane was synthesized in accordance with the synthetic route below using [2.2]paracyclophane-4-diphenylimidazole-13-carbaldehyde obtained in the synthesis of Example 4 above. The synthetic route is shown below.

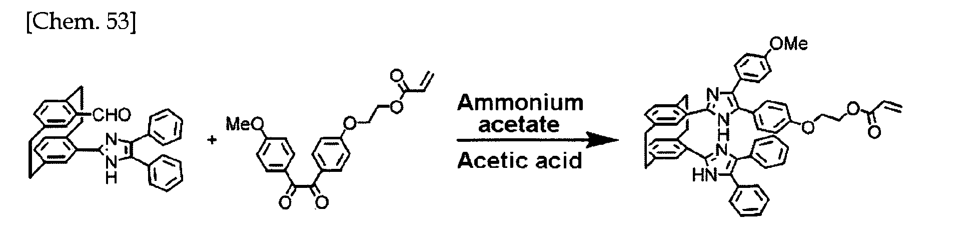

To a 30 mL flask were added [2.2]paracyclophane-4-diphenylimidazole-13-carbaldehyde (0.13 g, 0.29 mmol), an acrylate group-containing benzil (0.10 g, 0.29 mmol), ammonium acetate (0.34 g, 4.4 mmol), *p*-methoxyphenol (3.6 mg, 0.029 mmol), and 2 mL of acetic acid, and heating was carried out at 80°C for 5 hours. After allowing to cool to room temperature, while cooling with ice, neutralization with aqueous ammonia and extraction with dichloromethane were carried out. The organic layer was washed with saturated brine and vacuum concentrated. This mixture was purified by silica gel column chromatography (solvent: hexane/THF = 2/1), thus giving Ac-*pseudogem*-bisDPIH[2.2]paracyclophane (0.11 g, 49%). The results of NMR measurement are shown below.

¹H-NMR (500 MHz, DMSO-d₆) δ = 11.61 (s, 1H), 11.47-11.46 (m, 1H), 7.29-7.26 (m, 2H), 7.19-7.04 (m, 12H), 7.00-6.98 (m, 2H), 6.73-6.57 (m, 8H), 6.39-6.59 (m, 1H), 6.27-6.22 (m, 1H), 6.00-5.97 (m,1H), 4.58-4.47 (m, 2H), 4.57-4.39 (m, 2H), 4.20-4.10 (m, 2H), 3.73-3.66 (m, 3H), 3.13-3.04 (m, 6H)

Subsequently, Ac-*pseudogem*-bisDPI[2.2]paracyclophane, which is a compound of the present invention for the purpose of making a polymer, was synthesized in accordance with the synthetic route below.

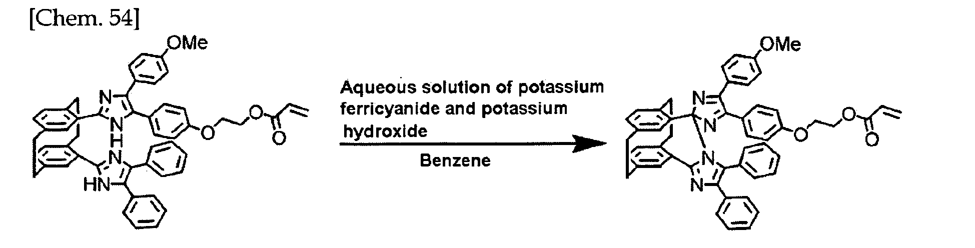

All solvents used in the reaction were degassed by bubbling nitrogen for 30 minutes, and all of the operations were carried out in the dark. In a 100 mL flask, the Ac-*pseudogem*-bisDPIH[2.2]paracyclophane (0.050 g, 0.060 mmol) obtained by the above synthesis was dissolved in 10 mL of degassed benzene, an aqueous solution (7 mL) of potassium ferricyanide (1.0 g, 3.2 mmol) and potassium hydroxide (0.36 g, 6.3 mmol) was added, and vigorous stirring was carried out for 30 minutes. The benzene layer was extracted, washed well with water, and then vacuum concentrated, thus giving the target Ac-*pseudogem*-bisDPI[2.2]paracyclophane (0.050 g, quant.). Since the oxidized form is a complicated mixture of isomers, clear assignment by ¹H-NMR could not be carried out.

¹H-NMR (500 MHz, DMSO-d₆) δ = 7.48-7.44 (m, 1H), 7.28-7.20 (m, 7H), 7.14-6.99 (m, 9H), 6.93-6.85 (m, 3H), 6.72 (s, 2H), 6.57-6.51 (m, 2H), 6.42-6.37 (m, 1H), 6.29-6.23 (m, 1H), 6.03-6.00 (m,1H), 4.51-4.28 (m, 6H), 3.84-3.80 (m, 3H), 3.29-3.00 (m, 6H)

### Measurement of photochromic properties of Ac-pseudogem-bisDPI[2.2]paracyclophane

The photochromic properties of the Ac-*pseudogem*-bisDPI[2.2]paracyclophane obtained by the above synthesis were confirmed by nanosecond laser flash photolysis measurement under the same conditions as in Example 1. Ac*-pseudogem-*bisDPI[2.2]paracyclophane was dissolved in degassed benzene (concentration: 10⁻⁴ M), and measurement was carried out at 25°C under an atmosphere of argon.

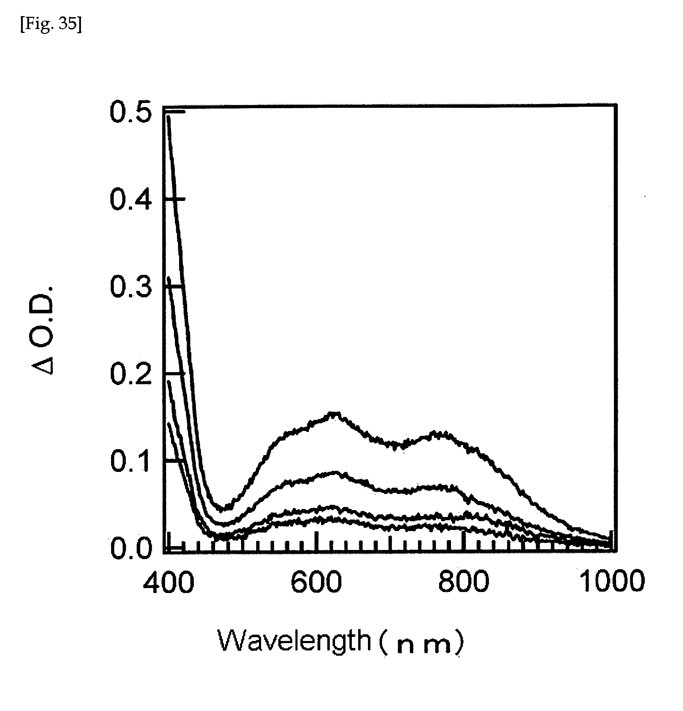

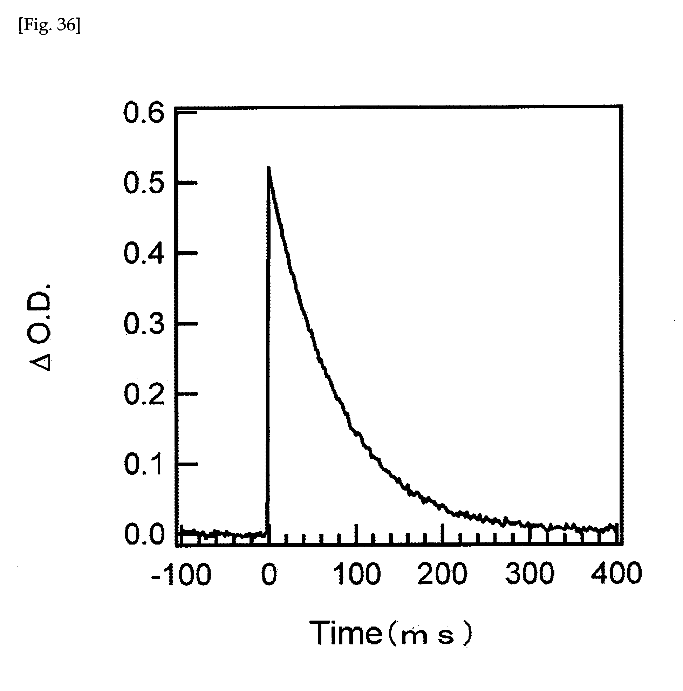

FIG. 35 shows the result of measurement of visible/near-IR absorption spectra under the same conditions as in Example 1. It was confirmed from the result of FIG. 35 that, compared with the absorption spectrum of *pseudogem-*BMPI-DPI[2.2]paracyclophane, which is an analogous compound mentioned above, the absorption spectrum of the photocolored form of Ac-*pseudogem-*bisDPI[2.2]paracyclophane resulting from irradiation with UV light was substantially the same.
Furthermore, FIG. 36 shows the result of measurement of decay over time of the absorption band at 400 nm under the same conditions as in Example 1. It was confirmed from the result of FIG. 36 that, with regard to the change over time of the absorbance at 400 nm of Ac-*pseudogem-*bisDPI[2.2]paracyclophane, it decayed with a half life (about 50 ms) comparable to that of *pseudogem*-BMPI-DPI[2.2]paracyclophane, which is an analogous compound mentioned above.

### [Example 15]

### Synthesis of poly-pseudogem-bisDPIH[2.2]paracyclophane

Poly-*pseudogem-*bisDPIH[2.2]paracyclophane was synthesized in accordance with the synthetic route below using the Ac-*pseudogem*-bisDPI[2.2]paracyclophane obtained by the synthesis of Example 13 above. The synthetic route is shown below.

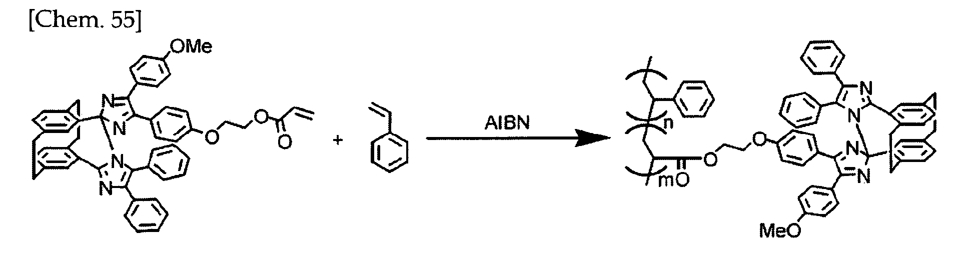

Ac-*pseudogem*-bisDPI[2.2]paracyclophane (0.025 g, 0.032 mmol), styrene (36 µL, 0.32 mmol), and 2,2'-azobisisobutyronitrile (1.6 mg, 9.5 µmol) were added to a sealable glass ampoule. After this was subjected to freezing/degassing about 10 times, it was sealed by fusing under vacuum. The sealed tube was heated at 80°C for 2 hours. Subsequently, the sealed tube was taken out, cooled in a liquid nitrogen bath to stop polymerization, the seal was opened, and the contents were diluted with dichloromethane. This was subjected to reprecipitation in methanol, and a solid thus precipitated was collected by filtration. The solid thus obtained was washed with methanol and hexane. thus giving the target poly-*pseudogem-*bisDPIH[2.2]paracyclophane (0.033 g, 57%).

The results of NMR measurement are shown below. From the results, it was confirmed that polymerization progressed since the signal due to acrylate disappeared. Furthermore, it was clear from the integration ratio thereof that the copolymerization ratio (m/n) was 1/8, and 1 unit of photochromic unit was introduced per 8 units of styrene. It was confirmed from gel filtration chromatography (solvent: tetrahydrofuran) that a polymer having a weight-average molecular weight Mw = 135000, a number-average molecular weight Mn = 93000, and a molecular weight distribution Mw/ Mn = 1.46 was obtained. It was confirmed from the result of differential scanning calorimetry analysis that the glass transition temperature of the poly-*pseudogem*-bisDPIH[2.2]paracyclophane was 110°C, which was comparable to that of polystyrene (glass transition temperature: 100°C).

¹H-NMR (500 MHz, CDCl₃) δ = 7.48 (br, 2H), 7.37-6.31 (br, 143H), 4.55 (br, 2H), 4.03-3.49 (br, 14H), 3.37-2.89 (br, 14H), 2.28-1.08 (br, 75H)

### Measurement of photochromic properties of poly-pseudogem-bisDPIH[2.2]paracyclophane solution

The photochromic properties in the solution state of the poly*-pseudogem-*bisDPIH[2.2]paracyclophane obtained by the above synthesis were confirmed by nanosecond laser flash photolysis measurement under the same conditions as in Example 1. Poly-*pseudogem*-bisDPIH[2.2]paracyclophane was dissolved in degassed benzene (concentration: 0.1 mg /mL), and measurement was carried out at 25°C under an atmosphere of argon.

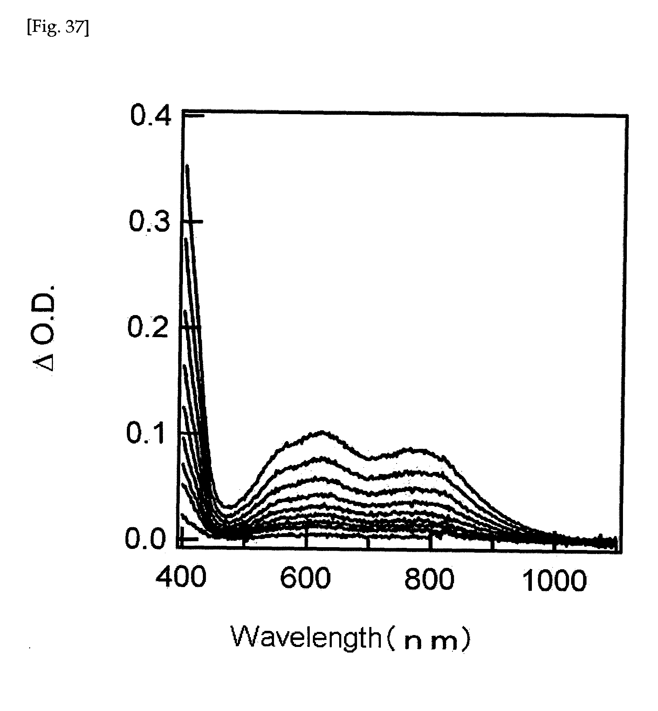

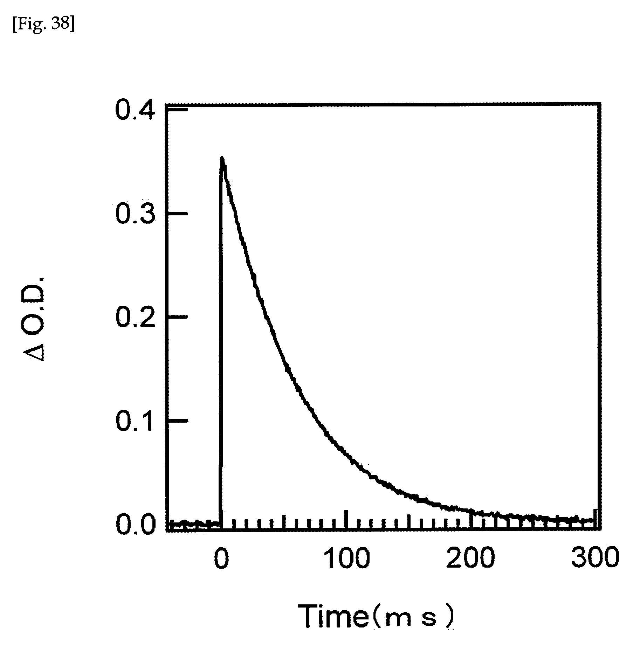

FIG. 37 shows the result of measurement of visible/near-IR absorption spectra under the same conditions as in Example 1. It was confirmed from the result of FIG. 37 that the absorption spectrum of the photocolored form of poly*-pseudogem-*bisDPIH[2.2]paracyclophane resulting from irradiation with UV light was substantially the same as the absorption spectrum of Ac*-pseudogem-*bisDPI[2.2]paracyclophane, which is the above-mentioned monomer.
Furthermore, FIG. 38 shows the result of measurement of decay over time of the absorption band at 400 nm under the same conditions as in Example 1. It was confirmed from the result of FIG. 38 that, with regard to the change over time of the absorbance at 400 nm of poly-*pseudogem*-bisDPIH[2.2]paracyclophane, it decayed with a half life (about 50 ms) comparable to that of Ac*-pseudogem-*bisDPI[2.2]paracyclophane, which is the analogous compound mentioned above.

### Measurement of photochromic properties of poly-pseudogem-bisDPIH[2.2]paracyclophane thin film

The photochromic properties in a thin film state of the poly*-pseudogem-*bisDPIH[2.2]paracyclophane obtained by the above synthesis were confirmed by nanosecond laser flash photolysis measurement under the same conditions as in Example 1. 5 mg of poly-*pseudogem-*bisDPIH[2.2]paracyclophane was dissolved in chloroform (concentration: 50 mg /mL), a thin film was prepared on a quartz plate by a spin coating method, and measurement was carried out at 25°C.

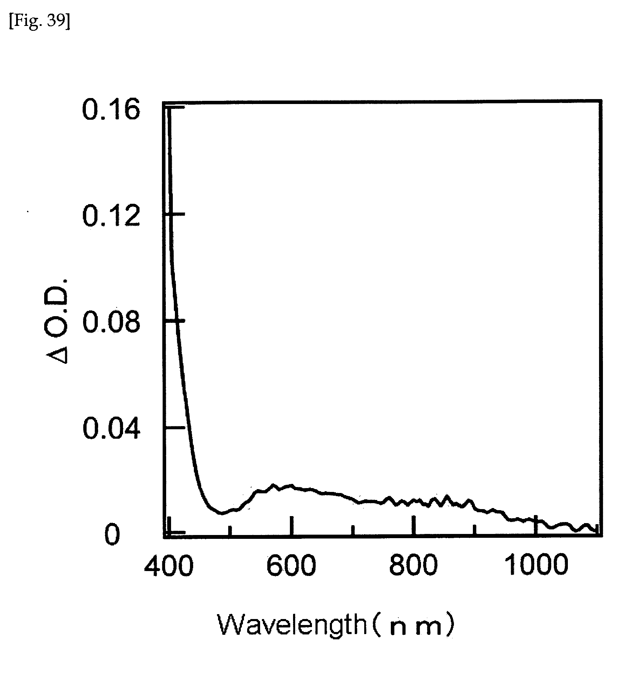

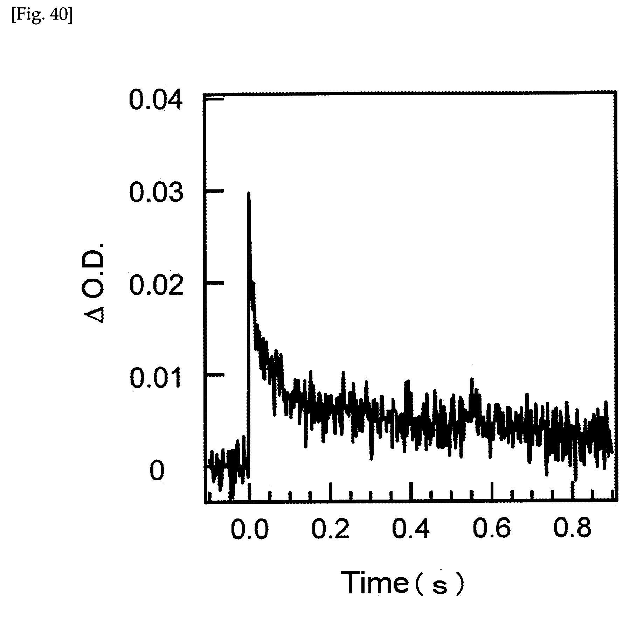

FIG. 39 shows the result of measurement of the visible/near-IR absorption spectrum under the same conditions as in Example 1. It was confirmed from the result of FIG. 39 that the absorption spectrum of the photocolored form of poly-pseudogem-bisDPIH[2.2]paracyclophane resulting from irradiation with UV light had broad absorption in the visible light region that was similar to the absorption spectrum of Ac*-pseudogem*-bisDPI[2.2]paracyclophane, which is the above-mentioned monomer.
Furthermore, FIG. 40 shows the result of measurement of decay over time of the absorption band at 400 nm under the same conditions as in Example 1. It was confirmed from the result of FIG. 40 that, with regard to the change over time of absorbance at 400 nm of poly*-pseudogem-*bisDPIH[2.2]paracyclophane, it decayed completely in about 1 second.

### [Example 16]

### Synthesis of pseudogem-bisDPI[2.2]paracyclophane dimer

*pseudogem*-BisDPIH[2.2]paracyclophane dimer was synthesized in accordance with the synthetic route below using [2.2]paracyclophane-4-diphenylimidazole-13-carbaldehyde obtained in the synthesis of Example 4 above. 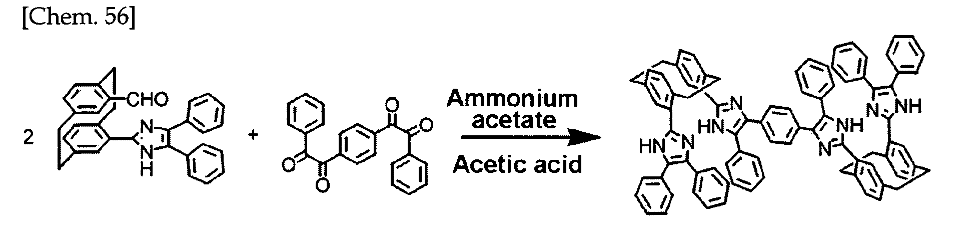 To a 30 mL flask were added [2.2]paracyclophane-4-diphenylimidazole-13-carbaldehyde (0.14 g, 0.31 mmol), 1,4-bisbenzil (0.053 g, 0.15 mmol), ammonium acetate (0.48 g, 6.2 mmol), and 5 mL of acetic acid, and heating and refluxing were carried out for 20 hours. After allowing to cool to room temperature, while cooling with ice, neutralization with aqueous ammonia and filtration under reduced pressure were carried out. The filtrate was washed with water and then dried under vacuum. This reaction mixture was purified by silica gel column chromatography (solvent: hexane/THF = 2/1 → 1/1), thus giving *pseudogem-*bisDPIH[2.2]paracyclophane dimer (0.070 g, 38%). The results of NMR measurement are shown below.

¹H-NMR (500 MHz, DMSO-d₆) δ: 11.67-11.61 (m, 4H), 7.27-6.64 (m, 46H), 4.80-4.51 (br, 2H), 3.31-3.09 (br, 6H).

Subsequently, *pseudogem*-bisDPI[2.2]paracyclophane dimer was synthesized in accordance with the synthetic route below. 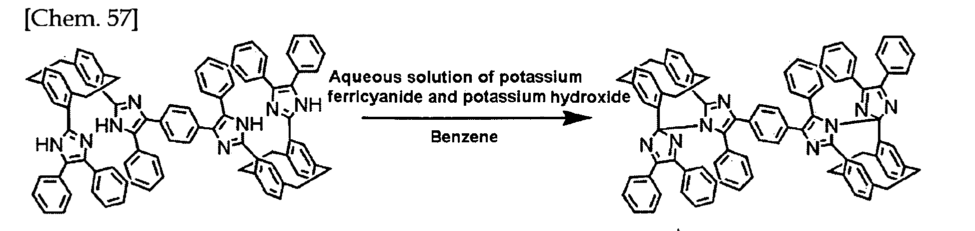 All solvents used in the reaction were degassed by bubbling nitrogen for 30 minutes, and all of the operations were carried out in the dark. In a 200 mL flask, the *pseudogem-*bisDPIH[2.2]paracyclophane dimer obtained by the synthesis above (0.045 g, 0.041 mmol) was dissolved in 20 mL of degassed benzene, an aqueous solution (7.5 mL) of potassium ferricyanide (1.3 g, 4.1 mmol) and potassium hydroxide (0.55 g, 9.8 mmol) was added, and vigorous stirring was carried out for 30 minutes. The benzene layer was extracted, washed well with water, and then vacuum concentrated. The reaction mixture was purified by silica gel column chromatography (solvent: hexane/THF = 2/1) and then subjected to size exclusion chromatography (solvent: THF), thus giving the target *pseudogem-*bisDPI[2.2]paracyclophane dimer (0.012 g, 27%).

### Photochromic properties of pseudogem-bisDPI[2.2]paracyclophane dimer

The photochromic properties of *pseudogem*-bisDPI[2.2]paracyclophane dimer were confirmed by nanosecond laser flash photolysis measurement under the same conditions as in Example 1. *pseudogem*-BisDPI[2.2]paracyclophane dimer was dissolved in degassed benzene (concentration 1.07 × 10⁻⁴ mol/L), the atmosphere was changed to argon, and measurement was then carried out. It was confirmed that, with regard to the change over time of the absorbance at 400 nm, it decayed with a half life (about 40 ms) comparable to that of the *pseudogem-*bisDPI[2.2]paracyclophane, which is a monomer, as shown in FIG. 8. Furthermore, it was confirmed that the absorption spectrum of the photocolored form was substantially the same as that of *pseudogem*-bisDPI[2.2]paracyclophane as shown in FIG. 9, whereas the absorbance per unit molarity increased about 2 times compared with *pseudogem*-bisDPI[2.2]paracyclophane.

### [Example 17]

### Synthesis of pseudogem-bisDPI[2.2]paracyclophane trimer

*pseudogem*-BisDPIH[2.2]paracyclophane trimer was synthesized in accordance with the synthetic route below using [2.2]paracyclophane-4-diphenylimidazole-13-carbaldehyde obtained in the synthesis of Example 4 above. 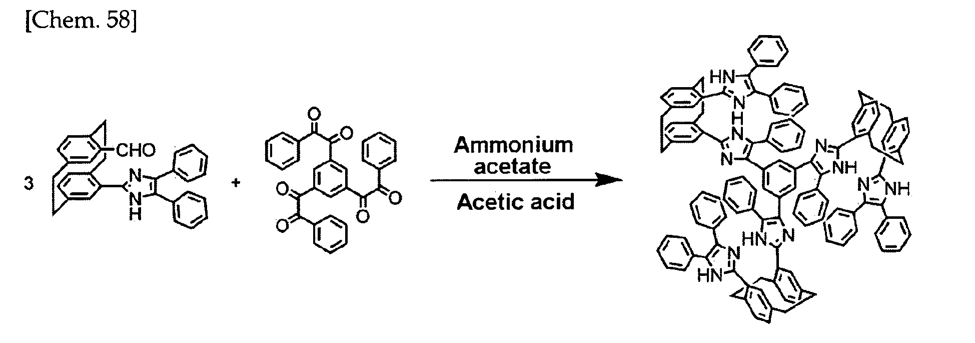 To a 50 mL flask were added [2.2]paracyclophane-4-diphenylimidazole-13-carbaldehyde (0.40 g, 0.88 mmol), 1,3,5-trisbenzil (0.47 g, 0.29 mmol), ammonium acetate (0.85 g, 6.5 mmol), and 10 mL of acetic acid, and heating and refluxing were carried out for 3 days. After allowing to cool to room temperature, while cooling with ice, neutralization with aqueous ammonia and filtration under reduced pressure were carried out. The filtrate was washed with water and then dried under vacuum. A chloroform-soluble portion of this reaction mixture was subjected to silica gel column chromatography (solvent: hexane/THF = 3/2) and then to size exclusion chromatography (solvent: THF), thus giving *pseudogem-*bisDPIH[2.2]paracyclophane trimer (0.014 g, 2.6%). The results of NMR measurement are shown below.

¹H-NMR (500 MHz, CDCl₃): δ: 8.0-6.0 (br), 3.8-2.8 (br).

Subsequently, *pseudogem*-bisDPI[2.2]paracyclophane trimer was synthesized in accordance with the synthetic route below. 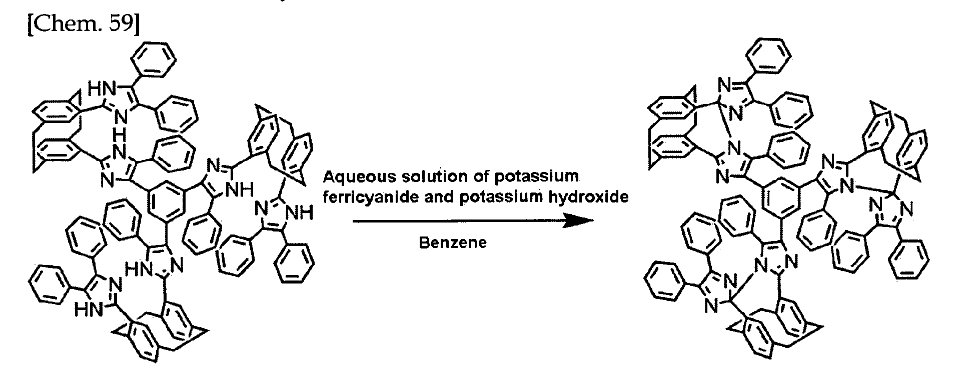 All solvents used in the reaction were degassed by bubbling nitrogen for 30 minutes, and all of the operations were carried out in the dark. In a 100 mL flask, the *pseudogem*-bisDPIH[2.2]paracyclophane trimer obtained in the synthesis above (0.014 g, 7.7 µmol) was dissolved in 15 mL of degassed benzene, an aqueous solution (10 mL) of potassium ferricyanide (0.52 g, 2.1 mmol) and potassium hydroxide (0.53 g, 9.5 mmol) was added, and vigorous stirring was carried out for 1 hour. The benzene layer was extracted, washed well with water, and then vacuum concentrated. The reaction mixture was purified by silica gel column chromatography (solvent: ethyl acetate/dichloromethane = 1/4), thus giving the target *pseudogem-*bisDPI[2.2]paracyclophane trimer (0.0098 g, 73%). The results of NMR measurement are shown below.

¹H NMR (500 MHz, C₆D₆): δ: 8.0-6.0 (br), 4.9-4.5 (br), 3.5-2.5 (br).

### Photochromic properties of pseudogem-bisDPI[2.2]paracyclophane trimer

The photochromic properties of the *pseudogem*-bisDPI[2.2]paracyclophane trimer were confirmed by laser flash photolysis measurement under the same conditions as in Example 1. *pseudogem*-BisDPI[2.2]paracyclophane trimer was dissolved in degassed benzene (concentration 5.64 × 10⁻⁵ mol/L), the atmosphere was changed to argon, and measurement was then carried out. It was confirmed that, with regard to the change over time of the absorbance at 400 nm, it decayed with a half life (about 60 ms) comparable to that of *pseudogem*-bisDPI[2.2]paracyclophane, which is a monomer, as shown in FIG. 10. Furthermore, it was confirmed that the absorption spectrum of the photocolored form was substantially the same as that of *pseudogem*-bisDPI[2.2]paracyclophane as shown in FIG. 11, whereas the change in absorbance per unit molarity increased about 3 times compared with *pseudogem-*bisDPI[2.2]paracyclophane.

### [Industrial Applicability]

Compared with a conventional photochromic material, a photochromic material containing the bridged hexaarylbisimidazole compound of the present invention has excellent thermal stability and stability over time and has rapid color switching characteristics and high color density, and in particular the photochromic property of the color disappearing visually at the same time as irradiation with light stops can be realized. Furthermore, by making an asymmetric structure in which the structures of the two triarylimidazole moieties are optimally designed according to the intended application and purpose, precise control of tone and density of coloration, etc. becomes possible. Moreover, compared with a conventional production method, the production method of the present invention in which synthesis is carried out using the precursor compound of the present invention as a key compound can increase the degree of freedom in terms of molecular structure design and synthesis, and synthesis of photochromic compounds having various structures such as asymmetric compounds or multimer compounds can be realized. Therefore, the bridged hexaarylbisimidazole compound of the present invention, the method for producing the compound, and the precursor compound used in the production method are industrially highly applicable as an excellent photochromic compound and production method therefor in a wide field including sunlight-sensitive light control materials, optical switch elements, optical information display devices, etc.

## Claims

1. A compound represented by general formula (1) below 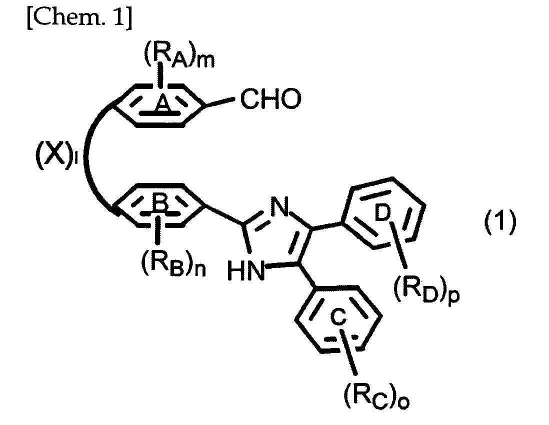 wherein, the two aryl groups A and B are bridged to each other via carbon atoms by means of a bridging group X (excluding 1,8-naphthalenylene),
1 is an integer of 1 to 5,
when the bridging group X contains hydrogen atoms, said hydrogen atoms may be mutually independently replaced by one or more substituents Rx,
the Rxs are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons), the four aryl groups A to D mutually independently may or may not have substituents R_{A} to R_{D},
m and n are mutually independently an integer of 0 to 4,
o and p are mutually independently an integer of 0 to 5,
the substituents R_{A} and R_{B} are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom,
a halogen atom, or a straight-chain or branched alkoxy group having 1 to 8 carbons),
the substituents R_{C} and R_{D} are independently identical to or different from each other and are one or more substituents selected from the group consisting of a substituent having the same meaning as that of the above substituents R_{A} and R_{B}, a substituent represented by partial structural formula (i) below 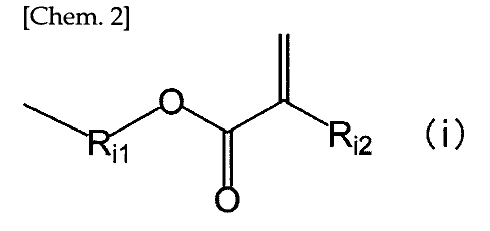 (here, Rᵢ₁ denotes an alkylene group or alkoxylene group having 1 to 20 carbons, and
Rᵢ₂ denotes hydrogen or an alkyl group having 1 to 3 carbons),
a substituent represented by partial structural formula (ii) below 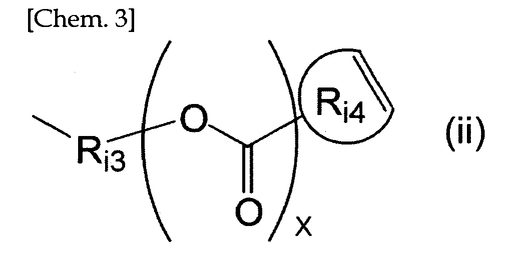 (here, Rᵢ₃ denotes an alkylene group or alkoxylene group having 1 to 20 carbons, Rᵢ₄ denotes a cyclic olefin having a total number of carbons and silicons of 5 to 10, and x denotes 0 or 1), and a substituent represented by partial structural formula (iii) below 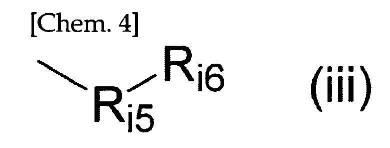 (here, Rᵢ₅ denotes an alkylene group or alkoxylene group having 1 to 20 carbons, and Rᵢ₆ denotes an ethylene group or an acetylene group),
the above substituents R_{A} to R_{D} furthermore may or may not form an aliphatic or aromatic ring together with the carbon atom to which they are bonded and another substituent, and said ring may or may not further have a substituent having the same meaning as that of the above substituents R_{C} and R_{D}.

2. The compound according to Claim 1, wherein the bridging group is a bridging group that does not conjugate with a triarylimidazolyl radical (TAIR).

3. The compound according to Claim 1 or 2, wherein m and n are 0 and the bridging group X is unsubstituted, or the substituents R_{A}, R_{B}, and Rx are methyl groups.

4. The compound according to any one of Claim 1 to 3, wherein it is represented by general formula (la) below.

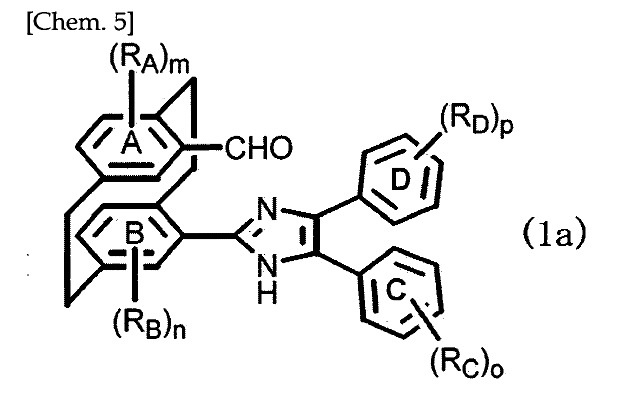

5. A compound represented by general formula (2) below 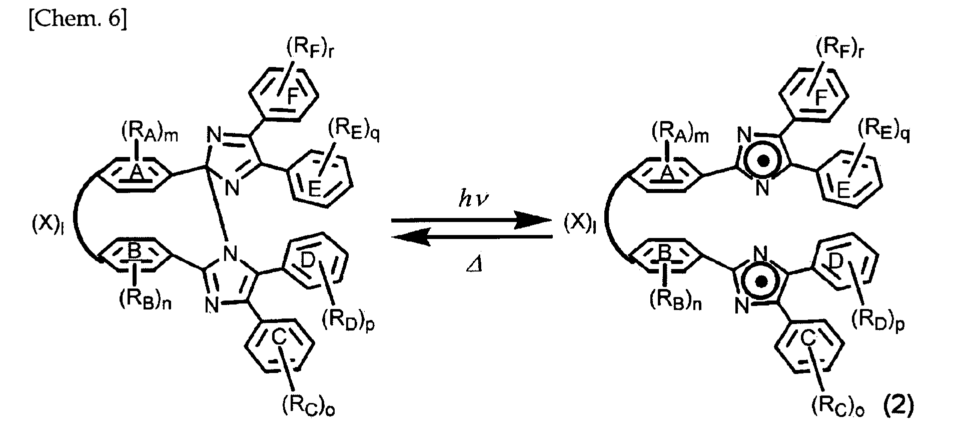 wherein, the two aryl groups A and B are bridged to each other via carbon atoms by means of a bridging group X (excluding 1,8-naphthalenylene),
1 is an integer of 1 to 5,
when the bridging group X contains hydrogen atoms, said hydrogen atoms may mutually independently be replaced by one or more substituents Rx,
the Rxs are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons), the six aryl groups A to F may or may not, mutually independently, have substituents R_{A} to R_{F},
m and n are mutually independently an integer of 0 to 4,
o to r are mutually independently an integer of 0 to 5,
the substituents R_{A} and R_{B} are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, and a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons), the substituents R_{C} to R_{F} are independently identical to or different from each other and are one or more substituents selected from the group consisting of a substituent having the same meaning as that of the above substituents R_{A} and R_{B}, a substituent represented by partial structural formula (i) below 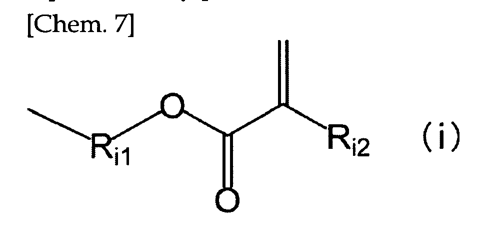 (here, Rᵢ₁ denotes an alkylene or alkoxylene group having 1 to 20 carbons, and Rᵢ₂ denotes hydrogen or an alkyl group having 1 to 3 carbons),
a substituent represented by partial structural formula (ii) below 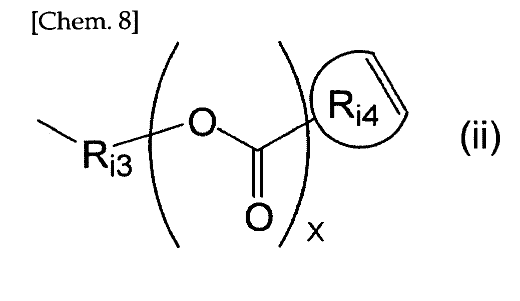 (here, Rᵢ₃ denotes an alkylene or alkoxylene group having 1 to 20 carbons, Rᵢ₄ denotes a cyclic olefin having a total number of carbons and silicons of 5 to 10, and x denotes 0 or 1), and a substituent represented by partial structural formula (iii) below 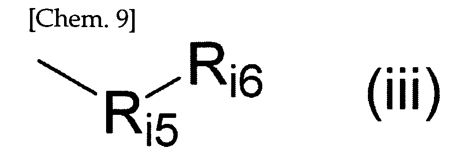 (here, Rᵢ₅ denotes an alkylene or alkoxylene group having 1 to 20 carbons, and Rᵢ₆ denotes an ethylene group or an acetylene group),
the substituents R_{A} to R_{F} may or may not form an aliphatic or aromatic ring together with the carbon atom to which they are bonded and another substituent, and said ring may or may not further have a substituent having the same meaning as that of the above substituents R_{C} to R_{F}.

6. The compound according to Claim 5, wherein at least one of the substituents R_{A} to R_{F} is not hydrogen.

7. The compound according to Claim 5 or 6, wherein it is asymmetric in that the structure of a triarylimidazole moiety containing the aryl group A and the structure of a triarylimidazole moiety containing the aryl group B are different.

8. The compound according to any one of Claims 5 to 7, wherein the bridging group is a bridging group that does not conjugate with a triarylimidazolyl radical (TAIR).

9. The compound according to any one of Claims 5 to 8, wherein the bridging group is one or more types of bridging group selected from the group consisting of a -CH₂CH₂- group, a -CH₂CH₂CH₂- group, a -CH₂CH₂CH₂CH₂- group, an -Si(CH₃)₂Si(CH₃)₂- group, an -Si(CH₃)₂Si(CH₃)₂Si(CH₃)₂- group, an - Si(CH₃)₂Si(CH₃)₂Si(CH₃)₂Si(CH₃)₂- group, an -SiH₂OSiH₂- group, an - Si(CH₃)₂OSi(CH₃)₂- group, an -Si(CH₃CH₂)₂OSi(CH₂CH₃)₂- group, a -CH₂SCH₂-group, a -CH₂OCH₂- group, an -OCH₂CH₂O- group, and a -CH₂OCH₂CH₂OCH₂-group.

10. The compound according to any one of Claims 5 to 8, wherein m and n are 0 and the bridging group X is unsubstituted, or the substituents R_{A}, R_{B}, and Rx are methyl groups.

11. The compound according to any one of Claims 5 to 10, wherein general formula (2) according to Claim 5 is represented by general formula (2a) below 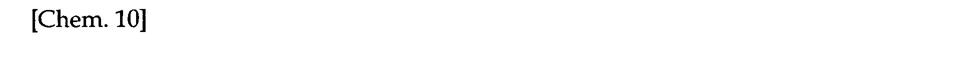 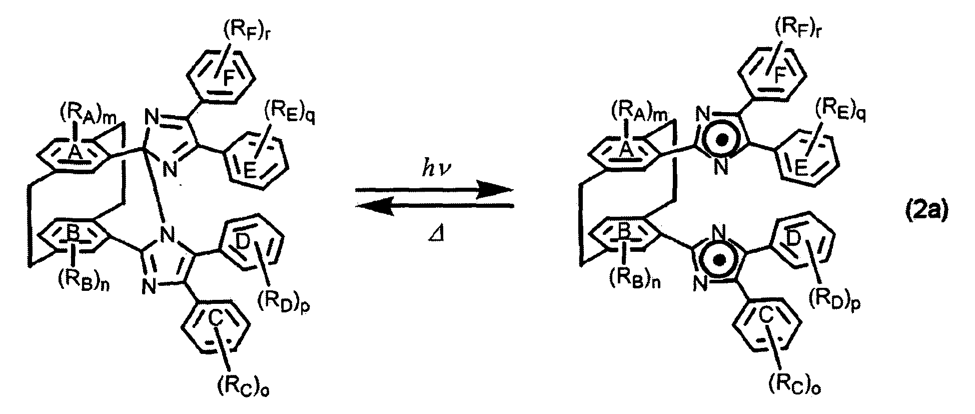 wherein, m and n are mutually independently 0 to 3,
o to r are mutually independently an integer of 0 to 5, and
the substituents R_{A}, R_{B}, and R_{C} to R_{F} are independently identical to or different from each other and have the same meanings as those of the substituents R_{A}, R_{B}, and R_{C} to R_{F} respectively in general formula (2) according to Claim 5.

12. A compound represented by general formula (3) below 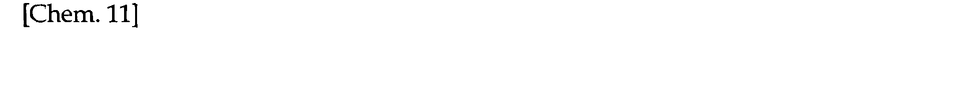 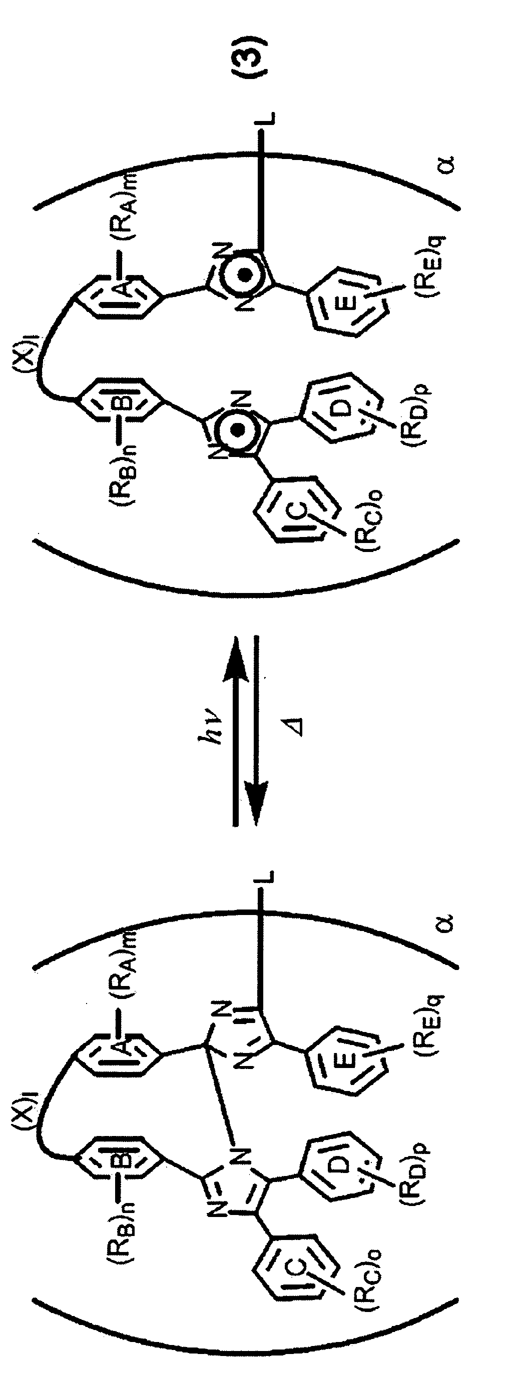 wherein, the two aryl groups A and B are bridged to each other via carbon atoms by means of a bridging group X (excluding 1,8-naphthalenylene),
1 is an integer of 1 to 5,
when the bridging group X contains hydrogen atoms, said hydrogen atoms may mutually independently be replaced by one or more substituents Rx,
α is an integer of 1 to 9,
the linking group L is a monocyclic or polycyclic aromatic compound comprising 1 to 12 aromatic rings having 5 to 8 carbon atoms per ring structure,
the five aryl groups in the bisimidazole skeleton-containing structural unit may or may not, mutually independently, have substituents R_{A}, R_{B}, and R_{C} to R_{E},
all of the substituents R_{A}, R_{B}, R_{C} to R_{E}, and Rx are independently identical to or different from each other and have the same meanings as those of the substituents R_{A}, R_{B}, R_{C} to R_{E}, and Rx respectively in general formula (2) according to Claim 5,
m and n are mutually independently an integer of 0 to 4,
o to r are mutually independently an integer of 0 to 5,
the substituents R_{A}, R_{B}, and R_{C} to R_{E} may or may not form an aliphatic or aromatic ring together with the carbon atom to which they are bonded and another substituent, and said ring and the aromatic ring of the linking group L may or may not further have a substituent R_{L}.

13. The compound according to Claim 12, wherein general formula (3) according to Claim 12 is represented by general formula (3b) below 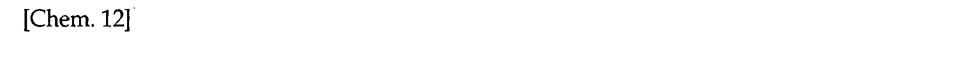 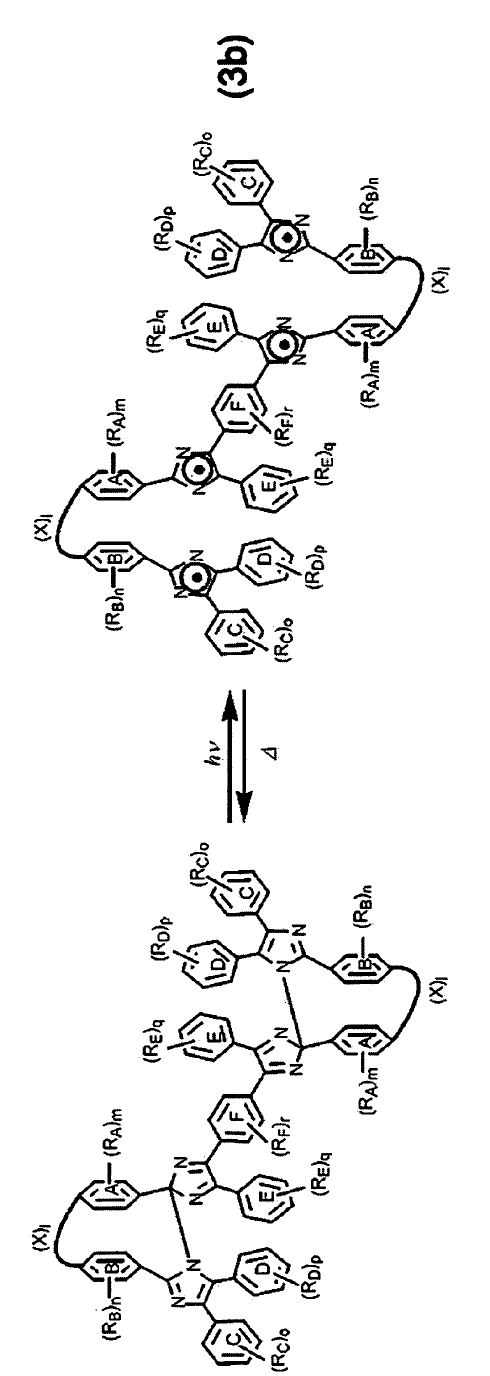 wherein, the 11 aryl groups may or may not have substituents R_{A}, R_{B}, and R_{C} to R_{F},
all of the substituents R_{A}, R_{B}, and R_{C} to R_{F} of the aryl groups are independently identical to or different from each other and have the same meanings as those of the substituents R_{A}, R_{B}, and R_{C} to R_{F} respectively in general formula (2) according to Claim 5,
m, n, and r are mutually independently an integer of 0 to 4, and
o to q are mutually independently an integer of 0 to 5.

14. The compound according to Claim 12, wherein general formula (3) according to Claim 12 is represented by general formula (3c) below 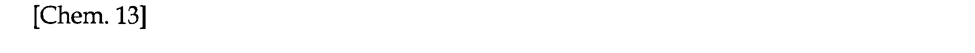 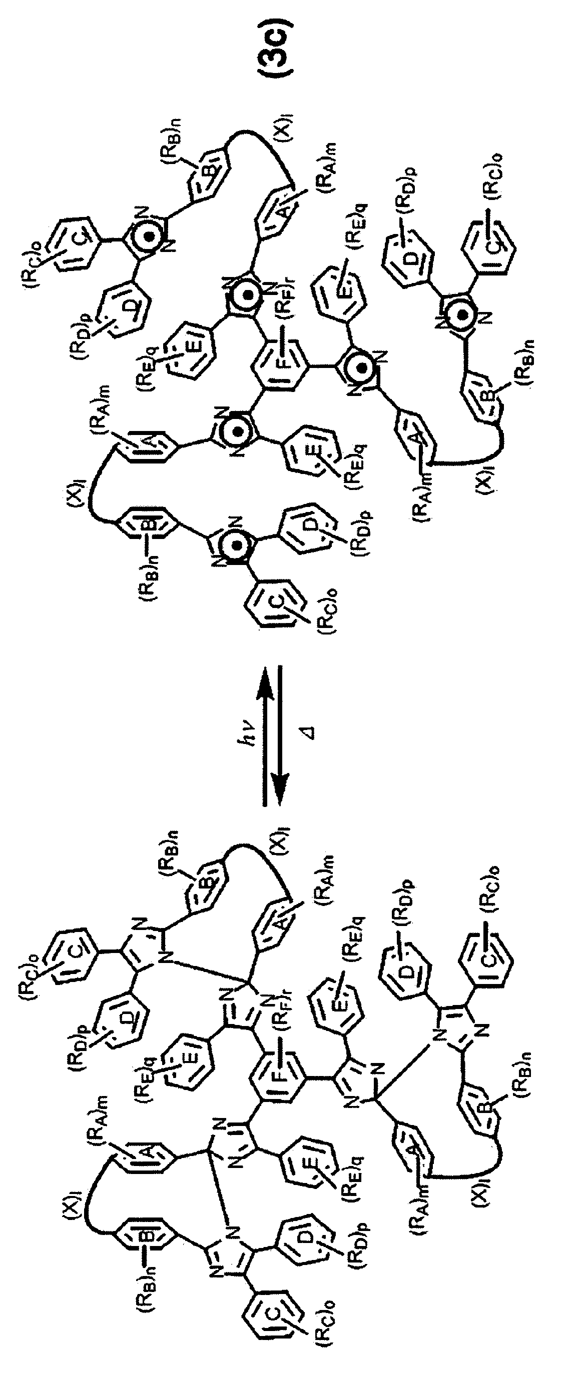 wherein, the 16 aryl groups may or may not have substituents R_{A}, R_{B}, and R_{C} to R_{F}, all of the substituents R_{A}, R_{B}, and R_{C} to R_{F} of the aryl groups are independently identical to or different from each other and have the same meanings as those of the substituents R_{A}, R_{B}, and R_{C} to R_{F} respectively in general formula (2) according to Claim 5,
m and n are mutually independently an integer of 0 to 4,
o to q are mutually independently an integer of 0 to 5, and
r is an integer of 0 to 3.

15. The compound according to any one of Claims 12 to 14, wherein the bridging group is a bridging group that does not conjugate with a triarylimidazolyl radical (TAIR).

16. The compound according to any one of Claims 12 to 15, wherein the bridging group is one or more types of bridging group selected from the group consisting of a -CH₂CH₂- group, a -CH₂CH₂CH₂- group, a -CH₂CH₂CH₂CH₂- group, an -Si(CH3)₂Si(CH3)₂- group, an -Si(CH₃)₂Si(CH₃)₂Si(CH₃)₂- group, an - Si(CH₃)₂Si(CH₃)₂Si(CH₃)₂Si(CH₃)₂- group, an -SiH₂OSiH₂- group, an - Si(CH₃)₂OSi(CH₃)₂- group, an -Si(CH₃CH₂)₂OSi(CH₂CH₃)₂- group, a -CH₂SCH₂-group, a -CH₂OCH₂- group, an -OCH₂CH₂O- group, and a -CH₂OCH₂CH₂OCH₂-group.

17. The compound according to any one of Claims 12 to 15, wherein m and n are 0 and the bridging group X is unsubstituted, or the substituents R_{A}, R_{B}, and R_{X} are methyl groups.

18. The compound according to any one of Claims 12 to 17, wherein the structure represented by partial structural formula (vi) below 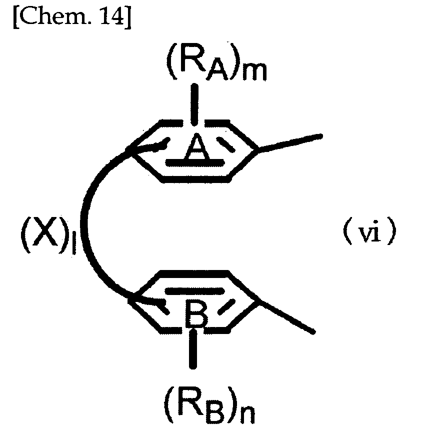 is represented by partial structural formula (vii) below 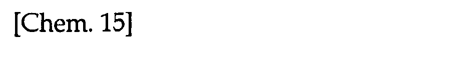 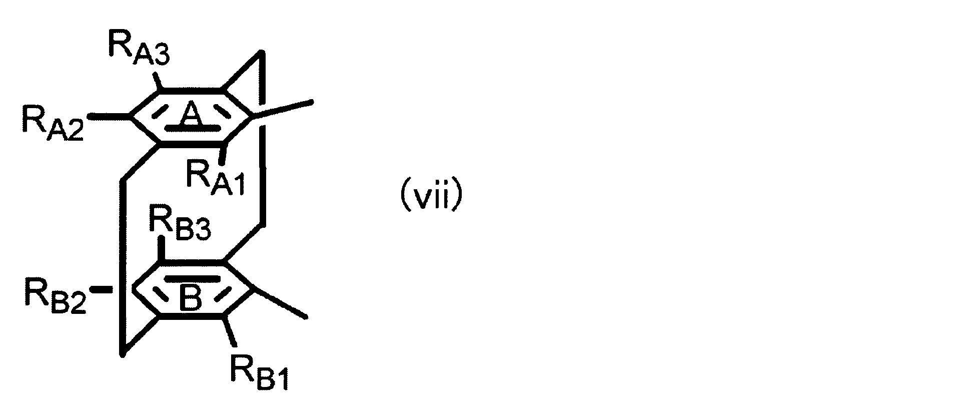 wherein, substituents R_{A1} to R_{A3} and R_{B1} to R_{B3} are independently identical to or different from each other and are substituents having the same meanings as those of the substituents R_{A} and R_{B} respectively in general formula (3) according to Claim 12 or hydrogen atoms.

19. A polymer compound having a repeating structural unit represented by partial structural formula (iv) below
[Chem. 16] **-[(A)**γ**-(B)**_{δ}**]**_{ε}**-** **(iv)**
and/or partial structural formula (v) below 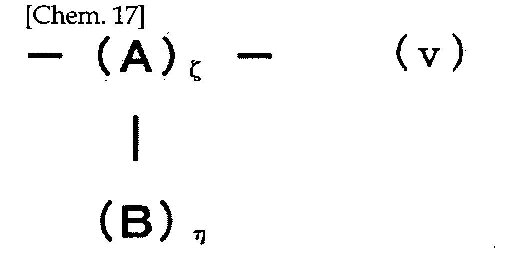 wherein, A is any linking group comprising one or more types of atoms selected from the group consisting of carbon, nitrogen, and oxygen atoms,
B is a derivative of the compound according to any one of Claims 5 to 18,
A-B denotes a bond between the linking group and one or two substituents selected from substituents R_{C} to R_{F} and R_{L} of the compound,
γ is an integer of 0 or greater, and
δ, ε, ζ, and η are mutually independently an integer of 1 or greater.

20. A photochromic material comprising the compound and/ or polymer compound according to any one of Claims 5 to 19.

21. A solvent comprising the compound and/or polymer compound according to any one of Claims 5 to 19.

22. A resin comprising the compound and/or polymer compound according to any one of Claims 5 to 19.

23. A material composition having photochromism and comprising one or more types selected from the group consisting of the compound, polymer compound, photochromic material, solvent, and resin according to any one of Claims 5 to 22, the material composition being selected from the group consisting of a light control material, a holographic material, an ink material, an optical information display device, an optical switch element, and a photoresist material.

24. A method for producing the compound according to any one of Claims 5 to 11, comprising reacting the compound according to any one of Claims 1 to 4 and a compound represented by general formula (4) below 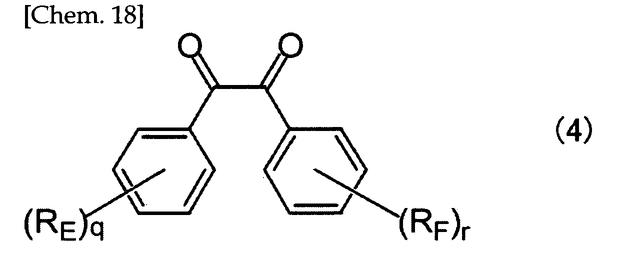 wherein, the two aryl groups may or may not, mutually independently, have substituents R_{E} and R_{F},
said substituents R_{E} and R_{F} are independently identical to or different from each other and are one or more substituents selected from the group consisting of a halogen atom, a nitro group, a cyano group, a trifluoromethyl group, a hydroxy group, a thiol group, an amino group, a diphenylamino group, a carbazole group, a straight-chain or branched alkyl, alkylamino, or alkoxy group having 1 to 20 carbons, a -Y₁-SiZ₁Z₂Z₃ group, a -Y₁-SiY₂Z₁Z₂ group, a -Y₁-SiY₂Y₃Z₁ group (here, Y₁ to Y₃ are independently identical to or different from each other and denote a straight-chain or branched alkyl or alkylene group having 1 to 20 carbons, and Z₁ to Z₃ are independently identical to or different from each other and denote a hydrogen atom, a halogen atom, or a straight-chain or branched alkoxy group having 1 to 8 carbons), a substituent represented by partial structural formula (i) below 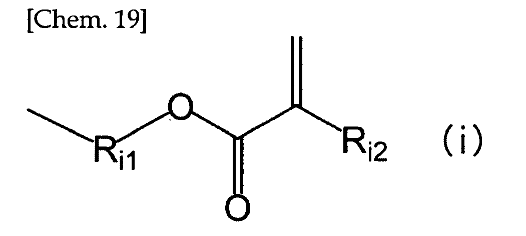 (here, Rᵢ₁ denotes an alkylene or alkoxylene group having 1 to 20 carbons and Rᵢ₂ denotes hydrogen or an alkyl group having 1 to 3 carbons),
a substituent represented by partial structural formula (ii) below 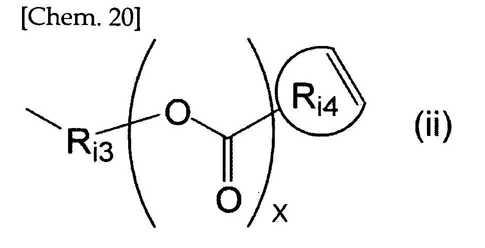 (here, Rᵢ₃ denotes an alkylene or alkoxylene group having 1 to 20 carbons, Rᵢ₄ denotes a cyclic olefin having a total number of carbons and silicons of 5 to 10, and x denotes 0 or 1), and a substituent represented by partial structural formula (iii) below 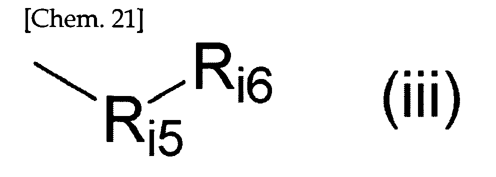 (here, Rᵢ₅ denotes an alkylene or alkoxylene group having 1 to 20 carbons and Rᵢ₆ denotes an ethylene group or an acetylene group),
q and r are mutually independently an integer of 0 to 5,
the substituent of the aryl group may or may not form, together with the carbon atom to which it is bonded and another substituent, an aliphatic or aromatic ring,
and said ring may or may not have a substituent having the same meaning as that of the substituent of the aryl group.

25. A method for producing the compound according to any one of Claims 12 to 18, comprising reacting the compound according to any one of Claims 1 to 4 and a compound represented by general formula (5) below 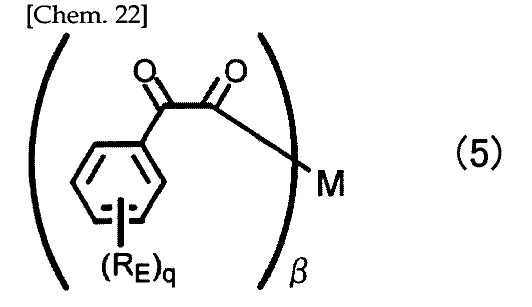 wherein, β is an integer of 1 to 9,
the linking group M is a monocyclic or polycyclic aromatic compound comprising 1 to 12 aromatic rings having 5 to 8 carbon atoms per ring structure,
the aryl group in the 1,2-diketone skeleton-containing structural unit may or may not have a substituent R_{E},
all of the substituents of the aryl group are independently identical to or different from each other and have the same meaning as that of R_{E} of general formula (4) according to Claim 24,
q is an integer of 1 to 5,
the substituent R_{E} may or may not form, together with the carbon atom to which it is bonded and another substituent, an aliphatic or aromatic ring, and said ring and the aromatic ring of the linking group M may or may not further have a substituent R_{M}.
